# EUROPEAN PATENT SPECIFICATION

(11) **EP 1 320 529 B1**
(45) Date of publication and mention of the grant of the patent: **24.05.2006**
(21) Application number: 01985253.2
(22) Date of filing: 19.09.2001
(51) Int. Cl.: C07D 401/06, C07D 401/12, C07D 401/14, C07D 471/04, A61K 31/47

(54) **QUINOLINE DERIVATIVES AS ANTIBACTERIALS**
CHINOLINDERIVATE ALS ANTIBAKTERIELLE MITTEL
DERIVES DE QUINOLINE EN TANT QU'AGENTS ANTIBACTERIENS

(30) Priority: 21.09.2000 GB 0023211; 22.01.2001 GB 0101628
(43) Date of publication of application: 25.06.2003
(73) Proprietor: SMITHKLINE BEECHAM PLC, Brentford, Middlesex TW8 9GS (GB)
(72) Inventor: DAVIES, David,T., GlaxoSmithKline, Harlow, Essex CM19 5AW (GB); MARKWELL, Roger,E., GlaxoSmithKline, Harlow, Essex CM19 5AW (GB); PEARSON, Neil, D., GlaxoSmithKline, Harlow, Essex CM195AW (GB)
(74) Representative: Valentine, Jill Barbara
(86) International application number: PCT/EP2001/010976
(87) International publication number: WO 2002/024684

(56) References cited:
- EP-A- 1 104 764
- WO-A-00/21948
- WO-A-01/07432
- US-A- 4 652 562
- US-A- 5 688 960

## Description

This invention relates to novel compounds, compositions containing them and their use as antibacterials.

WO99/37635, WO00/21948, WO00/21952, WO00/43383, WO0078748, WO01/07432, WO01/07433 disclose piperidine and piperazine derivatives having antibacterial activity.

WO9802434, WO9802438, WO9703069 and WO9639145 disclose certain bicyclic heteroaromatic compounds having protein tyrosine kinase inhibitor, cell proliferation inhibitor and human epidermal growth factor receptor type 2 inhibitor activity.

We have now found a novel group of aminopiperidines which have antibacterial activity.

This invention provides a compound of formula (I) or a pharmaceutically acceptable derivative thereof: 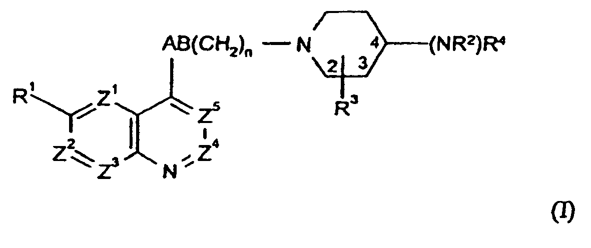 wherein:
one of Z¹, Z², Z³, Z⁴ and Z⁵ is N, one is CR^{1a} and the remainder are CH , or one or two of Z¹, Z², Z³, Z⁴ and Z⁵ are independently CR^{1a} and the remainder are CH;
R ¹ and R^{1a} are independently hydrogen; hydroxy; (C₁₋₆)alkoxy optionally substituted by (C₁₋₆)alkoxy, amino, piperidyl, guanidino or amidino any of which is optionally N-substituted by one or two (C₁₋₆)alkyl, acyl or (C₁₋₆)alkylsulphonyl groups, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted(C₁₋₆)alkyl; halogen; (C₁₋₆)alkyl; (C₁₋₆)alkylthio; trifluoromethyl; trifluoromethoxy; nitro; azido; acyl; acyloxy; acylthio; (C₁₋₆)alkylsulphonyl; (C₁₋₆)alkylsulphoxide; arylsulphonyl; arylsutphoxide or an amino, piperidyl, guanidino or amidino group optionally N-substituted by one or two (C₁₋₆)alkyl, acyl or (C₁₋₆)alkylsulphonyl groups;
or when Z⁵ is CR^{1a}, R^{1a} may instead be cyano, hydroxymethyl or carboxy;
provided that when Z¹, Z², Z³, Z⁴ and Z⁵ are CR^{1a} or CH, then R¹ is not hydrogen;
R² is hydrogen, or (C₁₋₄)alkyl or (C₂₋₄)alkenyl optionally substituted with 1 to 3 groups selected from:
   amino optionally substituted by one or two (C₁₋₄)alkyl groups; carboxy; (C₁₋₄)alkoxycarbonyl; (C₁₋₄)alkylcarbonyl; (C₂₋₄)alkenyloxycarbonyl; (C₂₋₄)alkenylcarbonyl; aminocarbonyl wherein the amino group is optionally substituted by hydroxy, (C₁₋₄)alkyl, hydroxy(C₁₋₄)alkyl, aminocarbonyl(C₁₋₄)alkyl, (C₂₋₄)alkenyl, (C₁₋₄)alkylsulphonyl, trifluoromethylsulphonyl, (C₂₋₄)alkenylsulphonyl, (C₁₋₄)alkoxycarbonyl, (C₁₋₄)alkylcarbonyl, (C₂₋₄)alkenyloxycarbonyl or (C₂₋₄)alkenylcarbonyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; 5-oxo-1,2,4-oxadiazol-3-yl; halogen; (C₁₋₄)alkylthio; trifluoromethyl; hydroxy optionally substituted by (C₁₋₄)alkyl, (C₂₋₄)alkenyl, (C₁₋₄)alkoxycarbonyl, (C₁₋₄)alkylcarbonyl, (C₂₋₄)alkenyloxycarbonyl, (C₂₋₄)alkenylcarbonyl; oxo; (C₁₋₄)alkylsulphonyl; (C₂₋₄)alkenylsulphonyl; or (C₁₋₄)aminosulphonyl wherein the amino group is optionally substituted by (C₁₋₄)alkyl or (C₂₋₄)alkenyl;
R³ is hydrogen; or
   R³ is in the 2-, 3- or 4-position and is:
   carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
   (C₁₋₄)alkyl or ethenyl optionally substituted with any of the substituents listed above for R³ and/or 0 to 2 groups R¹² independently selected from:
      halogen; (C₁₋₆)alkylthio; trifluoromethyl; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocarbonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl; or when R³ is in the 3-position, hydroxy optionally substituted as described above; in addition when R³ is disubstituted with a hydroxy or amino containing substituent and carboxy containing substituent these may together form a cyclic ester or amide linkage, respectively;
R⁴ is a group -X¹-X²-X³-X⁴ in which:
   X¹ is CH₂, CO or SO₂;
   X² is CR¹⁴R¹⁵;
   X³ is NR¹³, O, S, SO₂ or CR¹⁴R¹⁵; wherein:
      each of R¹⁴ and R¹⁵ is independently selected from: hydrogen; halo; (C₁₋₄)alkoxy; (C₁₋₄)alkylthio; trifluoromethyl; cyano; carboxy; nitro; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; carboxy(C₁₋₄)alkyl; halo(C₁₋₄)alkoxy; halo(C₁₋₄)alkyl; (C₁₋₄)alkylcarbonyloxy; (C₁₋₄)alkoxycarbonyl(C₁₋₄)alkyl; hydroxy(C₁₋₄)alkyl; mercapto(C₁₋₄)alkyl; (C₁₋₄)alkoxy; hydroxy, amino or aminocarbonyl optionally substituted as for corresponding substituents in R³; (C₁₋₄)alkylsulphonyl; (C₂₋₄)allcenylsulphonyl; or aminosulphonyl wherein the amino group is optionally mono- or di-substituted by (C₁₋₄)alkyl or (C₂₋₄)alkenyl, provided that R¹⁴ and R¹⁵ on the same carbon atom are not both selected from optionally substituted hydroxy and optionally substituted amino; or
      R¹⁴ and R¹⁵ together represent oxo;
      R¹³ is hydrogen; trifluoromethyl; (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl; or
      two R¹⁴ groups or an R¹³ and an R¹⁴ group on adjacent atoms together represent a bond and the remaining R¹³, R¹⁴ and R¹⁵ groups are as above defined;
      two R¹⁴ groups and two R¹⁵ groups on adjacent atoms together represent bonds such that X² and X³ is triple bonded;
      two R¹⁴ groups or an R¹³ and an R¹⁴ group in X² and X³ together with the atoms to which they are attached form a 5 or 6 membered carbocyclic or heterocyclic ring and the remaining R^{15a} groups are as above defined;
   X⁴ is phenyl or C or N linked monocyclic aromatic 5- or 6-membered heterocycle containing up to four heteroatoms selected from O, S and N and optionally C-substituted by up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C ₁₋₄)alkylcarbonyl; (C₂₋₄)alkenyloxycarbonyl; (C₂₋₄)allcenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; aryl, aryl(C₁₋₄)alkyl or aryl(C₁₋₄)alkoxy; and
   optionally N substituted by trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl(C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
n is 0 or 1;
A is NR¹¹, O or CR⁶R⁷ and B is NR¹¹, O, SO₂ or CR⁸R⁹ and wherein:
   each of R⁶, R⁷, R⁸ and R⁹ is independently selected from: hydrogen; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or aminosulphonyl wherein the amino group is optionally substituted by (C₁₋₆)alkyl or (C₂₋₆)alkenyl;
   or when n=1 R⁶ and R⁸ together represent a bond and R⁷ and R⁹ are as above defined;
   or R⁶ and R⁷ or R⁸ and R⁹ together represent oxo;
provided that:
   when A is NR¹¹, B is not NR¹¹ or O;
   when A is CO, B is not CO, O or SO₂;
   when n is 0 and A is NR¹¹, CR⁸R⁹ can only be CO;
   when A is CR⁶R⁷ and B is SO₂, n is 0;
   when n is 0, B is not NR¹¹ or O or R⁸ and R⁹ are not optionally substituted hydroxy or amino;
   when A is O, B is not NR¹¹, O, SO₂ or CO and n=1; and
   when A-B is CR⁷=CR⁹, n is 1
R¹⁰ is selected from (C₁₋₄)alkyl; (C₂₋₄)alkenyl and aryl any of which may be optionally substituted by a group R¹² as defined above; carboxy; aminocarbonyl wherein the amino group is optionally substituted by hydroxy, (C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl or (C₂₋₆)alkenyl; (C₁₋₆)alkylsulphonyl; trifluoromethylsulphonyl; (C₂₋₆)alkenylsulphonyl; (C₁₋₆)alkoxycarbonyl; (C₁₋₆)alkylcarbonyl; (C₂₋₆)alkenyloxycarbonyl; and (C₂₋₆)alkenylcarbonyl; and
R¹¹ is hydrogen; trifluoromethyl, (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
or where one of R³ and R⁶, R⁷, R⁸ or R⁹ contains a carboxy group and the other contains a hydroxy or amino group they may together form a cyclic ester or amide linkage.

This invention also provides a method of treatment of bacterial infections in mammals, particularly in man, which method comprises the administration to a mammal in need of such treatment an effective amount of a compound of formula (I), or a pharmaceutically acceptable derivative thereof.

The invention also provides the use of a compound of formula (I), or a pharmaceutically acceptable derivative thereof, in the manufacture of a medicament for use in the treatment of bacterial infections in mammals.

The invention also provides a pharmaceutical composition comprising a compound of formula (I), or a pharmaceutically acceptable derivative thereof, and a pharmaceutically acceptable carrier.

In one aspect, R⁴ is not phenylpropyl or phenylpropenyl in which the phenyl group is optionally substituted by up to five groups R^{a} wherein R^{a} is halogen, mercapto, (C₁₋₄)akyl, phenyl, (C₁₋₄)alkoxy, hydroxy(C₁₋₄)alkyl, mercapto (C₁₋₄)alkyl, halo(C₁₋₄)alkyl, hydroxy, optionally substituted amino, nitro, carboxy, (C₁₋₄)alkylcarbonyloxy, (C₁₋₄)alkoxycarbonyl, formyl, or (C₁₋₄)alkylcarbonyl. In a further aspect R⁴ is not optionally substituted phenylpropyl or phenylpropenyl.

In another aspect, when X¹ is CH₂, X⁴ is phenyl optionally substituted by up to five groups R^{a} and X² and X³ are each CR¹⁴R¹⁵ one of R¹⁴ and R¹⁵ cannot be hydroxy when the remainder are hydrogen. In a further aspect when X¹ is CH₂, X⁴ is optionally substituted phenyl and X² and X³ are each CR¹⁴R¹⁵ one of R¹⁴ and R¹⁵ cannot be hydroxy when the remainder are hydrogen.

In another aspect, when X¹ is CH₂, X⁴ is phenyl optionally substituted by up to five groups R^{a}, -X¹-X²-X³- is not -(CH₃)₂O-, or -(CH₃)₂CO-. In a further aspect when _{X}1 is CH₂, X⁴ is optionally substituted phenyl, -X¹-X²-X³- is not -(CH₃)₂O-, or -(CH₃)₂CO-.

In another aspect, R⁴ is not a heteroaryl (C₁₋₃)alkyl or heteroaroyl (C₁₋₂)alkyl group in which the heteroaryl ring is optionally substituted by up to three groups R^{b} selected from optionally substituted amino, halogen, (C₁₋₄)alkyl, (C₁₋₄)alkoxy, halo(C₁₋₄)alkyl, hydroxy, carboxy, carboxy salts, carboxy esters such as (C₁₋₄)allcoxycarbonyl, (C₁₋₄)alkoxycarbonyl(C₁₋₄)alkyl, aryl, and oxo groups. In a further aspect, R⁴ is not an optionally substituted heteroaryl (C₁₋₃)alkyl or heteroaroyI(C₁₋₂) alkyl group.

In one further aspect:
one of Z¹, Z², Z³, Z⁴ and Z⁵ is N, one is CR^{1a} and the remainder are CH , or one of Z¹, Z², Z³, Z⁴ and Z⁵ is CR^{1a} and the remainder are CH;
X³ is other than S;
each of R¹⁴ and R¹⁵ is independently selected from: hydrogen; (C₁₋₄)alkoxy; (C₁₋₄)allcylthio; trifluoromethyl; cyano; (C₁₋₄)alkyl; (C₂₋₄)alkenyl; (C₁₋₄)akoxycarbonyl; (C₁₋₄)alkylcarbonyl; (C₂₋₄)akenyloxycarbonyl; (C₂₋₄)alkenylcarbonyl; hydroxy, amino or aminocarbonyl optionally substituted as for corresponding substituents in R³; (C₁₋₄)alkylsulphonyl; (C₂₋₄)alkenylsulphonyl; or aminosulphonyl wherein the amino group is optionally mono- or di-substituted by (C₁₋₄)alkyl or (C₂₋₄)alkenyl; or R¹⁴ and R¹⁵ together represent oxo;
R¹³ is hydrogen; trifluoromethyl, (C₁₋₆)alkyl; (C₂₋₆)alkenyl; (C₁₋₆)alkoxycarbonyl; (C₁₋₆)alkylcarbonyl; aminocarbonyl wherein the amino group is optionally substituted by (C₁₋₆)alkoxycarbonyl, (C _{1 -6})alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl; or
two R¹⁴ groups or an R¹³ and an R¹⁴ group on adjacent atoms together represent a bond and the remaining R¹³, R¹⁴ and R¹⁵ groups are as above defined;

In another further aspect:
one of Z¹, Z², Z³, Z⁴ and Z⁵ is N, one is CR^{1a} and the remainder are CH , or one of Z¹, Z², Z³, Z⁴ and Z⁵ is CR^{1a} and the remainder are CH;
R¹ and R^{1a} are other than trifluoromethoxy;
X¹ is CH₂;
X³ is other than S;
each of R¹⁴ and R¹⁵ is independently selected from: hydrogen; (C₁₋₄)alkoxy; (C₁₋₄)alkylthio; trifluoromethyl; cyano; (C₁₋₄)alkyl; (C₂₋₄)alkenyl; (C₁₋₄)alkoxycarbonyl; (C₁₋₄)alkylcarbonyl; (C₂₋₄)alkenyloxycarbonyl; (C₂₋₄)alkenylcarbonyl; hydroxy, amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; or R¹⁴ and R¹⁵ together represent oxo;
R¹³ is hydrogen; trifluoromethyl, (C₁₋₆)alkyl; (C₂₋₆)alkenyl; (C₁₋₆)alkoxycarbonyl; (C₁₋₆)alkylcarbonyl; aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl; or
two R¹⁴ groups or an R¹³ and an R¹⁴ group on adjacent atoms together represent a bond and the remaining R¹³, R¹⁴ and R¹⁵ groups are as above defined;
A is NR¹¹ or CR⁶R⁷ and B is NR¹¹, O, SO₂ or CR⁸R⁹ and wherein: each of R⁶, R⁷, R⁸ and R⁹ is independently selected from: hydrogen; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or aminosulphonyl wherein the amino group is optionally substituted by (C₁₋₆)alkyl, or (C₂₋₆)alkenyl;
or R⁶ and R⁸ together represent a bond and R⁷ and R⁹ are as above defined;
or R⁶ and R⁷ or R⁸ and R⁹ together represent oxo;
provided that:
when A is NR¹¹, B is not NR¹¹, O or SO₂;
when A is CO, B is not CO, O or SO₂;
when n is 0 and A is NR¹¹, CR⁸R⁹ can only be CO;
when A is CR⁶R⁷ and B is SO₂, n is 0;
when n is 0, B is not NR¹¹ or O or R⁸ and R⁹ are both hydrogen; and
when A-B is CR⁷=CR⁹, n is 1;
and when one of R³ and R⁶, R⁷, R⁸ or R⁹ contains a carboxy group and the other contains a hydroxy or amino group they do not together form a cyclic ester or amide linkage.

Preferably Z⁵ is CH orN, Z³ is CH or CF and Z¹, Z² and Z⁴ are each CH, or Z¹ is N, Z³ is CH or CF and Z², Z⁴ and Z⁵ are each CH.

When R¹ or R^{1a} is substituted alkoxy it is preferably (C₂₋₆)alkoxy substitituted by optionally N-substituted amino, guanidino or amidino, or (C₁₋₆)alkoxy substituted by piperidyl. Suitable examples of R¹ and R^{1a} alkoxy include methoxy, trifluoromethoxy, n-propyloxy, iso-butyloxy, aminoethyloxy, aminopropyloxy, aminobutyloxy, aminopentyloxy, guanidinopropyloxy, piperidin-4-ylmethyloxy, phthalimido pentyloxy or 2-aminocarbonylprop-2-oxy. -

Preferably R¹ and R^{1a} are independently methoxy, amino(C₃₋₅)alkyloxy, guanidino(C₃₋₅)alkyloxy, piperidyl(C₃₋₅)allcyloxy, nitro or fluoro; R¹ is more preferably methoxy, amino(C₃₋₅)alkyloxy or guanidino(C₃₋₅)alk-yloxy. R^{1a} is more preferably H or F. Most preferably R¹ is methoxy and R^{1a} is H or when Z³ is CR^{1a} it may be C-F.

When Z⁵ is CR^{1a}, R^{1a} is preferably hydrogen, cyano, hydroxymethyl or carboxy, most preferably hydrogen.

Preferably n is 0.

R² is preferably hydrogen; (C₁₋₄)alkyl substituted with carboxy, optionally substituted hydroxy, optionally substituted aminocarbonyl, optionally substituted amino or (C₁₋₄)alkoxycarbonyl; or (C₂₋₄)alkenyl substituted with (C₁₋₄)alkoxycarbonyl or carboxy. More preferred groups for R² are hydrogen, carboxymethyl, hydroxyethyl, aminocarbonylmethyl, ethoxycarbonylmethyl, ethoxycarbonylallyl and carboxyallyl, most preferably hydrogen.

Preferred examples of R³ include hydrogen; optionally substituted hydroxy; (C₁₋₄) alkyl; ethenyl; optionally substituted 1-hydroxy-(C₁₋₄) alkyl; optionally substituted aminocarbonyl; carboxy(C₁₋₄)alkyl; optionally substituted aminocarbonyl(C₁₋₄)alkyl; cyano(C₁₋₄)alkyl; optionally substituted 2-oxo-oxazolidinyl and optionally substituted 2-oxo-oxazolidinyl(C₁₋₄alkyl). More preferred R³ groups are hydrogen; CONH₂; 1-hydroxyalkyl e.g. CH₂OH, CH(OH)CH₂CN; CH₂CO_{Z}H; CH₂CONH₂; -CONHCH₂CONH₂; 1,2-dihydroxyalk-yi e.g. CH(OH)CH₂OH; CH₂CN; 2-oxo-oxazolidin-5-yl and 2-oxo-oxazolidin-5-yl(C₁₋₄alkyl). Most preferably R³ is hydrogen.

R³ is preferably in the 3- or 4-position.

When R3 and R⁶, R⁷, R⁸ or R⁹ together form a cyclic ester or amide linkage, it is preferred that the resulting ring is 5-7 membered.

When A is CH(OH) the R-stereochemistry is preferred.

Preferably A is NH, NCH₃, CH₂, CHOH, CH(NH₂), C(Me)(OH) or CH(Me).

Preferably B is CH₂ or CO.

Preferably A-B is CHOH-CH₂, NR¹¹-CH₂ or NR¹¹-CO.

Particularly preferred are those compounds where n=0, A is NH and B is CO, or A is CHOH and B is CH₂, when more preferably A is the R-isomer of CHOH.

Preferably R¹¹ is hydrogen or (C₁₋₄)alkyl e.g. methyl, more preferably hydrogen.

R¹³, R¹⁴ and R¹⁵ are preferably H or (C₁₋₄)alkyl such as methyl, more preferably hydrogen.

X¹ is preferably CH₂.

X² is preferably CH₂ or together with X³ forms a CH=CH group.

X3 is preferably CH₂, O or NH, or together with X2 forms a CH=CH group.

Preferred linker groups -X¹-X²-X³- include -(CH₂)₂-O-, -CH₂-CH=CH-, - (CH₂)₃-, -(CH₂)₂-NH- or -CH₂CONH-.

Monocyclic aromatic heterocyclic groups for X⁴ include pyridyl, pyrazinyl, pyrimidinyl, triazolyl, tetrazolyl, thienyl, isoimidazolyl, thiazolyl, furanyl and imidazolyl, 2H-pyridazone, 1H-pyrid-2-one. Preferred aromatic heterocyclic groups include pyrid-2-yl, pyrid-3-yl, thiazole-2-yl, pyrimidin-2-yl, pyrimidin-5-yl and fur-2-yl.

Preferred substituents on heterocyclic X⁴ include halo especially fluoro, trifluoromethyl and nitro.

Preferred substituents on phenyl X⁴ include halo, especially fluoro, nitro, cyano, trifluoromethyl, methyl, methoxycarbonyl and methylcarbonylamino.

Preferably X⁴ is 2-pyridyl, 3-fluorophenyl, 3,5-difluorophenyl or thiazol-2-yl.

When used herein, the term "alkyl" includes groups having straight and branched chains, for instance, methyl, ethyl, n-propyl, iso-propyl,n-butyl, iso-butyl, sec-butyl, t-butyl, pentyl and hexyl. The term'allcenyl' should be interpreted accordingly.

Halo or halogen includes fluoro, chloro, bromo and iodo.

Haloalkyl moieties include 1-3 halogen atoms.

Unless otherwise defined, the term "heterocyclic" as used herein includes optionally substituted aromatic and non-aromatic, single and fused, rings suitably containing up to four hetero-atoms in each ring selected from oxygen, nitrogen and sulphur, which rings may be unsubstituted or C-substituted by, for example, up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; optionally substituted aryl, aryl(C₁₋₄)alkyl or aryl(C₁₋₄)alkoxy and oxo groups.

Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

Where an amino group forms part of a single or fused non-aromatic heterocyclic ring as defined above suitable optional substituents in such substituted amino groups include H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C₁₋₄)alkyl; (C₁₋₄)alkoxycarbonyl; (C ₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl.

When used herein the term "aryl", includes optionally substituted phenyl and naphthyl.

Aryl groups may be optionally substituted with up to five, preferably up to three, groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano, carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; phenyl, phenyl(C₁₋₄)alkyl or phenyl(C₁₋₄)alkoxy

The term "acyl" includes formyl and (C₁₋₆)alkylcarbonyl group. Preferred compounds of formula (I) include:
2-{1-[(R)-2-Hydroxy-2-(6-methoxy-[1,5]-naphthyridine-4-yl)-ethyl]-piperidin-4-ylamino}-N-phenyl-acetamide;
4- {*trans*-1-[3,5-Difluorophenyl]-3-propenyl}amino-1-[2-(R)-hydroxy-2-(6-methoxyquinolin-4-yl)]ethylpiperidine;
(R)-1-(6-Methoxy-[1,5]naphthyridin-4-yl)-2-{4-[2-(pyridin-2-yloxy)-ethylamino]-piperidin-1-yl}-ethanol;
(R)-1-(6-Methoxy-[1,5]naphthyridin-4-yl)-2-[4-((E)-3-pyridin-2-yl-allylamino)-piperidin-1-yl]-ethanol;
4-[2-(3,5-Difluorophenylamino)-ethyl]amino-1-[2-(R)-hydroxy-2-(6-methoxyquinolin-4-yl)]ethylpiperidine;
4-[2-(3-Fluorophenylamino)-ethyl]amino-1-[2-(R)-hydroxy-2-(6-methoxyquinolin-4-yl)]ethylpiperidine; and
4-[*trans*-1-(2-thiazolyl)-3-propenyl]amino-1-[2-(R)-hydroxy-2-(6-methoxy-[1,5]-naphthyridin-4-yl)]ethylpiperidine
and pharmaceutically acceptable derivatives thereof.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or pharmaceutically acceptable derivative thereof.

Particular compounds according to the invention include those mentioned in the examples and their pharmaceutically acceptable derivatives.

Pharmaceutically acceptable derivatives of the above-mentioned compounds of formula (I) include the free base form or their acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. Compounds of formula (I) may also be prepared as the N-oxide. Compounds of formula (I) having a free carboxy group may also be prepared as an *in vivo* hydrolysable ester. The invention extends to all such derivatives.

Examples of suitable pharmaceutically acceptable *in vivo* hydrolysable ester-forming groups include those forming esters which break down readily in the human body to leave the parent acid or its salt. Suitable groups of this type include those of part formulae (i), (ii), (iii), (iv) and (v):

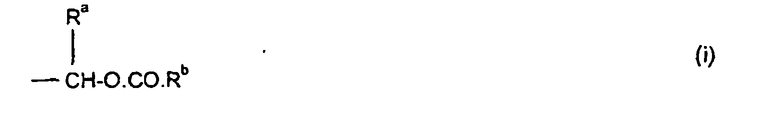

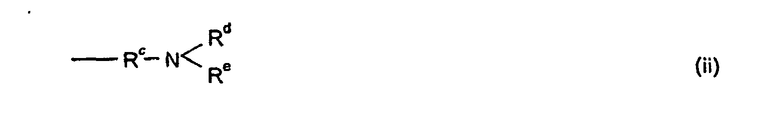

―CH₂―OR^{f} (iii)

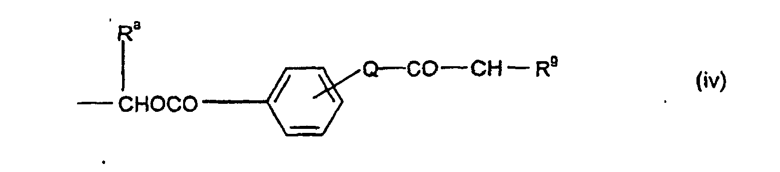

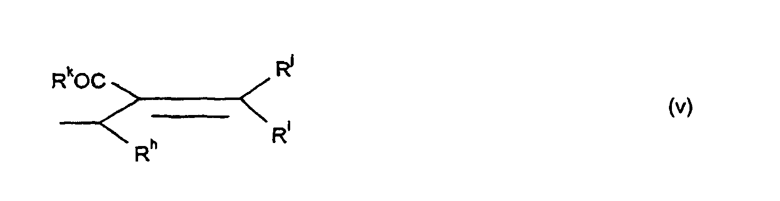

wherein R^{a} is hydrogen, (C₁₋₆) alkyl, (C₃₋₇) cycloalkyl, methyl, or phenyl, R^{b} is (C₁₋₆) alkyl, (C₁₋₆) alkoxy, phenyl, benzyl, (C₃₋₇) cycloalkyl, (C₃₋₇) cycloalkyloxy, (C₁₋₆) alkyl (C₃₋₇) cycloalkyl, 1-amino (C₁₋₆) alkyl, or 1-(C₁₋₆ alkyl)amino (C₁₋₆) alkyl; or R^{a} and R^{b} together form a 1,2-phenylene group optionally substituted by one or two methoxy groups; R^{c} represents (C₁₋₆) alkylene optionally substituted with a methyl or ethyl group and R^{d} and R^{e} independently represent (C₁₋₆) alkyl; R^{f} represents (C₁₋₆) alkyl; R^{g} represents hydrogen or phenyl optionally substituted by up to three groups selected from halogen, (C₁₋₆) alkyl, or (C₁₋₆) alkoxy; Q is oxygen or NH; R^{h} is hydrogen or (C₁₋₆) alkyl; Rⁱ is hydrogen, (C₁₋₆) alkyl optionally substituted by halogen, (C₂₋₆) alkenyl, (C₁₋₆) alkoxycarbonyl, aryl or heteroaryl; or R^{h} and Rⁱ together form (C₁₋₆) alkylene; R^{j} represents hydrogen, (C₁₋₆) alkyl or (C₁₋₆) alkoxycarbonyl; and R^{k} represents (C₁₋₈) alkyl, (C₁₋₈) alkoxy, (C₁₋₆) alkoxy(C₁₋₆)alkoxy or aryl.

Examples of suitable *in vivo* hydrolysable ester groups include, for example, acyloxy(C₁₋₆)alkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; (C₁₋₆)alkoxycarbonyloxy(C₁₋₆)alkyl groups, such as ethoxycarbonyloxymethyl, α-ethoxycarbonyloxyethyl and propoxycarbonyloxyethyl; di(C₁₋₆)alkylamino(C₁₋₆)alkyl especially di(C₁₋₄)alkylamino(C₁₋₄)alkyl groups such as dimethylaminomethyl, dimethylaminoethyl, diethylaminomethyl or diethylaminoethyl; 2-((C₁₋₆)alkoxycarbonyl)-2-(C₂₋₆)alkenyl groups such as 2-(isobutoxycarbonyl)pent-2-enyl and 2-(ethoxycarbonyl)but-2-enyl; lactone groups such as phthalidyl and dimethoxyphthalidyl.

A further suitable pharmaceutically acceptable *in vivo* hydrolysable ester-forming group is that of the formula: 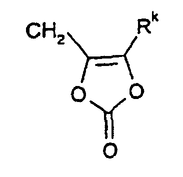 wherein R^{k} is hydrogen, C₁₋₆ alkyl or phenyl.
R is preferably hydrogen.

Compounds of formula (I) may also be prepared as the corresponding N-oxides.

Certain of the compounds of formula (I) may exist in the form of optical isomers, e.g. diastereoisomers and mixtures of isomers in all ratios, e.g. racemic mixtures. The invention includes all such forms, in particular the pure isomeric forms. For example the invention includes compound in which an A-B group CH(OM-CH₂ is in either isomeric configuration, the R-isomer is preferred. The different isomeric forms may be separated or resolved one from the other by conventional methods, or any given isomer may be obtained by conventional synthetic methods or by stereospecific or asymmetric syntheses.

In a further aspect of the invention there is provided a process for preparing compounds of formula (1), and pharmaceutically acceptable derivatives thereof, which process comprises:
reacting a compound of formula (IV) with a compound of formula (V): 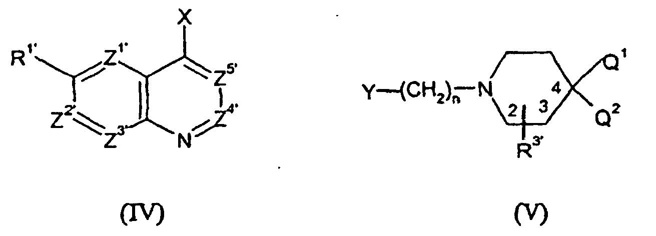
wherein n is as defined in formula (I); Z^{1'}, Z^{2'}, Z^{3'}, Z^{4'}, Z^{5'}, R^{1'} and R^{3'} are Z¹, Z², Z³, Z⁴, Z⁵, R¹ and R³ as defined in formula (I) or groups convertible thereto;
Q¹ is NR²R⁴ or a group convertible thereto wherein R^{2'} and R^{4'} are R² and R⁴ as defined in formula (I) or groups convertible thereto and Q² is H or R^{3'} or Q¹ and Q² together form an optionally protected oxo group;
and X and Y may be the following combinations:
(i) X is A'-COW, Y is H and n is 0;
(ii) X is CR⁶=CR⁸R⁹, Y is H and n is 0;
(iii) X is oxirane, Y is H and n is 0;
(iv) X is N=C=O and Y is H and n is 0;
(v) one of X and Y is CO₂R^{y} and the other is CH₂CO₂R^{x};
(vi) X is CHR⁶R⁷ and Y is C(=O)R⁸;
(vii) X is CR⁷=PR^{z} ₃ and Y is C(=O)R⁹ and n=1;
(viii) X is C(=O)R⁷ and Y is CR⁹=PR^{z} ₃ and n=1;
(ix) Y is COW and X is NHR^{11'} or NR^{11'}COW and n=0 or 1 or when n=1 X is COW and Y is NHR^{11'}, NCO or NR^{11'}COW;
(x) X is C(=O)R⁶ and Y is NHR^{11'} or X is NHR^{11'} and Y is C(=O)R⁸ and n=1;
(xi) X is NHR^{11'} and Y is CR⁸R⁹W and n=1;
(xii) X is CR⁶R⁷W and Y is NHR^{11'} or OH and n=1; or
(xiii) X is CR⁶R⁷SO₂W and Y is H and n=0;
(xiv) X is W or OH and Y is CH₂OH and n is 1;
(xv) X is NHR^{11'} and Y is SO₂W or X is NR^{11'}SO₂W and Y is H, and n is 0;
(xvi) X is NR^{11'}COCH₂W and Y is H and n=0;
in which W is a leaving group, e.g. halo or imidazolyl; R^{x} and RY are (C₁₋₆)alkyl; R^{z} is aryl or (C₁₋₆)alkyl; A' and NR^{11'} are A and NR¹¹ as defined in formula (I), or groups convertible thereto; and oxirane is: 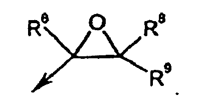 wherein R⁶, R⁸ and R⁹ are as defined in formula (I);
and thereafter optionally or as necessary converting Q¹ and Q² to NR^{2'}R^{4'}; converting
A', Z^{1'}, Z^{2'}, Z^{3'}, Z^{4'}, Z^{5'}, R^{1'}, R^{2'}, R^{3'}, R^{4'} and NR^{11'} to A, Z¹, Z², Z³, Z⁴, Z⁵, R¹, R², R³, R⁴ and NR¹¹; converting A-B to other A-B, intercoaverting R¹, R², R³ and/or R⁴, and/or forming a pharmaceutically acceptable derivative thereof

Process variant (i) initially produces compounds of formula (I) wherein A-B is A'-CO.

Process variant (ii) initially produces compounds of formula (I) wherein A-B is CHR⁶-CR⁸R⁹.

Process variant (iii) initially produces compounds of formula (I) wherein A-B is CR⁶(OH)-C_{R}8_{R}9.

Process variant (iv) initially produces compounds of formula (I) where A-B is NH-CO.

Process variant (v) initially produces compounds of formula (I) wherein A-B is CO-CH₂ or CH₂-CO.

Process variant (vi) initially produces compounds of formula (I) wherein A-B is CR⁶R⁷-CR⁸OH.

Process variant (vii) and (viii) initially produce compounds of formula (I) wherein A-B is CR⁷=CR⁹.

Process variant (ix) initially produces compounds of formula (I) where A-B is CO-NR¹¹ or NR¹¹-CO.

Process variant (x) initially produces compounds of formula (I) wherein A-B is NR¹¹-CHR⁸.

Process variant (xi) initially produces compounds of formula (I) wherein A-B is NR^{11'}-CR⁸R⁹.

Process variant (xii) initially produces compounds of formula (I) wherein A-B is CR⁶R⁷- NR^{11'} or CR⁶R⁷-O.

Process variant (xiii) initially produces compounds of formula (I) where A-B is CR⁶R⁷-SO₂.

Process variant (xiv) initially produces compounds of formula (I) wherein A-B is O-CH₂.

Process variant (xv) initially produces compounds where AB is NR¹¹SO₂.

Process variant (xvi) initially produces compounds of formula (I) where A-B is NR¹¹'-CO and n=1.

In process variants (i) and (ix) the reaction is a standard amide or urea formation reaction involving e.g.:
1. Activation of a carboxylic acid (e.g. to an acid chloride, mixed anhydride, active ester, O-acyl-isourea or other species), and treatment with an amine (Ogliaruso, M.A.; Wolfe, J.F. in *The Chemistry of Functional Groups (Ed. Patai, S.) Suppl. B: The Chemistry of Acid Derivatives, Pt. 1* (John Wiley and Sons, 1979), pp 442-8; Beckwith, A.L.J. in *The Chemistry of Functional Groups (Ed. Patai, S.) Suppl. B: The Chemistry of Amides (Ed. Zabricky, J.)* (John Wiley and Sons, 1970), p 73 ff. The acid and amide are preferably reacted in the presence of an activating agent such as 1-(dimethylaminopropyl)-3-ethylcarbodiimide hydrochloride (EDC) or 1-hydroxybenzotriazole (HOBT) or O-(7-azabenzotriazol-1-yl)-*N*,*N*,*N*',*N*'-tetramethyluronium hexafluorophosphate (HATU); or
2. The specific methods of:
   *a. in situ* conversion of an acid into the amine component by a modified Curtius reaction procedure (Shioiri, T., Murata, M., Hamada, Y., *Chem. Pharm. Bull.* 1987, 35, 2698)
   b. *in situ* conversion of the acid component into the acid chloride under neutral conditions (Villeneuve, G. B.; Chan, T. H., *Tetrahedron. Lett.* 1997, 38, 6489).

A' may be, for example. protected hydroxymethylene.

The process variant (ii) is a standard addition reaction using methods well known to those skilled in the art. The process is preferably carried out in a polar organic solvent e.g. acetonitrile in the presence of an organic base e.g. triethylamine.

In process variant (iii) the coupling may be effected in acetonitrile at room temperature in the presence of one equivalent of lithium perchlorate as catalyst (general method of J.E. Chateauneuf *et al, J. Org. Chem*., 56, 5939-5942, 1991) or more preferably with ytterbium triflate in dichloromethane. In some cases an elevated temperature such as 40 - 70 °C may be beneficial. Alternatively, the piperidine may be treated with a base, such as one equivalent of butyl lithium, and the resulting salt reacted with the oxirane in an inert solvent such as tetrahydrofuran, preferably at an elevated temperature such as 80°C. Use of a chiral epoxide will afford single diastereomers. Alternatively, mixtures of diastereomers may be separated by preparative HPLC or by conventional resolution through crystallisation of salts formed from chiral acids.

The process variant (iv) is a standard urea formation reaction from the reaction of an isocyanate with an amine and is conducted by methods well known to those skilled in the art (for example see March, J; *Advanced Organic Chemistry, Edition* 3 (John Wiley and Sons, 1985), p802-3). The process is preferably carried out in a polar solvent such as N,N-dimethylforamide.

In process variant (v) the process is two step: firstly a condensation using a base, preferably sodium hydride or alkoxide, sodarnide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. 68, 2688-2692 (1946). Similar Claisen methodology is described in Soszko et. al., Pr.Kom.Mat. Przyr.Poznan.Tow.Przyj.Nauk., (1962), 10, 15.

In process variant (vi) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) J. Am. Chem. Soc. 100, 576).

In process variants (vii) and (viii) if a base is used it is preferably NaH, KH, an alkyl lithium e.g. BuLi, a metal alkoxide e.g. NaOEt, sodamide or lithium dialkylamide e.g.di- isopropylamide. An analogous method is described in US 3989691 and M.Gates et. al. (1970) J. Amer.Chem.Soc., 92, 205, as well as Taylor et al. (1972) JACS 94, 6218.

In process variant (x) where X or Y is CHO the reaction is a standard reductive alkylation using, e.g., sodium borohydride or sodium triacetoxyborohydride (Gribble, G. W. in *Encyclopedia of Reagents for Organic Synthesis (Ed. Paquette, L. A.)* (John Wiley and Sons, 1995), p 4649).

The process variants (xi) and (xii) are standard alkylation reactions well known to those skilled in the art, for example where an alcohol or amine is treated with an alkyl halide in the presence of a base (for example see March, J; *Advanced Organic Chemistry, Edition* 3 (John Wiley and Sons, 1985), p364-366 and p342-343). The process is preferably carried out in a polar solvent such as N,N-dimethylformamide

In process variant (xiii) the reaction is a standard sulphonamide formation reaction well known to those skilled in the art. This may be e.g. the reaction of a sulphonyl halide with an amine.

In process variant (xiv) where X is W such as halogen, methanesulphonyloxy or trifluoromethanesulphonyloxy, the hydroxy group in Y is preferably converted to an OM group where M is an alkali metal by treatment of an alcohol with a base. The base is preferably inorganic such as NaH, lithium diisopropylamide or sodium. Where X is OH, the hydroxy group in Y is activated under Mitsunobu conditions (Fletcher et.al. J Chem Soc. (1995), 623). Alternatively the X=O and Y=CH₂OH groups can be reacted directly by activation with dichlorocarbodiimide (DCC) (Chem. Berichte 1963, 95, 2997 or Angewante Chemie 1963 75, 377).

In process variant (xv) the reaction is conducted in the presence of an organic base such as triethylamine or pyridine such as described by Fuhrman et.al., J. Amer. Chem. Soc.; 67,1245, 1945. The X=NR^{11'}SO₂W or Y=S0₂W intermediates can be formed from the requisite amine e.g. by reaction with SO₂Cl₂ analogously to the procedure described by the same authors Fuhrman et.al., J. Amer. Chem. Soc.; 67, 1245, 1945.

In process variant (xvi) the reaction is an alkylation, examples of which are described in J. Med. chem. (1979) 22(10) 1171-6. The compound of formula (IV) may be prepared from the corresponding compound where X is NHR^{11'} by acylation with an appropriate derivative of the acid WCH₂COOH such as the acid chloride.

Reduction of a carbonyl group A or B to CHOH can be readily accomplished using reducing agents well known to those skilled in the art, e.g. sodium borohydride in aqueous ethanol or lithium aluminium hydride in ethereal solution. This is analogous to methods described in EP53964, US384556 and J. Gutzwiller *et al, J. Amer. Chem. Soc*., 1978, 100, 576.

The carbonyl group A or B may be reduced to CH₂ by treatment with a reducing agent such as hydrazine in ethylene glycol, at e.g. 130-160°C, in the presence of potassium hydroxide.

Reaction of a carbonyl group A or B with an organometallic reagent yields a group where R⁸ is OH and R⁹ is alkyl.

A hydroxy group on A or B may be oxidised to a carbonyl group by oxidants well known to those skilled in the art, for example, manganese dioxide, pyridinium chlorochromate. or pyridiniwn dichromate.

A hydroxyalkyl A-B group CHR⁷CR⁹OH or CR⁷(OH)CHR⁹ may be dehydrated to give the group CR⁷=CR⁹ by treatment with an acid anhydride such as acetic anhydride.

Methods for conversion of CR⁷=CR⁹ by reduction to CHR⁷CHR⁹ are well known to those skilled in the art, for example using hydrogenation over palladium on carbon as catalyst. Methods for conversion of CR⁷=CR⁹ to give the A-B group CR⁷(OH)CHR⁹ or CHR⁷CR⁹OH are well known to those skilled in the art for example by epoxidation and subsequent reduction by metal hydrides, hydration, hydroboration or oxymercuration.

An amide carbonyl group may be reduced to the corresponding amine using a reducing agent such as lithium aluminium hydride.

A hydroxy group in A or B may be converted to azido by activation and displacement e.g. under Mitsunobu conditions using hydrazoic acid or by treatment with diphenylphosphorylazide and base, and the azido group in turn may be reduced to amino by hydrogenation.

An example of a group Q¹ convertible to NR² R⁴ is NR^{2'}R^{4'} or halogen. Halogen may be displaced by an amine HNR^{2'}R^{4'} by a conventional alkylation.

When Q¹ Q² together form a protected oxo group this may be an acetal such as ethylenedioxy which can subsequently be removed by acid treatment to give a compound of formula (VI): 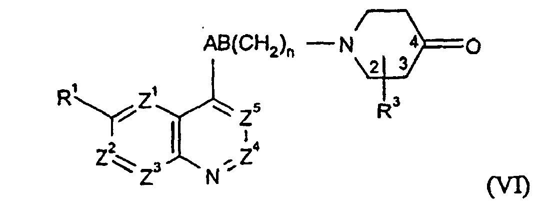 wherein the variables are as described for formula (I)

Intermediates of formula (VI) are novel and as such form part of the invention.

The ketone of formula (VI) is reacted with an amine HNR^{2'}R^{4'} by conventional reductive alkylation as described above for process variant (x).

Examples of groups Z^{1'}, Z^{2'}, Z^{3'}, Z^{4'}, Z⁵' convertible to Z¹, Z², Z³, Z⁴ and Z⁵ include CR^{1a'} where R^{1a'} is a group convertible to R^{1a}. Z^{1'}, Z^{2'}, Z^{3'}, Z^{4'} and Z^{5'} are preferably Z¹, Z², Z³, Z⁴ and Z⁵.

R^{1a'}, R^{1'} and R^{2'} are preferably R^{1a}, R¹ and R². R^{1'} is preferably methoxy. R^{2'} is preferably hydrogen. R^{3'} is R³ or more preferably hydrogen, vinyl, alkoxycarbonyl or carboxy. R^{4'} is R⁴ or more preferably H or an N-protecting group such as t-butoxycarbonyl, benzyloxycarbonyl or 9-fluorenylmethyloxycarbonyl.

Conversions of R^{1'}, R^{2'}, R^{3'} and R^{4'} and interconversions of R¹, R², R3 and R⁴ are conventional. In compounds which contain an optionally protected hydroxy group, suitable conventional hydroxy protecting groups which may be removed without disrupting the remainder of the molecule include acyl and alkylsilyl groups. N-protecting groups are removed by conventional methods.

For example R^{1'} methoxy is convertible to R^{1'} hydroxy by treatment with lithium and diphenylphosphine (general method described in Ireland *et al, J. Amer. Chem. Soc.,* 1973, 7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ alkoxy substituted by optionally N-substituted amino, piperidyl, guanidino or amidino.

R³ alkenyl is convertible to hydroxyalkyl by hydroboration using a suitable reagent such as 9-borabicyclo[3.3.1]nonane, epoxidation and reduction or oxymercuration.

R³ 1,2-dihydroxyalkyl can be prepared from R^{3'} alkenyl using osmium tetroxide or other reagents well known to those skilled in the art (see Advanced Organic Chemistry, *Ed. March, J*., John Wiley and Sons, 1985, p 732-737 and refs. cited therein) or epoxidation followed by hydrolysis (see Advanced Organic Chemistry, *Ed. March, J.* John Wiley and Sons, 1985, p 332,333 and refs. cited therein).

R³ vinyl can be chain extended by standard homologation, e.g. by conversion to hydroxyethyl followed by oxidation to the aldehyde, which is then subjected to a Wittig reaction.

Opening an epoxide-containing R^{3'} group with cyanide anion yields a CH(OH)-CH₂CN group.

Opening an epoxide-containing R^{3'} group with azide anion yields an azide derivative which can be reduced to the amine. Conversion of the amine to a carbamate is followed by ring closure with base to give the 2-oxo-oxazolidinyl containing R³ group.

Substituted 2-oxo-oxazolidinyl containing R³ groups may be prepared from the corresponding aldehyde by conventional reaction with a glycine anion equivalent, followed by cyclisation of the resulting amino alcohol (M. Grauert *et al, Ann. Chem*., 1985, 1817; Rozenberg *et al, Angew. Chem. Int. Ed. Engl.,* 1994, 33(1), 91). The resulting 2-oxo-oxazolidinyl group contains a carboxy group which can be converted to other R¹⁰ groups by standard procedures.

Carboxy groups within R³ may be prepared by Jones' oxidation of the corresponding alcohols CH₂OH using chromium acid and sulphuric acid in water/methanol (E.R.H. Jones *et al, J. Chem. Soc.,* 1946, 39). Other oxidising agents may be used for this transformation such as sodium periodate catalysed by ruthenium trichloride (G.F. Tutwiler *et al, J. Med. Chem*., 1987, *30*(6), 1094), chromium trioxidepyridine (G. Just *et al, Synth. Commun*., 1979, *9*(7), 613), potassium permanganate (D.E. Reedich *et al, J. Org. Chem.,1985,* 50(19), 3535), and pyridinium chlorochromate (D. Askin *et al, Tetrahedron Lett.,* 1988, 29(3), 277).

The carboxy group may alternatively be formed in a two stage process, with an initial oxidation of the alcohol to the corresponding aldehyde using for instance dimethyl sulphoxide activated with oxalyl chloride (N.Cohen *et al*, J. Am. Chem. Soc., 1983, 105, 3661) or dicyclohexylcarbodiimide (R.M.Wengler, Angew. Chim. Int. Ed. Eng., 1985, 24(2), 77), or oxidation with tetrapropylammonium perruthenate (Ley *et al*, J. Chem.Soc. Chem Commun.,1987,1625). The aldehyde may then be separately oxidised to the corresponding acid using oxidising agents such as silver (II) oxide (R.Grigg *et al*, J. Chem. Soc. Perkin1,1983, 1929), potassium permanganate (A.Zurcher, Helv. Chim. Acta., 1987, 70 (7), 1937), sodium periodate catalysed by ruthenium trichloride (T.Sakata *et al*, Bull. Chem. Soc. Jpn., 1988, 61(6), 2025), pyridinium chlorochromate (R.S.Reddy *et al*, Synth. Commun., 1988, 18(51), 545) or chromium trioxide (R.M.Coates *et al*, J. Am. Chem. Soc.,1982, 104, 2198).

An R³ CO₂H group may also be prepared from oxidative cleavage of the corresponding diol, CH(OH)CH₂OH, using sodium periodate catalysed by ruthenium trichloride with an acetontrile-carbontetrachloride-water solvent system (V.S.Martin *et al*, Tetrahedron Letters, 1988, *29*(22), 2701).

Other routes to the synthesis of carboxy groups within R³ are well known to those skilled in the art.

R³ groups containing a cyano or carboxy group may be prepared by conversion of an alcohol to a suitable leaving group such as the corresponding tosylate by reaction with para-toluenesulphonyl chloride (M.R. Bell, *J. Med. Chem*.,1970, 13, 389), or the iodide using triphenylphosphine, iodine, and imidazole (G. Lange, *Synth. Commun*., 1990, 20, 1473). The second stage is the displacement of the leaving group with cyanide anion (L.A. Paquette *et al, J. Org. Chem*., 1979, 44(25), 4603; P.A. *Grieco et al, J. Org. Chem*., 1988, 53(16), 3658. Finally acidic hydrolysis of the nitrile group gives the desired acids (H.Rosemeyer et al, Heterocycles, 1985, 23 (10), 2669). The hydrolysis may also be carried out with base e.g. potassium hydroxide (H.Rapoport, J. Org. Chem.,1958, 23, 248) or enzymatically (T. Beard et al, Tetrahedron Asymmetry, 1993, 4 (6), 1085).

Other functional groups in R³ may be obtained by conventional conversions of carboxy or cyano groups.

Tetrazoles are conveniently prepared by reaction of sodium azide with the cyano group (e.g. F. Thomas *et al, Bioorg. Med. Chem. Lett.,* 1996, 6(6), 631; K. Kubo *et al, J. Med. Chem*., 1993, 36, 2182) or by reaction of azidotri-n-butyl stannane with the cyano group followed by acidic hydrolysis (P.L. Ornstein, *J. Org. Chem*., 1994, 59, 7682 and *J. Med. Chem*, 1996, 39(11), 2219).

The 3-hydroxy-3-cyclobutene-1,2-dion-4-yl group (e.g. R.M. Soll, *Bioorg. Med. Chem. Lett*., 1993, 3(4), 757 and W.A. Kinney, *J. Med. Chem*., 1992, 35(25), 4720) can be prepared by the following sequence:- (1) a compound where R³ is (CH₂)ₙCHO (n = 0,1,2) is treated with triethylamine, carbon tetrabromide-triphenylphosphine to give initially (CH₂)ₙCH=CHBr; (2) dehydrobromination of this intermediate to give the corresponding bromoethyne derivative (CH₂)ₙC≡CBr (for this 2 stage sequence see D. Grandjean *et al, Tetrahedron Lett.,* 1994, 35(21), 3529); (3) palladium-catalysed coupling of the bromoethyne with 4-(1-methylethoxy)-3-(tri-n-butylstannyl)cyclobut-3-ene-1,2-dione (Liebeskind *et al, J. Org. Chem*., 1990, 55, 5359); (4) reduction of the ethyne moiety to -CH₂CH₂- under standard conditions of hydrogen and palladium on charcoal catalysis(see Howard *et al, Tetrahedron,* 1980, 36, 171); and finally (4) acidic hydrolysis of the methyl ethoxyester to generate the corresponding 3-hydroxy-3-cyclobutene-1,2-dione group (R.M. Soll, *Bioorg. Med. Chem. Lett*., 1993, 3(4), 757).

The tetrazol-5-ylaminocarbonyl group may be prepared from the corresponding carboxylic acid and 2-aminotetrazole by dehydration with standard peptide coupling agents such as 1,1'-carbonyldiimidazole (P.L. Ornstein *et al, J. Med Chem,* 1996, 39(11), 2232).

The alkyl- and alkenyl-sulphonylcarboxamides are similarly prepared from the corresponding carboxylic acid and the alkyl- or alkenyl-sulphonamide by dehydration with standard peptide coupling agents such as 1,1'-carbonyldiimidazole (P.L. Ornstein *et al, J. Med. Chem*., 1996, 39(11), 2232).

The hydroxamic acid groups are prepared from the corresponding acids by standard amide coupling reactions e.g. N.R. *Patel et al, Tetrahedron,* 1987, 43(22), 5375.

2,4-Thiazolidinedione groups may prepared from the aldehydes by condensation with 2.4-thiazolidinedione and subsequent removal of the olefinic double bond by hydrogenation.

The preparation of S-oxo-1,2,4-oxadiazoles from nitriles is decribed by Y. Kohara *et al*, *Bioorg. Med. Chem.* Lett., 1995, 5(17), 1903.

1,2,4-Triazol-5-yl groups may be prepared from the corresponding nitrile by reaction with an alcohol under acid conditions followed by reaction with hydrazine and then an R¹⁰-substituted activated carboxylic acid (see J.B. Polya in "Comprehensive Heterocyclic Chemistry" Edition 1, p762, Ed A.R. Katritzky and C.W. Rees, Pergamon Press, Oxford, 1984 and J.J. Ares *et al, J. Heterocyclic Chem.,* 1991, 28(5), 1197).

Other substituents on R³ alkyl or alkenyl may be interconverted by conventional methods, for example hydroxy may be derivatised by esterification, acylation or etherification. Hydroxy groups may be converted to halogen, thiol, alkylthio, azido, allcylcarbonyl, amino, aminocarbonyl, oxo, alkylsulphonyl, alkenylsulphonyl or aminosulphonyl by conversion to a leaving group and substitution by the required group or oxidation as appropriate or reaction with an activated acid, isocyanate or alkoxyisocyanate. Primary and secondary hydroxy groups can be oxidised to an aldehyde or ketone respectively and alkylated with a suitable agent such as an organometallic reagent to give a secondary or tertiary alcohol as appropriate.

An NH₂ substituent on piperidine is converted to NR²R⁴ by conventional means such as amide or sulphonamide formation with an acyl derivative X⁴-X³-X²-COW or X⁴-X³-X²-SO₂W, for compounds where U is CO or SO₂ or, where U is CH₂, alkylation with an alkyl halide R⁴-halide in the presence of base, acylation/reduction with an acyl derivative X⁴-X³-X²-COW or reductive alkylation with an aldehyde X⁴-X³-X²-CHO.

Where one of R³ and R⁶, R⁷, R⁸ or R⁹ contains a carboxy group and the other contains a hydroxy or amino group they may together form a cyclic ester or amide linkage. This linkage may form spontaneously during coupling of the compound of formula (IV) and the piperidine moiety or in the presence of standard peptide coupling agents.

Examples of R^{4'} convertible to R⁴ include -CH₂-CO₂H which may be reacted with an amine X⁴-NH₂ in the presence of a coupling agent such as 1-(3-dimethylaminopropyl)-3-ethylcarbodiimide (EDC) to give R⁴=X⁴-NH-CO-CH₂-. This reaction may be carried out on the compound of formula (V) or after coupling (IV) and (V). Cyclisation of the hydroxy and amino (R¹⁴ and R¹³) groups may be effected with phosgene in base.

Interconversion of substituents on the aromatic group X⁴ may be carried out conventionally at any appropriate point in the synthesis. For example pyridine may be oxidised to the the N-oxide with and oxidising agent such as meta-chloroperbenzoic acid, hydrogen peroxide or t-butyl hydroperoxide after protection of any other nitrogen atoms. N-oxides may be rearranged in trifluoroacetic acid to give the hydroxypyridines.

It will be appreciated that under certain circumstances interconversions may interfere, for example, hydroxy groups in A or B and the piperidine substituent NH₂ will require protection e.g. as a carboxy- or silyl-ester group for hydroxy and as an acyl derivative for piperidine NH₂, during conversion of R^{1'}, R^{2'}, R^{3'} or R^{4'}, or during the coupling of the compounds of formulae (IV) and (V).

Compounds of formulae (IV) and (V) are known compounds, (see for example Smith *et al, J. Amer. Chem. Soc.,* 1946, 68, 1301) or prepared analogously.

Compounds of formula (IV) where X is CR⁶R⁷SO₂W may be prepared by a route analogous to that of Ahmed El Hadri *et al, J. Heterocyclic Chem*., 1993, 30(3), 631. Thus compounds of formula (IV) where X is CH₂SO₂OH may be prepared by reacting the corresponding 4-methyl compound with N-bromosuccinimide, followed by treatment with sodium sulfite. The leaving group W may be converted to another leaving group W, e.g. a halogen group, by conventional methods.

The isocyanate of formula (IV) may be prepared conventionally from a 4-amino derivative such as 4-amino-quinoline, and phosgene, or phosgene equivalent (eg triphosgene) or it may be prepared more conveniently from a 4-carboxylic acid by a "one-pot" Curtius Reaction with diphenyl phosphoryl azide (DPPA) [see T. Shiori et al. *Chem. Pharm. Bull*. **35**, 2698-2704 (1987)].

The 4-amino derivatives are commercially available or may be prepared by conventional procedures from a corresponding 4-chloro or 4-trifluoromethanesulphonate derivative by treatment with ammonia (O.G. Backeberg et. al., J. Chem Soc., 381, 1942) or propylamine hydrochloride (R. Radinov et. al., Synthesis, 886, 1986).

4-Alkenyl compounds of formula (IV) may be prepared by conventional procedures from a corresponding 4-halogeno-derivative by e.g. a Heck synthesis as described in e.g. *Organic Reactions,* 1982, 27, **345.**

4-Halogeno derivatives of compounds of formula (IV) are commercially available, or may be prepared by methods known to those skilled in the art. A 4-chloroquinoline is prepared from the corresponding quinolin-4-one by reaction with phosphorus oxychloride (POCl₃) or phosphorus pentachloride, PCl₅. A 4-chloroquinazoline is prepared from the corresponding quinazolin-4-one by reaction with phosphorus oxychloride (POCl₃) or phosphorus pentachloride PCl₅. A quinazolinone and quinazolines may be prepared by standard routes as described by T.A. Williamson in *Heterocyclic Compounds,* 6, 324 (1957) Ed. R.C. Elderfield.

Activated carboxy derivatives X=A'COW of formula (IV) may be prepared from X=A'CO₂H derivatives in turn prepared from CO₂H derivatives by conventional methods such as homologation.

4-Carboxy derivatives of compounds of formula (IV) are commercially available or may be prepared by conventional procedures for preparation of carboxy heteroaromatics well known to those skilled in the art. For example, quinazolines may be prepared by standard routes as described by T.A. Williamson in *Heterocyclic Compounds*, 6, 324 (1957) Ed. R.C. Elderfield. These 4-carboxy derivatives may be activated by conventional means, e.g. by conversion to an acyl halide or anhydride.

4-Carboxy derivatives such as esters may be reduced to hydroxymethyl derivatives with for example lithium aluminium hydride. Reaction with mesyl chloride and triethylamine would give the mesylate derivative.

A 4-oxirane derivative of compounds of formula (IV) is conveniently prepared from the 4-carboxylic acid by first conversion to the acid chloride with oxalyl chloride and then reaction with trimethylsilyldiazomethane to give the diazoketone derivative. Subsequent reaction with 5M hydrochloric acid gives the chloromethylketone. Reduction with sodium borohydride in aqueous methanol gives the chlorohydrin which undergoes ring closure to afford the epoxide on treatment with base, e.g. potassium hydroxide in ethanol-tetrahydrofuran.

Alternatively and preferably, 4-oxirane derivatives can be prepared from bromomethyl ketones which can be obtained from 4-hydroxy compounds by other routes well known to those skilled in he art. For example, hydroxy compounds can be converted to the corresponding 4-trifiuoromethanesuIphonafes by reaction with trifluoromethanesulphonic anhydride under standard conditions (see K. Ritter, Synthesis, 1993, 735). Conversion into the corresponding butyloxyvinyl ethers can be achieved by a Heck reaction with butyl vinyl ether under palladium catalysis according to the procedure of W. Cabri *et al,* J, Org. Chem, 1992, 57 (5), 1481. (Alternatively, the same intermediates can be attained by Sdlle coupling of the trifluoromethanesulphonates or the analogous chloro derivatives with (1-ethoxyvinyl)tributyl tin, T. R. Kelly, J. Org. Chem., 1996, **61**, 4623.) The atkyloxyvinyl ethers are then converted into the corresponding bromomethylketones by treatment with N-bromosuccinimide in aqueous tetrahydrofuran in a similar manner to the procedures of J. F. W. Keana, J. Org. Chem., 1983, **48**, 3621 and T. R. Kelly, J. Org. Chem., 1996, **61**, 4623.

The 4-hydroxyderivatives can be prepared from an aminoaromatic by reaction with methylpropiolate and subsequent cyclisation, analogous to the method described in N. E. Heindel et al, J. Het. Chem., 1969,**6**, 77. For example, 5-amino-2-methoxy pyridine can be converted to 4-hydroxy-6-methoxy-[1,5]naphthyridine using this method.

If a chiral reducing agent such as (+) or (-)-B-chlorodiisopinocamphenylborane ['DIP-chloride'] is substituted.for sodium borohydride, the prochiral chloromethylketone is converted into the chiral chlorohydrin with ee values generally 85-95% [see C. Bolm *et al, Chem. Ber.* 125, 1169-1190, (1992)]. Recrystallisation of the chiral epoxide gives material in the mother liquor with enhanced optical purity (typically ee 95%).

The (*R)*-epoxide, when reacted with a piperidine derivative gives ethanolamine compounds as single diastereomers with (R)-stereochemistry at the benzylic position.

Alternatively, the epoxide may be prepared from the 4-carboxaldehyde by a Wittig approach using trimethylsulfonium iodide [see G.A. Epling and K-Y Lin, *J. Het. Chem*., 1987, *24*, 853-857], or by epoxidation of a 4-vinyl derivative.

Pyridazines may be prepared by routes analogous to those described in Comprehensive Heterocyclic Chemistry, Volume 3, Ed A.J. Boulton and A. McKillop and napthyridines may be prepared by routes analogous to those described in Comprehensive Heterocyclic Chemistry, Volume 2, Ed A.J. Boulton and A. McKillop.

4-Hydroxy-1,5-naphthyridines can be prepared from 3-aminopyridine derivatives by reaction with diethyl ethoxymethylene malonate to produce the 4-hydroxy-3-carboxylic acid ester derivative with subsequent hydrolysis to the acid, followed by thermal decarboxylation in quinoline (as for example described for 4-Hydroxy-[1,5]naphthyridine-3-carboxylic acid, J. T. Adams *et al., J.Amer.Chem.Soc*., 1946, **68,** 1317). A 4-hydroxy-[1,5]naphthyridine can be converted to the 4-chloro derivative by heating in phosphorus oxychloride, or to the 4-methanesulphonyloxy or 4-trifluoromethanesulphonyloxy derivative by reaction with methanesulphonyl chloride or trifluoromethanesulphonic anhydride, respectively, in the presence of an organic base. A 4-amino 1,5-naphthyridine can be obtained from the 4-chloro derivative by reaction with n-propylamine in pyridine.

Similarly, 6-methoxy-1,5-naphthyridine derivatives can be prepared from 3-amino-6-methoxypyridine.

1,5-Naphthyridines may be prepared by other methods well known to those skilled in the art (for examples see P.A. Lowe in "Comprehensive Heterocyclic Chemistry" Volume 2, p581-627, Ed A.R. Katritzky and C.W. Rees, Pergamon Press, Oxford, 1984).

The 4-hydroxy and 4-amino-cinnolines may be prepared following methods well known to those skilled in the art [see A.R. Osborn and K. Schofield, *J. Chem. Soc.* 2100 (1955)]. For example, a 2-aminoacetopheneone is diazotised with sodium nitrite and acid to produce the 4-hydroxycinnoline with conversion to chloro and amino derivatives as described for 1,5-naphthyridines.

The compounds of formula (V) are either commercially available or may be prepared by conventional methods.

For compounds of formula (V), suitable amines (Q¹ = NH2 and Q² = H) may be prepared from the corresponding 4-substituted piperidine acid or alcohol. In a first instance, an N-protected piperidine containing an acid bearing substituent, can undergo a Curtius rearrangement and the intermediate isocyanate can be converted to a carbamate by reaction with an alcohol. Conversion to the amine may be achieved by standard methods well known to those skilled in the art used for amine protecting group removal. For example, an acid substituted N-protected piperidine can undergo a Curtius rearrangement e.g. on treatment with diphenylphosphoryl azide and heating, and the intermediate isocyanate reacts in the presence of 2-trimethylsilylethanol to give the trimethylsilylethylcarbamate (T.L. Capson & C.D. Poulter, *Tetrahedron Lett.,* 1984, 25, 3515). This undergoes cleavage on treatment with tetrabutylammonium fluoride to give the 4-amine substituted N-protected piperidine.

In a second instance, an N-protected piperidine containing an alcohol bearing substituent undergoes a Mitsunobu reaction (for example as reviewed in Mitsunobu, *Synthesis,* (1981), 1), for example with succinimide in the presence of diethyl azodicarboxylate and triphenylphosphine to give the phthalimidoethylpiperidine. Removal of the phthaloyl group, for example by treatment with methylhydrazine, gives the amine of formula (V).

Compounds of formula (V) containing an R^{3'} in the 4-position may be made from the corresponding 4-amino, 4-alkoxycarbonyl piperidine derivative from the commercially available acid. Standard transformations on this ester or acid yield the required R^{3'} group.

Addition of YCH₂ to piperidine may be via standard alkylations using for example an alkyl halide such as YCH₂Br or reductive alkylation with an aldehyde YCHO. Addition of acyl groups Y to piperidine eg carbonyl or sulphonyl may be carried out by conventional amide/sulponamide formation.

R⁴-halides, acyl derivative X⁴-X³-X²-COW and X⁴-X³-X²-SO₂W or aldehydes X⁴-X³-X²-CHO are commercially available or are prepared conventionally. The aldehydes may be prepared by partial reduction of the corresponding ester (see *Reductions by the Alumino- and Borohydrides in Organic Synthesis*, 2nd ed., Wiley, N.Y., 1997; *JOC,* 3197, 1984; *Org. Synth. Coll.,* 102, 1990; 136, 1998; *JOC*, 4260, 1990; *TL,* 995, 1988; *JOC,* 1721, 1999; *Liebigs Ann.*/*Recl.,* 2385, 1997; *JOC,* 5486, 1987).with lithium aluminium hydride or di-isobutylaluminium hydride or more preferably by reduction to the alcohol, with lithium aluminium hydride or sodium borohydride, followed by oxidation to the aldehyde with manganese (IT) dioxide. The aldehydes may also be prepared from carboxylic acids in two stages by conversion to a mixed anhydride for example by reaction with isobutyl chloroformate followed by reduction with sodium borohydride (R. J. Alabaster et al., Synthesis, 598, 1989) to give the benzylic alcohols and then oxidation with a standard oxidising agent such as pyridinium dichromate or manganese (II) dioxide. Acyl derivative may be prepared by activation of the corresponding ester. R⁴-halides such as bromides may be prepared from the alcohol R⁴OH by reaction with phosphorus tribromide in DCM/triethyiamine. Where X² is CO and X³ is NR¹³ the R⁴-halide may be prepared by coupling an X⁴-NH₂ amine and bromoacetyl bromide.

The amines R^{2'}R^{4'}NH are available commercially or prepared conventionally. For example amines X⁴-X³-X²-CH₂NH₂ may be prepared from a bromomethyl derivative by reaction with sodium azide in dimethylformamide (DMF), followed by hydrogenation of the azidomethyl derivative over palladium-carbon. An alternative method is to use potassium phthalimide/DMF to give the phthalimidomethyl derivative, followed by reaction with hydrazine in DCM to liberate the primary amine. Amines where X² is CO and X³ is NR¹³ may be prepared by reacting an N-protected glycine derivative HO₂C-X¹-NH₂ with X⁴-NH₂ by conventional coupling using eg EDC.

Conversions of R^{1a'}, R^{1'}, R^{2'}, R^{3'} and R⁴' may be carried out on the intermediates of formulae (IV), and (V) prior to their reaction to produce compounds of formula (I) in the same way as described above for conversions after their reaction.

Further details for the preparation of compounds of formula (I) are found in the examples.

The compounds of formula (I) may be prepared singly or as compound libraries comprising at least 2, for example 5 to 1,000 compounds, and more preferably 10 to 100 compounds of formula (I). Libraries of compounds of formula (I) may be prepared by a combinatorial "split and mix" approach or by multiple parallel synthesis using either solution phase or solid phase chemistry, by procedures known to those skilled in the art.

Thus according to a further aspect of the invention there is provided a compound library comprising at least 2 compounds of formula (I) or pharmaceutically acceptable derivatives thereof.

Novel intermediates of formulae (IV) and (V) are also part of this invention. The antibacterial compounds according to the invention may be formulated for administration in any convenient way for use in human or veterinary medicine, by analogy with other antibacterials.

The pharmaceutical compositions of the invention include those in a form adapted for oral, topical or parenteral use and may be used for the treatment of bacterial infection in mammals including humans.

The composition may be formulated for administration by any route. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also cotitain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl *p*-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable derivative thereof is administered in the above-mentioned dosage range.

The compound of formula (I) may be the sole therapeutic agent in the compositions of the invention or a combination with other antibacterials. If the other antibacterial is a β-lactam then a β-lactamase inhibitor may also be employed.

Compounds of formula (I) are active against a wide range of organisms including both Gram-negative and Gram-positive organisms.

All publications, including but not limited to patents and patent applications, cited in this specification are herein incorporated by reference as if each individual publication were specifically and individually indicated to be incorporated by reference herein as though fully set forth.

The following examples illustrate the preparation of certain compounds of formula (I) and the activity of certain compounds of formula (I) against various bacterial organisms.

### EXAMPLES

### Example 1.2-{1-[(R)-2-Hydroxy-2-(6-methoxy-quinolin-4-yl)-ethyl]-piperidin-4-ylamino}-N-phenyl-acetamide dioxalate

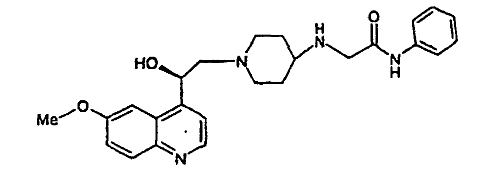

### (a) [R]-2-(6-Methoxyquinolin-4-yl)oxirane

A solution of 6-methoxyquinoline-4-carboxylic acid (10g) in dichloromethane was heated under reflux with oxalyl chloride (5ml) and dimethylformamide (2 drops) for 1 hour and evaporated to dryness. The residue, in dichloromethane (100ml) was treated with a 2M solution of trimethylsilyldiazomethane in hexane (50ml) and stirred at room temperature for 18 hours. 5M Hydrochloric acid (150ml) was added and the solution was stirred at room temperature for 3 hours. It was basified with sodium carbonate solution, extracted with ethyl acetate and chromatographed on silica gel eluting with ethyl acetate-hexane to give the chloromethyl ketone (4.2g). A batch of the chloromethyl ketone (20g) was reduced with (+)-B-chlorodiisopinocamphenylborane (40g) in dichloromethane (400ml) at room temperature for 18 hours followed by treatment with diethanolamine (30 g) for 3 hours. The product was chromatographed on silica gel eluting with ethyl acetate-hexane to give the chloroalcohol (16.8g), which was dissolved in tetrahydrofuran (100 ml) and reacted with sodium hydroxide (2.6g) in water (13ml) for 1.5 hours. The reaction mixture was evaporated to dryness and chromatographed on silica gel eluting with ethyl acetate - hexane to give the title compound as a solid (10.4 g) (84% ee by chiral HPLC). Recrystallisation from ether-pentane gave mother-liquor (7.0 g) (90% ee).
MS (+ve ion electrospray) m/z 202 (MH+)
The absolute stereochemistry was defined to be (R) by an NMR study on the Mosher's esters derived from the product obtained by reaction with 1-t-butylpiperazine.

### (b) 1-[2-(R)-Hydroxy-2-(6-methoxyquinolin-4-yl)]ethyl-4-oxo piperidine, ethylene ketal.

[R]-2-(6-Methoxyquinolin-4-yl)oxirane (1a) (470 mg) and 1,4-dioxa-8-azaspiro-[4,5]-decane (0.33 ml) were dissolved in dry dichloromethane (5 ml) and ytterbium triflate (30 mol%) was added. The mixture was stirred for 6 hours, filtered through celite, evaporated and chromatographed on silica gel (dichloromethane then methanol-dichloromethane).
MS (+ve ion electrospray) m/z 345 (MH+).

### (c) 4-Oxo-1-[2-(R)-hydroxy-2-(6-methoxyquinolin-4-yl)]ethylpiperidine.

The ketal (1b) was cleaved by treatment with 5M HCl (10 ml) in acetone (20 ml) at 60°C overnight. The mixture was basified with sodium bicarbonate solution and concentrated. Extraction into dichloromethane, evaporation and chromatography on silica gel (dichloromethane then methanol-dichloromethane) gave a yellow gum (482 mg).

### (d) Title compound

The ketone (1c) (0.113g), 2-amino-N-phenyl acetamide (prepared from N-t-Boc-glycine N-hydroxysuccinimide ester and aniline followed by deprotection in trifluoroacetic acid-dichloromethane) (0.065g) and sodium triacetoxyborohydride (0.08g) were added together to dry methanol (2ml), followed by 3A molecular sieves and the mixture was stirred slowly at room temperature for 18 hours. It was filtered, evaporated, and treated with sodium carbonate solution. The mixture was extracted with dichloromethane, dried, evaporated and chromatographed on silica gel (ethyl acetate then methanol-dichloromethane) to afford the title compound (0.093g).
MS (+ve ion electrospray) m/z 435 (MH+).
¹H NMR (CDCl₃) δ: 1.50-2.90 (10H, m), 3,25 (2H, m), 3.40 (2H, s), 3.95 (3H, s), 5.45 (1H, m) 7.10 (1H, brt), 7.15 (1H, d), 7.30-7.70 (7H, m), 8.05 (1H, d), 8.78 (1H, d), 9.30 (1H, brs).
The free base in dichloromethane was treated with 2 molar equivalents of oxalic acid in ether and the resulting solid was collected, triturated with ether, to afford the dioxalate salt as a white solid.

### Example 2. cis-2-{1-(2-(R)-Hydroxy-2-(6-methoxy-quinolin-4-yl)-ethyl]-3-(R/S)-ethoxycarbonyl-piperidin-4-(S/R)-ylamino}-N-phenyl-acetamide dioxalate

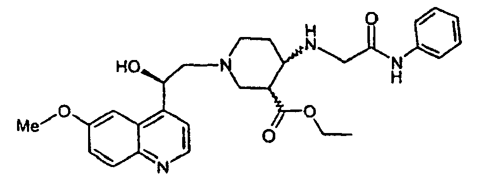

### (a) 4-Benzylamino-1-tert-butoxycarbonyl-3-ethoxycarbonyl-1,2,5,6-tetrahydropyridine

A solution of 1-*tert*-butoxycarbonyl-3-ethoxycarbonylpiperidin-4-one (prepared from 3-ethoxycarbonytpiperidin-4-one and di-*tert*-butyl-dicarbonate in dichloromethane and triethylamine) (25g) and benzylamine (10.85g) in toluene was heated under reflux in a Dean and Stark apparatus for 18 hours and then evaporated to dryness to give an oil.

### (b) cis-4-(S/R)-Benzylamino-1-tert-butoxycarbonyl-3-(R/S)-ethoxycarbonylpiperidine

The enamine (2a) (25g) in ethanol (300ml) was hydrogenated over platinum oxide (1.5g) for 4 days, filtered, and evaporated to dryness. It was chromatographed on silica gel (ethyl acetate-hexane) to afford the title compound as an oil.
MS (+ve ion electrospray) m/z 363 (MH+).

### (c) cis-4-(S/R)-Amino-1-tert-butoxycarbonyl-3-(R/S)-ethoxycarbonylpiperidine

The amine (2b) (4g) in ethanol (80ml) containing acetic acid (0.73g) was hydrogenated at 50psi (Parr reaction vessel) over 10% palladium-carbon (1g) for 18 hours, filtered and evaporated to dryness to afford the acetate salt of the title compound as a white solid (3g).
MS (+ve ion electrospray) m/z 273 (MH+).
It was converted to the oily free base by extraction using dichloromethane-sodium carbonate and drying over sodium sulfate.

### (d) cis-[1-tert-Butoxycarbonyl-3-(R/S)-ethoxycarbonyl-piperidin-4-(S/R)-ylamino]-N-phenyl-acetamide

A solution of the amine (2c) (0.8g) in dry dimethylformamide (5ml) containing anhydrous potassium carbonate (1.5g) was treated with 2-bromo-N-phenylacetamide (0.664g) and the mixture was stirred at room temperature for 18 hours. The mixture was evaporated to dryness, brine was added and the solution was extracted with dichloromethane. The organic fraction was dried, evaporated, and chromatographed on silica gel (ethyl acetate-hexane) to afford an oil (0.82g).
MS (+ve ion electrospray) m/z 406 (MH+).

### (e) cis-[3-(R/S)-Ethoxycarbonyl-piperidin-4-(S/R)-ylamino]-N-phenyl-acetamide

A solution of the acetamide (2d) (0.81g) in dichloromethane (30ml) was treated with trifluoroacetic acid (30ml) and the solution was left at room temperature for 2.5 hours. It was evaporated to dryness, sodium carbonate solution was added, and it was extracted with chloroform, dried, and evaporated to give an oil (0.6g).
MS (+ve ion electrospray) m/z 306 (MH+).

### (f) Title compound

A solution of [R]-2-(6-methoxyquinolin-4-yl)oxirane (1a) (0.13g) and the piperidine (2e) (0.197g) in acetonitrile (3ml) containing lithium perchlorate (0.068g) was left at room temperature for 5 days and evaporated to dryness. The product was dissolved in dichloromethane, washed with sodium carbonate, dried over sodium sulfate, and chromatographed on silica gel (ethyl acetate-hexane) to afford a foam (0.162g).
MS (+ve ion electrospray) m/z 507 (MH+). The free base was treated with 2 molar equivalents of oxalic acid in ether and the resulting solid was collected, triturated with ether, to afford the dioxalate salt as a white solid.

### Example 3. cis-2-{1-[2-(R)-Hydroxy-2-(6-methoxy-quinolin-4-yl)-ethyl]-3-(R/S)-bydroxymethyl-piperidin-4-(SIR)-ylamino }-N-pbenyl-acetamide dioxalate

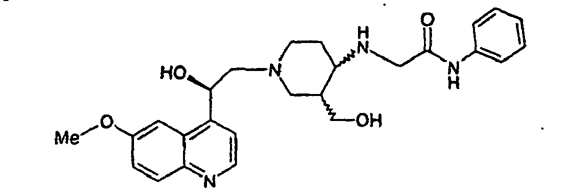

The ester Example (2) (0.095g) in dry tetrahydrofuran (3ml) at 0°C was treated with lithium aluminium hydride (0.36ml of a 1M solution in tetrahydrofuran) for 4 hours and then quenched by the addition of 2M sodium hydroxide. Dichloromethane and sodium sulfate were added and the solution was filtered and evaporated to dryness. The product was chromatographed on silica gel (methanol-dichloromethane) to afford the title compound (0.031g), as the oily free base.
MS (+ve ion electrospray) m/z 464 (MH+).
¹H NMR (CDCl₃) δ: 1.70-3.10 (10H, m), 3,40 (2H, brs), 3.95 (3H, d), 4.10 (2H,m), 5.45 (1H, m) 7.15 (2H, m), 7.30 (4H, m), 7.60 (3H,m), 8.05 (1H, d), 8.76 (1H, d), 9.15 (1H, brd).
The free base was converted to the dioxalate salt in the normal manner, affording a white solid.

### Example 4. cis-2-{1-[2-(R)-Hydroxy-2-(6-methoxy-quinolin-4-yl)-ethyl]-3-(R/S)-aminocarbonyl-piperidin-4-(S/R)-ylamino}-N-phenyl-acetamide dioxalate.

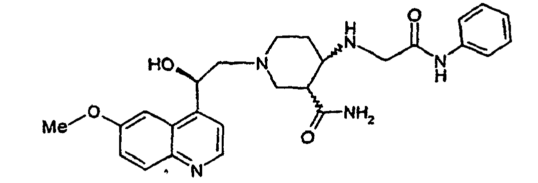

The ester Example (3) (0.035g) in methanol (2ml) was heated with ammonia (2ml) and sodium cyanide (1mg) at 50°C (sealed bomb) for 72 hours and evaporated to dryness. Chromatography on silica gel (ethyl-acetate then methanol-dichloromethane) gave the title compound (0.015g), as the free base.
MS (+ve ion electrospray) m/z 478 (MH+).
¹H NMR (CDCl₃) δ: 1.60-3.30 (10H, m), 3,50 (2H, m), 3.90 (3H, d), 5.45 (1H, m) 7.15 (2H, m), 7.30 (4H, m), 7.60 (3H,m), 8.0 (1H, m), 8.70 (1H, m), 9.40 (1H, brd).
The free base in dichloromethane-ether was converted to the dioxalate salt in the normal manner, affording a white solid.

### Example 5 cis-3-(R/S)-Ethoxycarbonyl-4-(S/R)-(phenylcarbamoylmethyl-amino)-piperidine-1-carboxylic acid (6-methoxy-quinolin-4-yl)-amide oxalate.

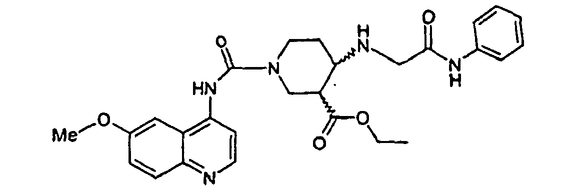

### (a) 4-Atnino-6-methoxyquinoline

Curtius rearrangement of 6-methoxyquinoline-4-carboxylic acid (4g, 20mmol) with diphenylphosphoryl azide (4.3ml, 20mmol) and triethylamine(3.5ml) in *tert*-butanol (25ml) at 85°C gave, after chromatography on silica gel (ethyl acetate-dichloromethane) the N-*tert*-butoxycarbamate (2.47g). Treatment with aqueous hydrochloric acid at reflux, followed by basification and extraction with ethyl acetate gave the 4-aminoquinoline (0.74g).
This compound may also be prepared from 4-hydroxy-6-methoxyquinoline by chlorination with phosphorus oxychloride, to give the 4-chloroquinoline, followed by treatment with n-propylamine hydrochloride.

### (b) 4-(1-Imidazolylcarbonyl)amino-6-methoxyquinoline

A suspension of 4-amino-6-methoxyquinoline (5a) (2.0g,11 mmol) in ethanol-free chloroform (60ml) was treated with 4-N,N-dimethylaminopyridine (1.4g, 11.4 mmol) and carbonyl diimidazole (2.6g, 15.9 mmol). The suspension became clear over 1 hour then precipitation occurred. After a further 2 hours, filtration afforded the product as a white solid which was dried *in vacuo* (1.8g, 59%).

### (c) cis-3-(R/S)-Ethoxycarbonyl-4-(S/R)-(phenylcarbamoylmethyl-amino)-piperidine-1-carboxylic acid (6-methoxy-quinolin-4-yl)-amide

A solution of 4-(1-imidazolylcarbonyl)amino-6-methoxyquinoline (5b) and amine (2e) (0.211g) in dry DMF (0.5ml) was heated at 50°C for 3 hours and evaporated to dryness. Sodium carbonate and brine were added and the mixture was extracted with dichloromethane, washed with water, dried, evaporated and chromatographed on silica gel (ethyl acetate) to give an oil (0.092g).
MS (+ve ion electrospray) m/z 506 (MH+).
The free base in dichloromethane-ether was converted to the oxalate salt in the normal manner, affording a white solid.

### Example 6 cis-3-(R/S)-Hydroxymethyl-4-(S/R)-(phenylcarbamoylmethyl-amino)-piperidine-1-carboxylic acid (6-methoxy-quinolin-4-yl)-amide oxalate.

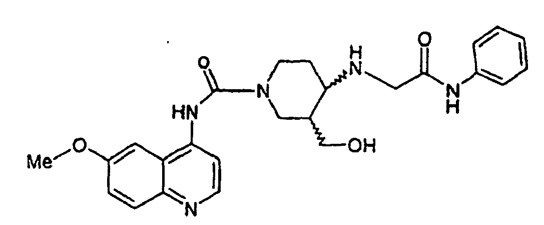

This was prepared from ester Example (5c) (0.096g) by reduction with lithium aluminium hydride by the Method of Example 3 to give an oil (0.031g)
¹H NMR (CDCl₃) δ: 1.55 (1H, m), 1.85 (1H, m), 2.40 (1H, m), 2.80-3.15 (3H, m), 3.40 (2H, d), 3.75 (2H, brd), 3.85 (3H, s), 4.05 (1H, m), 4.37 (1H, brd), 4.50 (1H, brd), 7.10 (3H, m), 7.15 (1H, d), 7.30 (3H, t), 7.60 (2H, d), 8.05 (1H, d), 8.35 (1H, d), 9.00 (1H, brs).
MS (+ve ion electrospray) m/z 464 (MH+).
The free base in dichloromethane-ether was converted to the oxalate salt in the normal manner, affording a white solid.

### The following Examples 7-12 were prepared by the Method of Example 1:

### Example7 2-{1-[(R)-2-Hydroxy-2-(6-methoxy-quinolin-4-yl)-ethyl]-piperidin-4-ylamino}-N-(3-pyridyl)-acetamide dioxalate

MS (+ve ion electrospray) m/z 436 (MH+).

### Example 8 2-{1-[(R)-2-Hydroxy-2-(6-metboxy-quinolin-4-yl)-ethyl]-piperidin-4-ylamino}-N-(4-pyridyl)-acetamide dioxalate

MS (+ve ion electrospray) mlz 436 (MH+).

### Example 9 2-{1-[(R)-2-Hydroxy-2-(6-methoxy-quinolin-4-yl)-ethylJ-piperidin-4-ylaminol-N-(1,3-dimethylpyrazoll-5-yl)-acetamide dioxalate

MS (+ve ion electrospray) m/z 453 (MH+).

### Example 10 2-{1-[(R)-2-Hydroxy-2-(6-methoxy-quinolin-4-yl)-ethyl]-piperidin-4-ylamino}-N-(1-methylpyrazol-5-yl)-acetamide dioxalate

MS (+ve ion electrospray) m/z 439 (MH+).

### Example 11 2-{1-[(R)-2-Hydroxy-2-(6-methoxy-quinolin-4-yl)-ethyl]-piperidin-4-ylamino}-N-methyl-N-phenyl-acetamide dioxalate

MS (+ve ion electrospray) m/z 449 (MH+).

### Example 12 2-{1-[(R)-2-Hydroxy-2-(6-methoxy-quinolin-4-yl)-ethyl]-piperidin-4-yl-N-methylamino}-N-phenyl-acetamide dioxalate

MS (+ve ion electrospray) m/z 449 (MH+).

### Example 13. 2-{1-[(R)-2-Hydroxy-2-(6-methoxy-quinolin-4-yl)-ethyl]-piperidin-4-ylamino}-N-pyridin-2-yl-acetamide dioxalate

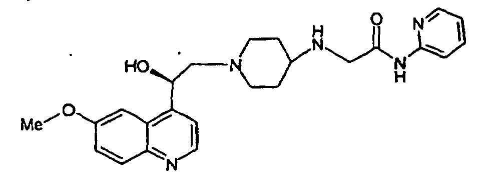

### (a) N-(2-Pyridyl)-2-bromo acetamide

To a solution of 2-aminopyridine (0.38 g, 4 mmol) in tetrahydrofuran (7ml) was added triethylamine (0.7ml, 5 mmol) and bromoacetyl bromide (0.44ml, 5 mmol) dropwise. The mixture was shaken overnight and then quenched with sodium bicarbonate solution( 20ml). Ethyl acetate (20m1) was added and the layers separated, the organic layer was evaporated and the residue chromatographed on silica gel (methanol-dichloromethane) to give a tan solid (300mg; 35%).

### (b) 4-tert-Butoxycarbonylamino-1-[2-(R)-hydroxy-2-(6-methoxyquinolin-4-yl)]ethylpiperidine.

To a stirred solution of [R]-2-(6-methoxyquinolin-4-yl)oxirane (1a) (8.07g, 40.3 mmol) and lithium perchlorate (4.44g, 40.3 mmol) in anhydrous DMF (100ml) was added 4-*tert*-butoxycarbonylaminopiperidine hydrochloride (11.0g, 45.7 mmol) and potassium carbonate (6.72g, 48.4 mmol). The mixture was heated at 90°C for 26 hours , then cooled, filtered and evaporated. The residue was dissolved in ethyl acetate, washed with water, dried and evaporated. The crude product was chromatographed on silica gel (methanol-dichloromethane) to give a gum (11.11g). MS (+ve ion electrospray) m/z 402 (MH+).

### (c) 4-Amino-1-[2-(R)-hydroxy-2-(b-methoxyquinolin-4-yl)Jethylpiperidine.

The *tert*-butoxycarbonylamino compound (13b) (11.11g, 27.7 mmol) was dissolved in dichloromethane (30ml), cooled in ice and treated with trifluoroacetic acid (30ml). The solution was stirred at room temperature for 1.5 hours, then evaporated *in vacuo.* After addition of toluene (50ml) and re-evaporation, the residue was treated with a 4M solution of hydrogen chloride in 1,4-dioxan (30ml). The resulting solid was triturated, filtered off and washed with ether. This was then recrystallised by dissolving in hbt methanol, concentrating the solution and diluting with dichloromethane, to give the trihydrochloride salt (9.4g). This was dissolved in water, basified to pH9 and evaporated to dryness. The residue was extracted several times with 10% methanol-dichloromethane (total 600ml). The extracts were filtered and evaporated to give the free base as a semisolid foam (6.45g).
MS (+ve ion electrospray) m/z 302 (MH+),

### (d) Title compound

N-(2-Pyridyl)-2-bromo acetamide (13a) (55mg, 0.18 mmol) was dissolved in dimethylformarnide (2ml). The amine (I3c) (55mg, 0.18 mmoI) and potassium carbonate (28mg, 0.20 mmol) were added and the mixture was stirred at room temperature for 72 hours and then the solvent was removed *in vacuo.* The residue was partitioned between dichloromethane (10ml) and water (10ml). The layers were separated and the aqueous layer was extracted with a further portion of dichloromethane (10ml). The combined extracts were evaporated and the residue chromatographed on silica gel (methanol-dichloromethane) to give a colorless oil (36mg; 45%).
MS (+ve ion electrospray) m/z 436 (MH+).
¹H NMR (CDCl₃) δ: 1.60 (2H, m), 2.0 (2H, m), 2.15 (1H, m), 2.40 (1H, m), 2.55 (2H, m), 2.85 (2H, m), 3.25 (1H, m), 3.45 (2H, s), 3.90 (3H, s), 5.40 (1H, dd), 7.05 (1H, dt), 7.15 (1H, dt), 7.35 (1H, dd), 7.65 (1H, d), 7.70 (1H, dt), 8.00 (1H, d), 8.25 (1H, d), 8.30 (1H, dd), 8.75 (1H, d), 9.8 (1H, brs)
The oil was treated with oxalic acid in the usual way to give the dioxalate salt.

The following Examples 14-28 were prepared by the Method of Example 13:

### Example 14. N-(2,3-Difluoro-phenyl)-2-{1-[(R)-2-hydroxy-2-(6-methoxy-quinolin-4-yl)-ethyl]-piperidin-4-ylamino)-acetamide dioxalate

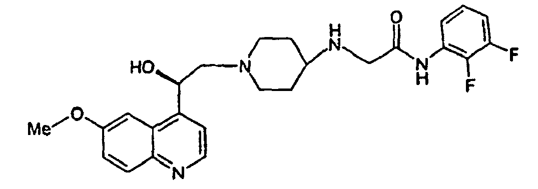

MS (+ve ion electrospray) m/z 471 (MH+)
¹H NMR (CDCl₃) δ: 1.55 (2H, m), 2.0 (2H, m), 2.25 (1H, m), 2.40 (1H, m), 2.55 (2H, m), 2.85 (2H, m), 3.30 (1H, m), 3.45 (2H, s), 3.90 (3H, s), 5.45 (1H, dd), 6.90 (1H, m), 7.10 (1H, m), 7.15 (1H, d), 7.40 (1H, dd), 7.60 (1H, d), 8.00 (1H, d), 8.15 (1H, t), 8.75 (1H, d), 9.85 (1H, brs).

### Example 15. N-(2-Chloro-5-fluoro-phenyl)-2-{1-[(R)-2-hydroxy-2-(6-methoxyquinolin-4-yl)-ethyl]-piperidin-4-ylamino)-acetamide dioxalate

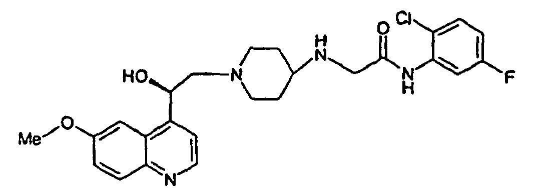

MS (+ve ion electrospray) m/z 487 (MH+)

### Example 16. N-(2,5-Difluoro-phenyl)-2-{1-[(R)-2-hydroxy-2-(6-methoxy-quinolin-4-yl)-ethyl]-piperidin-4-ylamino)-acetamide dioxalate

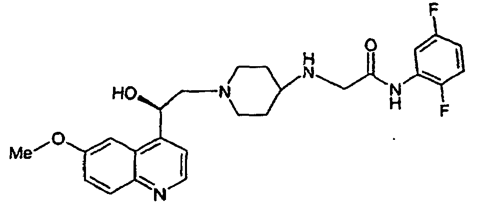

MS (+ve ion electrospray) m/z 471 (MH+)
¹H NMR (CDCI₃) δ: 1.55 (2H, m), 2.0 (2H, m), 2.20 (1H, m), 2.40 (1H, m), 2.55 (2H, m), 2.85 (2H, m), 3.30 (1H, m), 3.45 (2H, s), 3.90 (3H, s), 5.40 (1H, dd), 6.75 (1H, m), 7.00 (1H, m), 7.15 (1H, d), 7.35 (1H, dd), 7.65 (1H, d), 8.00 (1H, d), 8.25 (1H, m), 8.80 (1H, d), 9.85 (1H, brs).

### Example 17. N-(3,4-Difluoro-phenyl)-2-{1-[(R)-2-hydroxy-2-(6-methoxy-quinolin-4-yl)-ethyl]-piperidin-4-ylamino)-acetamide dioxalate

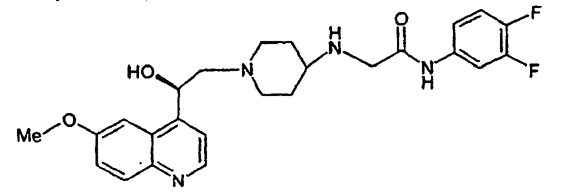

MS (+ve ion electrospray) m/z 471 (MH+)

### Example 18. N-(3,4-Dichloro-phenyl)-2-{1-[(R)-2-hydroxy-2-(6-methoxy-quinolin-4-yl)-ethylj-piperedin-4-ylamino}-acetamide dioxalate

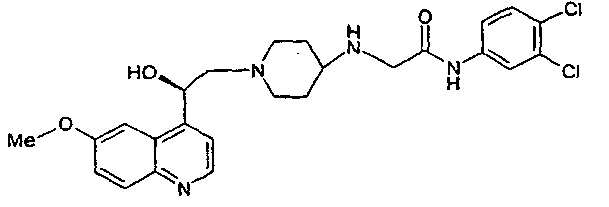

MS (+ve ion electrospray) m/z 503, 505 (MH+)

### Example 19. N-(2-Cyano-phenyl)-2-{1-[(R)-2-hydroxy-2-(6-methoxy-quinolin-4-yl)-ethyl]-piperidin-4-ylamino)-acetamide dioxalate

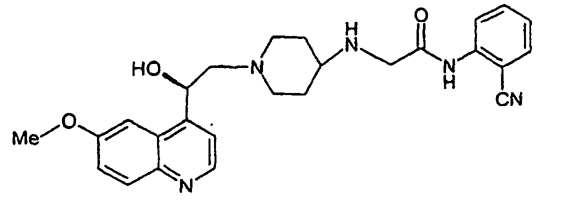

MS (+ve ion electrospray) m/z 460 (MH+)

### Example 20. 2-{1-[(R)-2-Hydroxy-2-(6-methoxy-quinolin-4-yl)-ethyl]-piperidin-4-ylamino}-N-(2-nitro-phenyl)-acetamide dioxalate

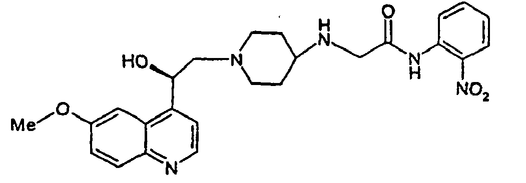

MS (+ve ion electrospray) m/z 480 (MH+)

### Example 21. N-(2,3-Dichioro-phenyl)-2-{1-[(R)-2-hydroxy-2-(6-methoxy-quinolin-4-yl)-ethyl]-piperidin-4-ylamino)-acetamide dioxalate

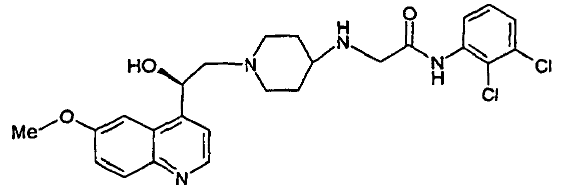

MS (+ve ion electrospray) m/z 503, 505 (MH+)

### Example 22. N-(3-Fluoro-2-methyl-phenyl)-2-{1-[(R)-2-hydroxy-2-(6-methoxy-quinolin-4-yl)-ethyl]-piperidin-4-ylamino)-acetamide dioxalate

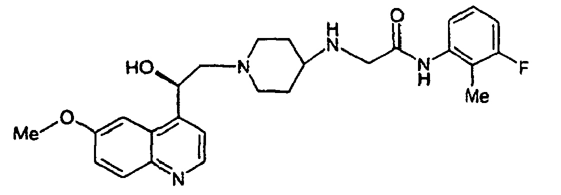

MS (+ve ion electrospray) m/z 467 (MH+)

### Example 23. N-(3,5-Dichloro-phenyl)-2-{1-[(R)-2-hydroxy-2-(6-methoxy-quinolin-4-yl)-ethyl]-piperidin-4-ylamino)-acetamide dioxalate

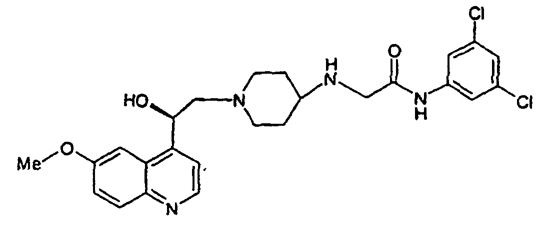

MS (+ve ion electrospray) m/z 503,505 (MH+)

### Example 24. N-[2-(1,1-Difluoro-methoxy)-phenyl]-2-{1-[(R)-hydroary-2-(6-methoxy-quinolin-4-yl)-ethyl]-piperidin-4-ylamino)-acetamide dioxalate

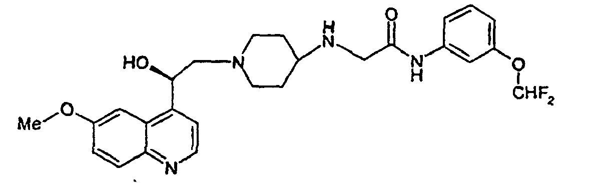

MS (+ve ion electrospray) m/z 501 (MH+)

### Example 25. 2-{1-[(R)-2-Hydroxy-2-(6-methoxy-quinolin-4-yl)-ethyl]-piperidin-4-ylamino}-N-(3-nitro-phenyl)-acetamide dioxalate

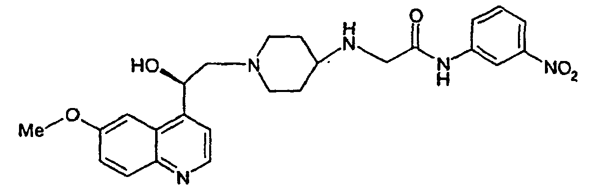

MS (+ve ion electrospray) m/z 480 (MH+)

### Example 26. N-[3-Acetyl-phenyl]-2-{1-[(R)-2-hydroxy-2-(6-methoxy-quinolin-4-yl)-ethyl]-piperidin-4-ylamino)-acetamide dioxalate

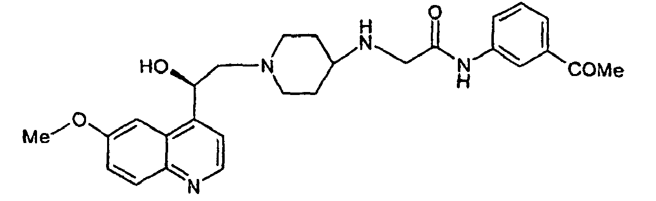

MS (+ve ion electrospray) m/z 477 (MH+)

### Example 27. 2-{1-[(R)-2-Hydroxy-2-(6-methoxy-quinolin-4-yl)-ethyl]-piperidin-4-ylamino}-N-(2-methoxycarbonyl-phenyl)-acetamide dioxalate

MS (+ve ion electrospray) m/z 493 (MH+)

### Example 28. 2-{1-[(R)-2-Hydroxy-2-(6-methoxy-quinolin-4-yl)-ethyl]-piperidin-4-ylamino}-N-(3-methoxycarbonyl-phenyl)-acetamide dioxalate

MS (+ve ion electrospray) m/z 493 (MH+)

### Example 29. N-(3,5-Difluoro-phenyl)-2-{1-[(R)-2-hydroxy-2-(6-methoxy-quinolin-4-yl)-ethyl]-piperidin-4-ylamino)-acetamide dioxalate

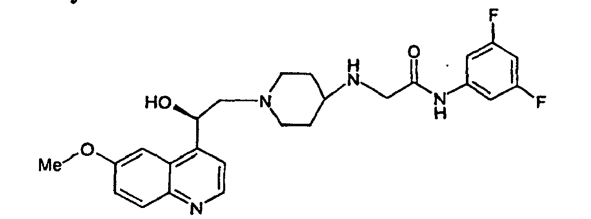

### (a) 2-Bromo-N-(3,5-difluoro-phenyl)-acetamide

To a solution of 3,5-difluoroaniline (0.5g) in dichloromethane (10ml) at 0°C was added N,N-diisopropylethylamine (0.75ml) and bromoacetyl bromide (0.37ml), dropwise. The mixture was stirred for 3 hours and then quenched with sodium bicarbonate solution (20ml). Dichloromethane was added and the layers separated, the organic layer was evaporated and the residue chromatographed on silica gel (dichloromethane) to give a tan solid.

### (b) Title compound

2-Bromo-N-(3,5-difluoro-phenyl)-acetamide (29a) (21.4mg) was dissolved in dimethylformamide (1ml). The amine (13c) (50mg) and potassium carbonate (25.2mg) were added and the mixture was stirred at room temperature for 3 hours and then the solvent was removed *in vacuo.* The residue was partitioned between dichloromethane (10ml) and water (10ml). The layers were separated and the aqueous layer was extracted with a further portion of dichloromethane (10ml). The combined extracts were evaporated and the residue chromatographed on silica gel (methanol-dichloromethane) to give a colourless oil (30mg).
MS (+ve ion electrospray) m/z 471 (MH+).
¹H NMR (CDCl₃) δ: 1.50 (2H, m), 1.98 (2H, brt), 2.25 (1H, brt), 2.41 (1H, brt), 2.55 (2H, brt), 2.85 (2H, dd), 3.30 (1H, brd), 3.40 (2H, s), 3.95 (3H, s), 5.45 (1H, m), 6.57 (1H, bt), 7.20 (3H, m) 7.45 (1H, dd), 7.62 (1H, d), 8.05 (1H, d), 8.78 (1H, d), 9.50 (1H, bs).
The oil was treated with oxalic acid in the usual way to give the dioxalate salt.

### Example 30 4-(Phenylcarbamoylmethyl-amino)-piperidine-1-carboxylic acid (6-methoxy-[1,5]-naphthyridin-4-yl)-amide oxalate.

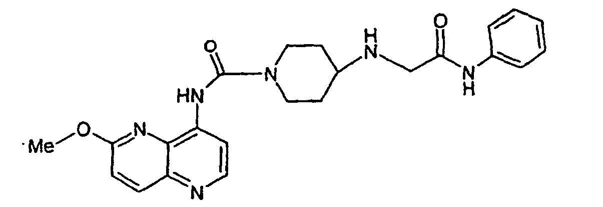

### (a) 4-Hydroxy-6-methoxy-[1,5]-naphthyridine-3-carboxylic acid ethyl ester

3-Amino-6-methoxypyridine (12.41 g) and diethyl ethoxymethylene malonate (20.2 ml) in Dowtherm A (400 ml) were heated at reflux, under argon for 1 hour. The cooled reaction mixture was poured into pentane (1 litre). The precipitated solid was collected by filtration, washed with pentane and dried to afforded a solid (24.78 g, crude). MS (+ve ion electrospray) m/z 249 (MH⁺).

### (b) 4-Hydroxy-6-methoxy-[1,5]-naphthyridine-3-carboxylic acid

The ester (30a) (0.642g) in 10% aqueous sodium hydroxide (115 ml) was heated at reflux for 1.5 hours. The reaction mixture was cooled then acidified with glacial acetic acid. The precipitated solid was collected by filtration, washed with water and dried in *vacuo* to afford a beige solid (0.542g). MS (+ve ion electrospray) m/z 221 (MH⁺).

### (c) 4-Chloro-6-methoxy-[1,5]-naphthyridine

The acid (30b) (6.82 g) was heated in quinoline (20ml) at reflux for 2 hours, the mixture was cooled and poured into ether and the orange solid was filtered and washed with ether. A sample (3.87g) of the dried solid was treated with phosphorus oxychloride (30ml) at room temp for 3 hours, the solvent was removed *in vacuo* and the residue quenched with crushed ice (200g). The mixture was basified with ammonia solution and filtered. The solid was washed with dichloromethane (10 x 100m1), which was evaporated and chromatographed on silica gel (dichloromethane as eluent) to give a yellow solid (3.0g).
MS (+ve ion electrospray) m/z 195, 197 (MH⁺).

### (d) 4-Amino-6-methoxy-[1,5]-naphthyridine

A solution of the chloro compound (30c) (2.0g) in pyridine (30ml) was treated with n-propylamine hydrochloride (6.0g) and the mixture heated at reflux for 16 hours. The reaction mixture was cooled and partitioned between water and ethyl acetate: The aqueous phase was washed with ethyl acetate, the combined organics dried (Na₂SO₄) and the solvent removed under reduced pressure. Purification by chromatography on silica gel (methanol in dichloromethane) afforded a yellow solid (1.0g).
¹H NMR (CDCl₃) δ: 4.05 (3H, s), 5.36 (2H, bs), 6.71 (1H, d, J=5 Hz), 7.08 (1H, d), 8.10 (1H, d), 8.40 (1H, d). MS (+ve ion electrospray) m/z: 176 (MH⁺).

### (e) 4-tert-Butoxycarbonylamino-1-(6-methoxy-[1,5]-naphthyridin-4-yl)aminocarbonylpiperidine

To a solution of 4-amino-6-methoxy-[1,5]-naphthyridine (30d) (2.1g,13.5 mmol) and 4-(dimethylamino)pyridine (1.62g) in anhydrous chloroform (45ml) was added N,N'-carbonyldiimidazole (3.28g, 20.1 mmol).The mixture was stirred for 4 hours at room temperature, then evporated and the residue was dissolved in anhydrous DMF (45ml). 4-*tert*-Butoxycarbonylaminopiperidine hydrochloride (3.34g, 13.5 mmol) and potassium carbonate (1.89g) were added, and the mixture was heated at 70°C overnight. The mixture was evaporated and the residue was mixed with water. The solid was filtered off and dried (3.89g).
MS (+ve ion electrospray) m/z 402 (MH+).

### (f) 4-Amino-1-(6-methoxy-[1,5]-naphthyridin-4-yl)aminocarbonylpiperidine

The *tert*-butoxycarbonylamino compound (30e)(3.88g, 9.7 mmol) was treated with trifluoroacetic acid in dichloromethane and the solution was stirred at room temperature for 1.5 hours, then evaporated *in vacuo*. After addition of toluene and re-evaporation, the residue was treated with a 4M solution of hydrogen chloride in 1,4-dioxan (30ml). The resulting solid was triturated, filtered off and washed with ether. The crude hydrochloride was dissolved in water and washed twice with dichloromethane, basified to pH9 and evaporated to dryness. Extraction with 10% methanol-dichloromethane gave the free base (3.45g). MS (+ve ion electrospray) m/z 302 (MH+).

### (g) Title compound

2-Bromo-N-phenyl-acetamide [prepared from aniline and bromoacetyl bromide by the method of Example (13a)] (71mg) was dissolved in dimethylformamide (2ml). The amine (30f) (100mg) and potassium carbonate (50mg) were added and the mixture was stirred at room temperature for 24 hours and then the solvent was removed *in vacuo.* The residue was partitioned between dichloromethane and water. The layers were separated and the aqueous layer was extracted with a further portion of dichloromethane. The combined extracts were evaporated and the residue chromatographed on silica gel (methanol-ethyl acetate) to give a colourless oil.
MS (+ve ion electrospray) m/z 435 (MH+).
The oil was treated with oxalic acid in the usual way to give the oxalate salt.

### Example 31. 2-{1-[(R)-2-Hydroxy-2-(6-methoay-[1,5]-naphthyridine-4-yl)-ethyl]-piperidin-4-ylamino}-N-phenyl-acetamide dioxalate

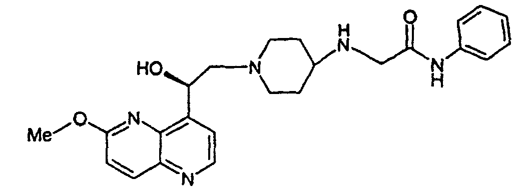

### (a) 4-Hydroxy-6-methoxy-[1,5]-naphthyridine

5-Amino-2-methoxypyridine (55g, 0.44mol) in methanol (1000ml) with methyl propiolate (40ml, 0.44mol) was stirred for 48 hours, then evaporated and the product purified by chromatography on silica gel (dichloromethane) followed by recrystallisation from dichloromethane-hexane (44.6g, 48%).
The unsaturated ester (10.5g, 0.05mol) in warm Dowtherm A (50ml) was added over 3 minutes to refluxing Dowtherm A, and after a further 20 minutes at reflux the mixture was cooled and poured into ether. The precipate was filtered to give the title compound (6.26g, 70%)

### (b) Bromomethyl-(6-methoxy-[1,5]-naphthyridin-4-yl)-ketone

The naphthyridine (31a) (10g, 0.057mol) in dichloromethane (200ml) containing 2,6-lutidine (9.94ml, 0.086mol) and 4-dimethylaminopyridine (0.07g, 0.0057mol) was cooled in ice and treated with trifluoromethanesulfonic anhydride (10.5ml, 0.063mol). After stirring for 2.5 hours the mixture was washed with saturated ammonium chloride solution, dried, evaporated and purified on silica (dichloromethane). The triflate (13.2g, 0.044mol) in DMF (200ml) with triethylamine (12ml, 0.086mol) butyl vinyl ether (22ml, 0.17mol), palladium (II) acetate (0.97g, 0.0044mol) and 1,3-bis(diphenylphosphino)propane (1.77g, 0.0044mol) was heated at 60°C for 3 hours then evaporated and chromatographed on silica gel (dichloromethane) to give a yellow solid (10.7g, 95%). This was dissolved in THF (250ml), water (40ml) and treated with N-bromosuccinimide (7.4g, 0.042 mol) for 1 hour, then evaporated and chromatographed on silica gel (dichloromethane) to give the ketone (10.42g, 98%).

### (c) [R]-2-Bromo-1-(6-methoxy-[1,5]-naphthyridin-4-yl)ethanol

The ketone (31b) (6.6g, 0.023mol) in toluene was treated with (+)-DIP-chloride (12g, 0.037mol) and stirred overnight, then diethanolamine (15g, 0.14mol) added and the mixture stirred for 3 hours, filtered and evaporated. Chromatography on silica gel (ethyl acetate-hexane )gave a white solid (4.73g, 73%).

### (d) [R]-2-(6-methoxy-[1,5]-naphthyridin-4-yl)oxirane

The alcohol (31 c) (4.8g, 0.017mol) in methanol (20ml) was stirred with potassium carbonate (2.6g, 0.019 mol) for 1 hour, then evaporated and chromatographed on silica gel (ethyl acetate-hexane-dichloromethane) to give a solid (3.14g, 92%), (91% ee by chiral HPLC).
MS (+ve ion electrospray) m/z 203 (MH+).

### (e) 4-tert-Butoxycarbonylamino-1-[2-(R)-hydroxy-2-(6-methoxy-[1,5]-naphthyridin-4-yl)]ethylpiperidine.

This was prepared from [R]-2-(6-methoxy-[1,5]-naphthyridin -4-yl)oxirane (31d) (3.0g, 14.9mmol) by the method of Example (13b), to give a foam (5.34g).
MS (+ve ion electrospray) m/z 403 (MH+).

### (f) 4-Amino-1-[2-(R)-hydroxy-2-(6-methoxy-[1,5]-naphthyridin-4-yl)]ethylpiperidine.

This was prepared from the *tert*-butoxycarbonylamino compound (31e) (5.15g) by the method of Example (13c), to give a solid (3.15g). MS (+ve ion electrospray) m/z 303 (MH+).

### (g) Title compound

This was prepared from the amine (31f) (0.1g) and 2-bromo-N-phenyl-acetamide (0. 07 1 g) by the method of Example (30g) to give the free base of the title compound (0.020g).
¹H NMR (CDCl₃) δ: 1.45-2.65 (9H, m), 2.85 (1H, m), 3.05 (1H, dd), 3.30 (1H, m), 3.45 (2H, s), 3.95 (3H, s), 5.70 (1H, m) 7.10 (2H, m), 7.35 (2H, t), 7.65 (2H, d), 7.78 (1H, m), 8.20 (1H, d), 8.78 (1H, d), 9.40 (1H, bs).
MS (+ve ion electrospray) m/z 436 (MH+).

This was converted to the dioxalate salt (0.027g) in the usual way

### Example 32. N-[3-(1-Hydroxy-ethyl)-phenyl]-2-{1-[(R)-2-hydroxy-2-(6-methoxy-quinolin-4-yl)-ethyl]-piperidin-4-ylamino)-acetamide dioxalate

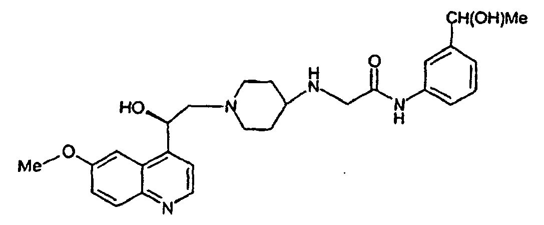

The ketone Example (26) (23 mg, 0.05 mmol) was dissolved in methanol (1ml) and sodium borohydride (1.8mg, 0.05 mmol) was added. The mixture was stirred at room temperature for 30 minutes. The solvent was evaporated and the residue partitioned between dichloromethane (5ml) and sodium bicarbonate solution (5ml). The organic layer was dried (MgSO₄), filtered and evaporated to give a colourless oil (23mg).
MS (+ve ion electrospray) m/z 479 (MH+)
The dioxalate salt was prepared in the usual manner.

### Example 33. N-[2-Carboxy-phenyl]-2-{1-[(R)-2-hydroay-Z-(6-methoxy-quinolin-4-yl)-ethyl]-piperidin-4-yiamino)acetamide dioxalate

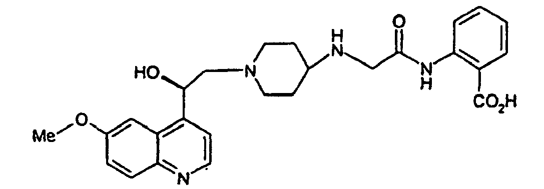

The ester Example (27) (36mg, 0.07 mmol) was dissolved in tetrahydrofuran (1mI) and 2M sodium hydroxide solution (1ml, 0.2 mmol) followed by water (0.5ml) were added. The mixture was heated at 60°C for 1 hour, cooled to room temperature and 5M hydrochloric acid added until the solution was pH 4. The mixture was evaporated to dryness and methanol (5ml) was added. The methanolic solution was filtered to give a solid, in quantitative yield.
MS (+ve ion electrospray) m/z 479 (MH+)
The dioxalate salt was prepared in the usual manner.

### Example 34. N-[3-Cyano-phenyl]-2-{1-[(R)-2-hydroxy-2-(6-methoxy-quinolin-4-yl)-ethyl)-piperidin-4-yfanuno)-acetamide dioxalate

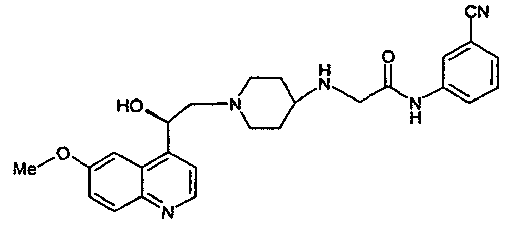

### (a) (l-Benzyl-pipendin-4-ylamino)-acetic acid benzyl ester

1-Benzyl-4-piperidone (23.5g, 124 mmol) and glycine benzyl ester (124 mmol) were stirred in dichloromethane (500rnl) with 3A molecular sieves at 0°C for 1 hour and sodium triacetoxyborohydride (18.2g) was added portionwise over 15 minutes, and the mixture was stirred at room temperature for 1.5 hours. The mixture was cooled to 0°C and sodium bicarbonate solution was added, and the organic layer was separated, dried, evaporated and chromatographed on silica gel (ammonia-methanol-ethyl acetate) to afford an oil (29.21g).

### (b) [(1-Benzyl-piperidin-4-yl)-tert-butoxycarbonyl-amino]-acetic acid benzyl ester

The amine (34a) (29.21g) in dichloromethane (200ml) was treated with di-tert-butyl dicarbonate (20.66g) and 4-dimethylaminopyridine (0.10g) and the solution was stirred at room temperature for 2 hours, evaporated and chromatographed on silica gel (ethyl acetate-dichloromethaane) to afford an oil (28.1g).

### (c) 4-[(Benzyloxycarbonylmethyl)-(tert-butoxycarbonyl)-amino]-piperidine-1-carboxylic acid 1-chloro-ethyl ester

The piperidine (34b) (5.15g) in dichloromethane (50ml) at 0°C was treated with di-isopropylethylamine (1.8g) and 1-chloroethyl chloroformate (1.4ml) for 1 hour, evaporated, and chromatographed on silica gel (ethyl acetate-dichloromethaane) to afford an oil (4.3g).

### (d) (tert-Butoxycarbonyl-piperidin-4-yl-amino)-acetic acid benzyl ester

The carbamate (34c) (11.9g) in methanol (100ml) was left at room temperature for 4 days, evaporated, and partitioned between ethyl acetate and sodium bicarbonate solution. The organic fraction was dried, evaporated, and chromatographed on silica gel to afford an oil (1.2g).

### (e) ((tert-Butoxycarbonyl)-{1-[(R)-2-hydroxy-2-(6-methoxy-quinolin-4-yl)-ethyl]-piperidin-4-yl}-amino)-acetic acid benzyl ester

The piperidine (34d) (500mg, 1.44 mmol) in acetonitrile (5ml) under argon at room temperature was treated with [R]-2-(6-methoxyquinolin-4-yl)oxirane (1a) (290mg, 1.44 mmol) and lithium perchlorate (153mg, 1.44 mmol). The mixture was stirred at this temperature for 96 hours then the solvent was evaporated and the residue partitioned between dichloromethane and sodium bicarbonate solution. The layers were separated and the aqueous portion was extracted with dichloromethane. The combined organic layers were evaporated and the residue chromatographed on silica gel (methanol-dichloromethane) to give a colourless oil (529mg, 67%).

### (f) ((tert-Butoxycarbonyl)-{1-[(R)-2-hydroxy-2-(6-methoxy-quinolin-4-yl)-ethyl]-piperidin-4-yl}-amino)-acetic acid

The ester (34e) (520mg, 0.93 mmol) was dissolved in ethanol (50ml) and 10% palladium on charcoal (0.5g) was added. The mixture was hydrogenated for 4 hours, then filtered and evaporated. The colourless solid (427mg) obtained was used crude in the next step.
MS (+ve ion electrospray) m/z 460 (MH+)

### (g) ((tert-Butoxycarbonyl)-N-[3-cyano-phenyl]-2- {1 -[(R)-2-hydroxy-2-(6-methoxy-quinolin-4-yl)-ethyl]-piperidin-4-yl-amino)-acetamide

The acid (34f) (100mg, 0.22 mmol) and 1-(3-dimethylaminopropyl)-3-ethyl carbodiimide hydrochloride (42mg, 0.22 mmol) were stirred at room temperature in dichloromethane for 10 minutes. 3-Cyanoaniline (26mg, 0.22 mmol) was added and the mixture stirred at room temperature for 12 hours. Dichloromethane and water were added and the layers separated. The aqueous phase was washed with dichloromethane and the combimed organics were evaporated and the residue chromatographed on silica gel, (methanol-dichloromethane) to give a colorless oil (42mg, 34%).
MS (+ve ion electrospray) m/z 560 (MH+)

### (h) Title compond

The piperidine (34g) (42mg, 0.07 mmol) was treated with dichloromethane (3ml) and trifluoroacetic acid (1ml) for 1 hour. The solvents were evaporated and the residue treated with oxalic acid in the usual manner to give the dioxalate (43 mg).
MS (+ve ion electrospray) m/z 460 (MH+)

The following Examples 35-36 were prepared by the Method of Example 34:

### Example 35. N-[4-Cyano-phenyl]-2-{1-[(R)-2-hydroxy-2-(6-methoxy-quinolin-4-yl)-ethyll-piperidia-4-ylantino)-acetamide dioxalate

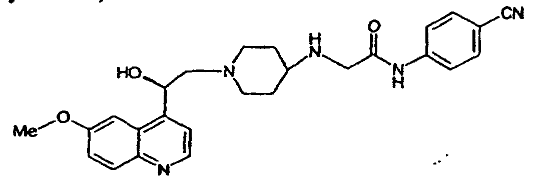

MS (+ve ion electrospray) m/z 460 (MH+)

### Example 36. N-[4-Fluoro-3-nitro-phenyl]-2-{1-[(R)-2-hydroxy-2-(6-methoxy-quinolin-4-yl)-ethyl]-piperidin-4-ylamino)-acetamide dioxalate

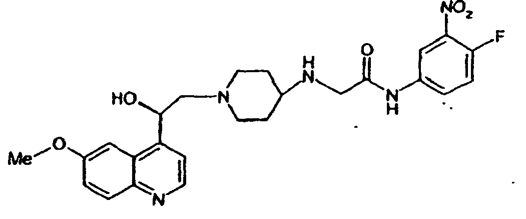

MS (+ve ion electrospray) m/z 498 (MH+)

### Example 37 2-{1-[2-(R)-Hydroxy-2-(6-methory-quinolin-4-yl)-ethyl]-4-methoxycarbonyl-piperidin-4-ylamino}-N-phenyl-acetamide dioxalate

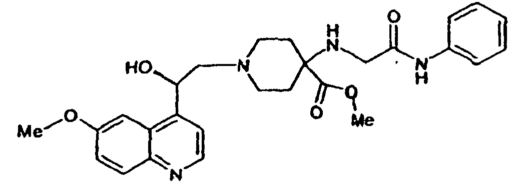

### (a) Methyl 4-benzyloxycarbonylamino-1-tert-butoxycarbonylpiperidine-4-carboxylate.

4-Amino-1-*tert*-butoxycarbonylpiperidine-4-carboxylic acid (18.0g) was dissolved in 0.03M aqueous sodium hydroxide (750ml), and 1,2-dimethoxyethanol (DME, 100ml). The pH was adjusted to 9.5 with dilute hydrochloric acid, and the suspension was added to an ice-cold solution of N-(benzyloxycarbonyloxy)succinimide (28.8g) in DME (100ml). The pH was kept at 9.5 by addition ofaqueous sodium hydroxide, and the mixture was stirred at room temperature. More N-(benzyloxycarbonyloxy)succinimide (3g) was added after 7 hours, then stirring was continued overnight. After evaporation, the aqueous residue was extracted with ether then acidified to pH4 and extracted with ethyl acetate. This extract was washed with dilute hydrochloric acid, water and brine, dried and evaporated. Trituration of the oily residue gave 4-benzyloxycarbonylamino-1-*tert*-butoxycarbonylpiperidine-4-carboxylic acid (23g). This acid (23g) was esterified by treatment with methyl iodide (3.8ml), and potassium carbonate (16.6g) in acetone (200ml) at room temperature for 3 days. Filtration, evaporation, partitioning of the residue between dichloromethane and water and evaporation of the organic phase gave the methyl ester (21.05g).
MS (+ve ion electrospray) m/z 293 (MH⁺- t-BOC)

### (b) Metlayl amino-1-tert-butoxycarbonylpiperidine-4-carboxylate

The piperidine (37a) (12.77g) was treated with ethanol (100m1) and 10% palladium on charcoal (2g). The mixture was hydrogenated for 72 hours, filtered and evaporated to give a colourless oil (5.66g, 65%).

### (c) [1-tert-Butoxycarbonyl-4-methoxycarbonyl-piperidin-4-ylamino]-N-phenyl-acetamide

The amine (37b) (3.57g, 12.6 mmol) was dissolved in dimethylformamide (20ml). Potassium carbonate (1.79g, 13 mmol) and N-phenyl bromoacetamide (2.8g, 13 mmol) were added and the mixture was stirred at room temperature for 24 hours and then the solvent removed *in vacuo*. The residue was partitioned between dichloromethane and water. The layers were separated and the aqueous layer was extracted with a further portion of dichloromethane. The combined extracts were evaporated and the residue chromatographed on silica gel (methanol-dichloromethane) to give a colourless oil (4.35g, 93%).
MS (+ve ion electrospray) m/z 392 (MH+)

### (d) [4-Methoxycarbonyl-piperidin-4-ylamino)-N-phenyl-acetamide

The piperidine (37c) (4.35g, 11.7 mmol) was treated with dichloromethane (20ml) and trifluoro acetic acid (10ml) for 1 hour. The solvents were evaporated and the residue treated with sodium bicarbonate solution. The aqueous mixture was extracted with dichloromethane (100m1) which contained impure product (1.5g, discarded). The aqueous layer was treated with 2M sodium hydroxide solution until pH 11 and then extracted with dichloromethane (5 x 100 ml). The combined organic extracts were evaporated to give a colourless oil (1.86g, 55%).
MS (+ve ion electrospray) m/z 292 (MH+)

### (e) Title compound

The piperidine (37d) (286mg, 1 mmoI) in acetonitrile (2ml) under argon at room temperature was treated with [R]-2-(6-methoxyquinolin-4-yl)oxirane (1a) (201mg, 1 mmol) and lithium perchlorate (106.4mg, 1 mmol). The mixture was stirred at this temperature for 20 hours then the solvent was evaporated and the residue partitioned between dichloromethane and sodium bicarbonate solution. The layers were separated and the aqueous portion was extracted with dichloromethane. The combined organic layers were evaporated and the residue chromatographed on silica gel (methanol-dichloromethane) to give a colourless oil (258mg, 52%).
MS (+ve ion electrospray) m/z 493 (MH+)
A portion was treated with oxalic acid to produce the dioxalate salt in the usual manner.

### Example 38 2-{1-[2-(R)-Hydroxy-2-(6-methoxy-quinolin-4-yl)-ethyl]-4-hydroxymethyl-piperidin-4-ylamino}-N-phenyl-acetamide dioxalate

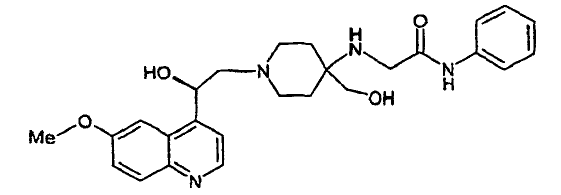

The ester Example (37) (109mg, 0.22 mmol) was dissolved in tetrahydrofuran (2ml) under argon and cooled to 0°C. A 1M solution of lithium aluminium hydride in tetrahydrofuran (0.22ml, 0.22 mmol) was added and the mixture stirred for 1 hour. 2M Sodium hydroxide solution (10ml) was added carefully followed by dichloromethane (15ml). The layers were separated and the aqueous layer was extracted with dichloromethane, the combined organics were evaporated and the residue chromatographed on silica gel (methanol-dichloromethane) to give a colourless oil (34mg, 31 %)
MS (+ve ion electrospray) m/z 465 (MH+)
¹H NMR (CDCl₃) δ: 1.70 (4H, m), 2.55 (3H, m), 2.85 (3H, m), 3.35 (2H, s), 3.50 (2H, s), 3.85 (3H, s), 5.45 (1H, dd), 7.15 (1H, d), 7.35 (3H, m), 7.40 (1H, dd), 7.60 (2H, d), 7.65 (1H, d), 8.05 (1H, d), 8.75 (1H, d), 9.30 (1H, brs).
It was treated with oxalic acid to produce the dioxalate salt in the usual manner.

### Example 39 4-Methoxycarbonyl-4-(phenylcarbamoylmethyl-amino)-piperidine-1-carboxylic acid (6-methoxy-quinolin-4-yl)-amide oxalate.

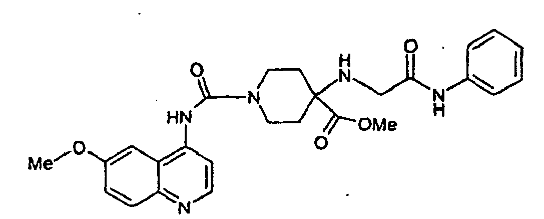

The piperidine (37d) (291mg, 1 mmol) was heated in dimethylformamide (2ml) with 4-(1-imidazolylcarbonyl)amino-6-methoxyquinoline (5b) (268mg, 1 mmol) at 70°C for 12 hours. The solvent was evaporated and the residue partitioned between dichloromethane and sodium bicarbonate solution. The layers were separated and the aqueous portion was extracted with dichloromethane. The combined organic layers were evaporated and the residue chromatographed on silica gel (methanol-dichloromethane) to give a colourless oil (154mg, 31%)
MS (+ve ion electrospray) m/z 492 (MH+)
It was treated with oxalic acid to produce the oxalate salt in the usual manner.

### Example 40 4-Hydroxymethyl-4-(phenylcarbamoylmethyl-amino)-piperidine-1-carboxylic acid (6-methoxy-quinolin-4-yl)-amide oxalate.

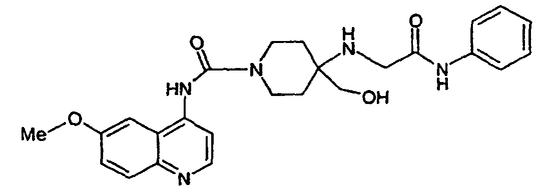

The ester Example 39 (140mg, 0.28 mmol) was dissolved in tetrahydrofuran (2ml) under argon and cooled to 0°C. A 1M solution of lithium aluminium hydride (0. 14ml, 0.14 mmol) was added and the mixture stirred for 10 minutes. Further lithium aluminium hydride (0.14ml) was added and the mixture stirred for 1 hour. 2M sodium hydroxide solution was added carefully followed by dichloromethane. The layers were separated and the aqueous layer was extracted with dichloromethane, the combined organics were evaporated and the residue chromatographed on silica gel (methanol-dichloromethane) to give a colourless oil (57mg, 44%).
MS (+ve ion electrospray) m/z 464 (MH+)
It was treated with oxalic acid to produce the oxalate salt in the usual manner.

### Example 41. N-(4-Fluoro-phenyl)-2-{1-[(R)-2-hydroxy-2-(6-methoxy-quinolin-4-yl)-etbyl]-piperidin-4-ylamino}-acetanaide dioxalate

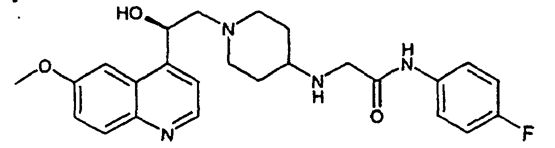

### (a) {1 -[(R)-2-Hydroxy-2-(6-methoxy-quinoIm-4-yl)-ethy!]-pipendin-4-ylamino} -acetic acid benzyl ester

A solution of ketone (1c) (0.5g) and benzyl glycinate (0.37g) in dichloromethane (5ml) was treated with sodium triacetoxyborohydride (0.47g). After 16 hours the mixture was partitioned between dichloromethane and saturated aqueous sodium bicarbonate solution. The dichloromethane extract was dried and evaporated to give a yellow solid. Chromatography on silica gel (methanol-dichloromethane) afforded the product as a white solid (0.27g)
MS (+ve ion electrospray) m/z 450 (MH+)

### (b) (tert-Butoxycarbonyl-{1-[(R)-2-tert-butoxycarbonyloxy-2-(6-methoxy-quinolin-4-yl)-ethyl]-piperidin-4-yl}-amino)-acetic acid benzyl ester

A solution of (41a) (0.27g) in chloroform (5ml) was treated with di-*tert*-butyl dicarbonate (0.4g) and 4-N,N-dimethylaminopyridine (40mg) for 16 hours The product was chromatography on silica gel (methanol/ethyl acetate) to afford an oil (0.16g)
MS (+ve ion electrospray) m/z 650 (MH+)

### (c) (tert-Butoxycarbonyl-{1-[(R)-2-tert-butoxycarbonyloxy-2-(6-methoxy-quinolin-4-yl)-ethyl]-piperidin-4-yl}-amino)-acetic acid

A solution of (41b) (0.16g) in ethanol (15ml) was hydrogenated over 10% palladium on charcol (0.2g) for 2 hours. Filtration and evaporation gave a mixture of (49c) and (tert-butotycarbonyl-{1-[2-(6-methoxy quinolin-4--yl)-ethyl)-piperidin-4-yl}-amino)-acetic acid as an oil (0.12g)
MS (+ve ion electrospray) m/z 560 (MH+) (41 c)
MS (+ve ion electrospray) m/z 444 (MH+) (by-product)

### (d) Carbonic acid (R)-2-(4-{tert-butoxycarbonyl-[(4-fluoro-phenylcarbamoyl)-methyl]-amino}-piperidin-1-yl)-1-(6-methoxy-quinolin-4-yl)-ethyl ester tert-butyl ester

A solution of (41c) (0.12g) in N,N-dimethylformamide (2ml) was treated with 4-fluoroaniline (30mg) and 1-(3-dimethylaminopropyl)-3-ethyl-carbodiimide hydrochloride (50mg). After 16 hours the mixture was partitioned between ethyl acetate and saturated aqueous sodium bicarbonate solution. The ethyl acetate extract was dried and evaporated to give a yellow oil. Chromatography on silica gel (methanol-ethyl acetate) afforded (41d) as an oil (40mg).
The earlier fractions from the column gave [(4-fluoro-phenylcarbamoyl)-methyl]-{1-[2-(6-methoxy-quinolin-4-yl)-ethyl]-piperidin-4-yl}-carbamic acid *tert*-butyl ester as an oil (17mg).

### (e) Title compound

A solution of (4 1 d) (40mg) in trifluoroacetic acid (5ml) was stirred for 2.5 hours then evaporated to dryness. The residue was partitioned between ethyl acetate and saturated aqueous sodium bicarbonate solution. The ethyl acetate extract was dried and evaporated to give a yellow oil. Chromatography on silica gel (methanol-ethyl acetate) afforded the title compound as an oil (20mg).
MS (+ve ion electrospray) m/z 453 (MH+).
This was converted to the dioxalate salt in the usual way.

### Example 42. N-(4-Fluoro-phenyl)-2-{1-[2-(6-methoxy-quinolin-4-yl)-ethyl]-piperidin-4-ylamino}-acetamide dioxalate

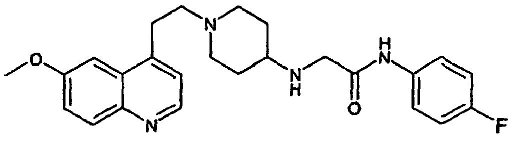

A solution of [(4-fluoro-phenylcarbamoyl)-methyl]-{1-[2-(6-methoxy-quinolin-4-yl)-ethyl]-piperidin-4-yl}-carbamic acid *tert*-butyl ester (17mg) (see earlier fraction in (41d) above) in trifluoroacetic acid (3ml) was stirred for 1 hour then evaporated to dryness. The residue was partitioned between ethyl acetate and saturated aqueous sodium bicarbonate solution. The ethyl acetate extract was dried and evaporated to give a yellow oil. Chromatography on silica gel (methanol-ethyl acetate) afforded the title compound as an oil (9mg).
MS (+ve ion electrospray) m/z 437 (MH+).
This was converted to the dioxalate salt in the usual way.

### Example 43. 4-[2-(3,5-Difluorophenoxy)-ethyl]amino-1-[2-(R)-hydroxy-2-(6-methoxyquinolin-4-yl)]ethylpiperidine dioxalate

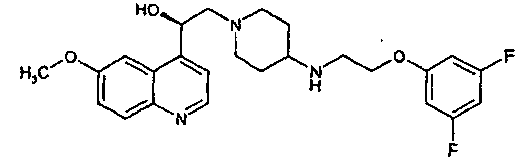

### (a) (3,5-Difluorophenoay)-acetaldehyde

A solution of bromoacetaldehyde dimethyl acetal (1 g) and 3,5-difluorophenol (0.8g) was treated with potassium carbonate (0.8g) and heated at 100 ^{o}C for 24hours. The mixture was partitioned between ether and 2N aqueous sodium hydroxide solution. The ether extract was dried and evaporated to afford 1-(2,2-dimethoxyethoxy)-3,5-difluorobenzene as an oil (0.2g) This was dissolved in a 2% solution of water in tetrahydrofuran (5 ml), treated with acidic Amberlyst-15 resin (0.75g) and heated at 60°C for 10 hours. The mixture was filtered and evaporated affording the title aldehyde as an oil (0.1g).
MS (+ve ion electrospray) m/z 173 (MH+)

### (b) Title compound

A mixture of aldehyde (43a) (0.1g) and amine (13c) (0.09g) in dichloromethane-methanol (2 ml/0.2 ml) was treated with 3A molecular sieves (0.2g) then after 0.5 hours with sodium triacetoxyborohydride (0.2g). After 16 hours the mixture was partitioned between dichloromethane and saturated aqueous sodium bicarbonate solution. The dichloromethane extract was dried and evaporated to give a yellow solid. Chromatography on silica gel (methanol-dichloromethane) afforded the product as a white solid (0.075g, 55%).
¹H NMR (CDCl₃) δ: 1.60 (2H, m), 2.00 (2H, m), 2.25 (1H, m),) 2.50 (3H, m), 2.80 (2H, m), 3.00 (2H, t), 3.25 (1H, m), 3.90 (3H, m), 4.05 (2H, m), 5.45 (1H, dd), 6.45 (3H, m), 7.20 (1H, d), 7.35 (1H, dd), 7.65 (1H, d), 8.05 (1H, d), 8.80 (1H, d)
MS (+ve ion electrospray) m/z 458 (MH+)
This was converted to the dioxalate salt by treating a deuterochloroform solution with a solution of oxalic acid (36mg) in ether. The precipitate was isolated by centrifugation, resuspended in ether and again isolated by centrifugation. Drying in *vacuo* afforded the title compound as a white solid (66mg).

### Example 44. 4-(2-Phenoxyethyl)amino-1-[2-(R)-hydroxy-2-(6-methoxyquinolin-4-yl)]ethylpiperidine dioxalate

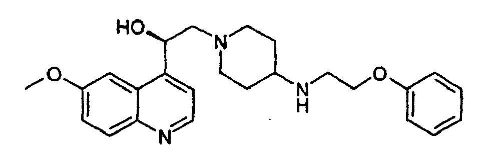

A solution of the ketone (1c) (1.0g) and 2-phenoxyethylamine (0.5g) in dichloromethane was treated with 3A molecular sieves (0.5g) and shaken for 1 hour. Sodium triacetoxyborohydride (1.0g) was added and the mixture shaken for 24 hours. The mixture was partitioned between dichloromethane and saturated aqueous sodium bicarbonate solution. The dichloromethane extract was dried and evaporated to give a yellow oil. Chromatography on silica eluting with a methanol/dichloromethane gradient afforded the product as an oil (0.97g). This was converted to the dioxalate salt (0.9g) by the same procedure as for Example (43).
MS (+ve ion electrospray) m/z 422 (MH+)
¹H NMR (CDCl₃) δ: 1.60 (2H, m), 2.00 (2H, m), 2.05 (1H, m), 2.50 (3H, m), 2.80 (2H, m), 3.02 (2H, t), 3.25 (1H, m), 3.90 (3H, m), 4.08 (2H, m), 5.50 (1H, dd), 6.95 (3H, m), 7.20 (1H, d), 7.30 (2H,m), 7.35 (1H, dd), 7.65 (1H, d), 8.05 (1H, d), 8.80 (1H, d)

### Example 45. 4-[2-(Pyridin-2-yloxy)-ethylamino]-1-[2-(R)-hydroxy-2-(6-methoxyquinolin-4-yl)]ethylpiperidine dioxalate

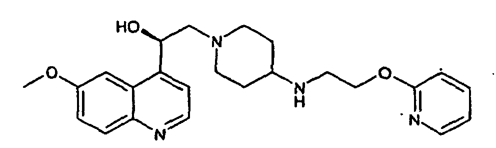

### (a) 2-(Pyridin-2-yloxy)-ethylamine

This was prepared in 71% yield from 2-chloropyridine and ethanolamine according to the procedure of G. Tarrago *et al*, Tetrahedron, 1988, 44, 91.
MS (+ve ion electrospray) m/z 139 (MH+)

### (b) Title compound

This was prepared by reductive alkylation with amine (45a) (70 mg) and ketone (1c) (0.15g) according to the same procedure as for Example (44), giving the title compound (free base) as a white foam (95 mg, 45%), which was converted to the dioxalate salt (120 mg) by the same procedure as for Example 43.
¹H NMR (CDCl₃) δ: 1.60 (2H, m), 1.95 (2H, m), 2.15 (1H, m), 2.50 (3H, m), 2.80 (2H, m), 3.08 (2H, t), 3.25 (1H, m), 3.90 (3H, m), 4.45 (2H, t), 5.45 (1H, dd), 6.78 (1H, d), 6.90 (1H, t), 7.25 (1H, d), 7.40 (1H, dd), 7.55 (1H, m), 7.65 (1H, d), 8.05 (1H, d), 8.20 (1H, m), 8.80 (1H, m).
MS (+ve ion electrospray) m/z 423 (MH+)

### Example 46. 4-(2-Phenoxyethylamino)-1-(6-methoxy-[1,5]-naphthyridin-4-yl)aminocarbonylpiperidine oxalate

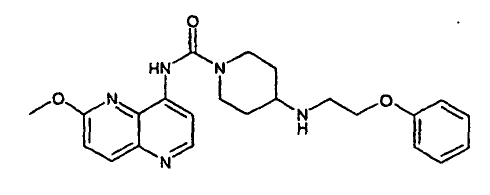

A solution of amine (30f) (0.1g) and phenoxyacetaldehyde (0.16g) in dichloromethane and methanol (1ml) was stirred for 0.5 hours, then sodium triacetoxyborohydride (0.11g) was added. After 6 hours the mixture was partitioned between dichloromethane and saturated aqueous sodium bicarbonate solution. The dichloromethane extract was dried and evaporated to give a yellow oil. Chromatography on silica (methanol-dichloromethane) afforded the free base of the title compound as a solid (0.025g). This was converted to the oxalate salt (0.024g) by the same procedure as for Example (44).
¹H NMR (CDCl₃) δ: 1.50 (2H, m), 2.00 (2H, m), 2.85 (1H, m), 3.05 (2H, t), 4.10 (7H, m), 6.95 (3H, m), 7.15 (1H, d), 7.30 (2H, m), 8.20 (1h, d), 8.30 (1H, d), 8.65 (1H, d), 9.10 (1H, bs).
MS (+ve ion electrospray) m/z 422 (MH+).
This was converted to the oxalate salt (0.024g) by the same procedure as for Example 44.

### Example 47. 4-[trans-1-(2-Furyl)-3-propenyl]amino-4-hydroxymethyl-1-[2-(R)-hydroxy-2-(6-methoxyquinolin-4-yl)]ethylpiperidine dioxalate

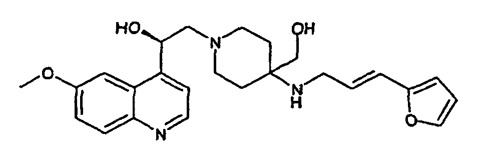

### (a) Allyl 4-benzyloxycarbonylaminopiperidine-4-carboxylate.

The acid intermediate described in Example 37a (9.62g) was esterified by treatment with allyl bromide (2.2ml), potassium carbonate (7.04g) and potassium iodide (catalytic) in acetone (100ml) under reflux for 20 hours. Evaporation, partitioning of the residue between dichloromethane and water and evaporation of the organic phase gave the allyl ester (10.2g). This was then treated with trifluoroacetic acid in dichloromethane for 1.5 hours at room temperature to give, after basification of the triflate salt and extraction with dichloromethane-methanol, the free base (6.58g).

### (b) 4-Benzyloxycarbonylamino-1-[(R)-2-hydroxy-2-(6-methoxyquinolin-4-yl)-ethyl]-piperidine-4-carboxylic acid, allyl ester

A solution of piperidine (47a) (1.85g, 5.8 mmol) and oxirane (1a) (1.2g, 5.8 mmol) in acetonitrile (10ml) was treated with lithium perchlorate (0.62g, 5.8 mmol) and stirred at room temperature for 20 hours. The mixture was evaporated and the residue partitioned between dichloromethane and saturated aqueous sodium bicarbonate solution. The dichloromethane extract was dried and evaporated to give an oil. Chromatography on silica gel (methanol-dichloromethane) afforded the product as an oil (1.6g). MS (+ve ion electrospray) m/z 520 (MH+).

### (c) 4-Amino-1-[(R)-2-hydroxy-2-(6-methoxyquinolin-4-yl)-ethyl]-piperidine-4-carboxylic acid, propyl ester

A solution of (47b) (1.58g) in ethanol (100ml) was hydrogenated over 10% palladium on charcol (0.1g) for 16 hours. Filtration, evaporation, and chromatography on silica gel (methanol/dichloromethane) afforded the product as an oil (0.53g).
MS (+ve ion electrospray) m/z 388 (MH+).

### (d) (R)-2-(4-Amino-4-hydroxymethylpiperidin-1-yl)-1-(6-methoxyquinolin-4-yl)-ethanol

A solution of ester (47c) (0.52 g, 1.3 mmol) in tetrahydrofuran (8ml) was treated at 0°C with a solution of lithium aluminium hydride in tetrahydrofuran (1M; 1.4ml, 1.4 mmol). After 1 hour aqueous sodium hydroxide (2M, 20ml) was added and the mixture was extracted with dichloromethane (30ml). Drying, evaporation, and chromatography on silica gel (methanol-dichloromethane) afforded the product as an oil (0.24g).
MS (+ve ion electrospray) m/z 332 (MH+).

### (e) Title compound

A solution of the amine (47d) (0.14g) and *trans*-(2-furyl)-propenal (0.06g) in dichloromethane (5ml) was treated with sodium triacetoxyborohydride (0.1g). After 16 hours, methanol (1ml) was added followed by sodium borohydride (50mg). After 0.25 hours the mixture was partitioned between dichloromethane and saturated aqueous sodium bicarbonate solution. The dichloromethane extract was dried and evaporated to give an oil. Chromatography on silica (methanol-dichloromethane) afforded an oil (45 mg) which was converted to the dioxalate salt (58 mg) by the procedure of Example (43). ¹H NMR (CDCl₃) δ: 1.80 (4H, m), 2.70 (2H, m), 2.90 (2H, m), 3.30 (2H, d), 3.50 (2H, s), 3.95 (3H, s), 4.05 (2H, m), 5.55 (1H, dd), 6.15-6.25 (2H, m), 6.35-6.50 (2H, m), 7.18 (1H, d), 7.30-7.40 (2H, m), 7.65 (1H, d), 8.05 (1H, d), 8.78 (1H, d)
MS (+ve ion electrospray) m/z 462 (MH+).

### Example 48. 4-[trans-1-(2-Furyl)-3-propenyl]amino-l-t2-(R)-hydroxy-2-(6-methoxyquinolin-4-yl)]ethylpiperidine dioxalate

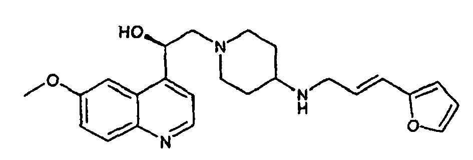

The aminopiperidine (13c) (0.60g,1.99 mmol) and *trans*-3-(2-furyl)propenal (0.24g) were heated under reflux in chloroform (10ml) over magnesium sulphate (2.5g) for 19 hours. After filtation and evaporation of solvent, the residue was dissolved in methanol (10ml), cooled in ice and sodium borohydride (90 mg) was added. After 4 hours stirring at room temperature, the mixture was evaporated and the residue was dissolved in water and extracted with dichloromethane. The extract was dried and evaporated and the residue was chromatographed on silica gel (methanol-dichloromethane) to give an oil (0.63g).
¹H NMR (CDCl₃): δ 1.50 (2H, m), 1.97 (2H, broad t), 2.22 (1H, td), 2.37-2.67 (3H, m), 2.82 (2H, m), 3.26 (1H, m), 3.43 (2H, d), 3.93 (3H, s), 5.43 (1H, dd), 6.21 (1H, d), 6.26 (1H, t), 6.35 (1H, m), 6.39 (1H, d), 7.18 (1H, d), 7.34 (1H, s), 7.36 (1H, dd), 7.64 (1H, d), 8.03 (1H, d), 8.77 (1H, d). MS (+ve ion electrospray) m/z 408 (MH+).

The free base (102mg) in dichloromethane was treated with 0.1 M oxalic acid in ether (5ml) to give the dioxalate salt (126mg).

### Example 49.4-[trans-1-Phenyl-3-propenyl]amino-1-[2-(R)-hydroxy-2-(6-methoxyquinolin-4-yl)]ethylpiperidine dioxalate

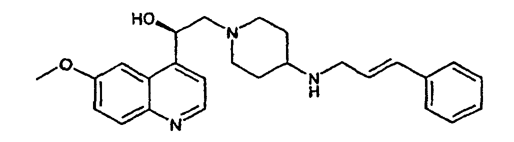

This was prepared from amine (13c) and cinnamaldehyde according to the same procedure as Example (43) affording an oil (11 mg, 15%).
¹H NMR (CDCl₃) δ: 1.50 (2H, m), 2.00 (2H, m), 2.20-2.60 (4H, m), 2.80 (2H, m), 3.25 (1H, m), 3.45 (2H, d), 3.95 (3H, s), 5.40 (1H, dd), 6.30 (1H, dt), 6.50 (1H, d), 7.20-7.40 (7H, m), 7.65 (1H, d), 8.05 (1H, d), 8.75 (1H, d).
MS (+ve ion electrospray) m/z 418 (MH+)
This was converted to the dioxalate (14 mg) by the same procedure as Example (1).

### Example 50. 4-[-3-Phenylpropyl]amino-1-[2-(R)-hydroxy-2-(6-methoxyquinolin-4-yl)]ethylpiperidine dioxalate

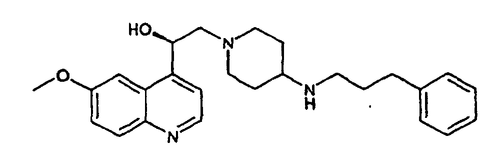

This was prepared from amine (13c) and 3-phenylpropionaldehyde according to the same procedure as Example (43) affording an oil (19mg, 14%), which was converted to the dioxalate salt (18mg) by the same procedure as Example (1).
¹H NMR (CDCl₃) δ: 1.50 (2H, m), 1.80-2.00 (4H, m), 2.20 (1H, t), 2.40 (1H, t), 2.55 (2H, m), 2.65 (4H, m), 2.85 (2H, m), 3.25 (1H, m), 3.90 (3H, s), 5.40 (1H, dd), 7.15 (3H, m), 7.25 (3H, m), 7.35 (1H, dd), 7.65 (1H, d), 8.00 (1H, d), 8.80 (1H, d).
MS (+ve ion electrospray) m/z 420 (MH+)

### Example 51. cis-3-(R/S)-Aminocarbonyl-4-(S/R)-[trans-1-(2-furyl)-3-propenyl]amino-1-[2-(R)-hydroxy-2-(6-methoxyquinolin-4-yl)]ethylpiperidine dioxalate

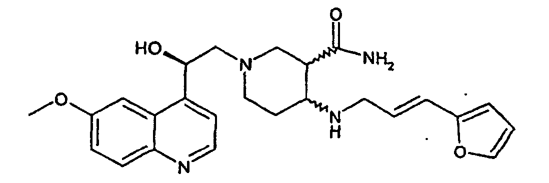

### (a) cis-1-tert-Butoxycarbonyl-3-(R/S)-ethoxycarbonyl-4-(S/R)- [trans-1-(2-furyl)-3-propenyl]amino piperidine.

The aminopiperidine(2c) (1.20g) was reductively alkyated with *trans*-3-(2-furyl)propenal (0.54g) by the method of Example (48). The crude product was chromatographed on silica gel (methanol-dichloromethane) to give the [*trans*-1-(2-furyl)-3-propenyl]amino compound (1.06g).
MS (+ve ion electrospray) m/z 379 (MH+).

### (b) cis-3-(R/S)-Ethoxycarbonyl-4-(S/R)-[trans-1-(2-furyl)-3-propenyl]amino piperidine.

The 1-*tert*-butoxycarbonylpiperidine (51a) (1.06g) was treated with trifluoroacetic acid (5ml) and 1,3-dimethoxybenzene (3.12ml) in dichloromethane (1). After 1.5 hours at room temperature, the mixture was evaporated and the residue was triturated with ether, dissolved in aqueous sodium bicarbonate and extracted with dichloromethane. The extracts were dried and evaporated, and the crude product was chromatographed on silica gel (methanol-dichloromethane) to give the piperidine (0.34g).
MS (+ve ion electrospray) m/z 279 (MH+).

### (c) cis-3-(R/S)-Ethoxycarbonyl-4-(S/R)-[trans-1-(2-furyl)-3-propenyl]amino-1-[2-(R)-hydroxy-2-(6-methoxyquinolin-4-yl)]ethylpiperidine

The piperidine (51b) (0.17g), [R]-2-(6-methoxyquinolin-4-yl)oxirane (1a) (0.12g) and lithium perchlorate (64mg) were stirred in acetonitrile (1ml) at room temp. for 3 days. The solvent was evaporated and the residue was dissolved in ethyl acetate, washed with water and brine, dried and evaporated. The crude product was chromatographed on silica gel (methanol-dichloromethane) to give the hydroxyethylpiperidine (0.16g).
MS (+ve ion electrospray) m/z 480 (MH+)

### (d) Title compound.

The 3-ethoxycarbonylpiperidine (51c) (50mg) was heated with liquid ammonia (2ml) and potassium cyanide (1-2mg) in methanol (2 ml) at 60°C in a pressure bomb. After 48 hours, more ammonia was added and heating continued for 48 hours. The mixture was evaporated and the residue was dissolved in dichloromethane, washed with water, dried and evaporated. Chromatography on silica gel (methanol-dichloromethane) gave the free base (12mg).
¹H NMR (CDCl₃): δ 1.60 (0.5H, broad d), 1.7-2.0 (1.5H, m), 2.15 (0.5 H,d), 2.3-2.5 (2H, m), 2.5-2.65( 2H, m), 2.65-2.8 (2H, m), 2.82 (0.5H, m), 2.96 (1H, m), 3.16 (0.5H, broad d), 3.35-3.6 (3H, m), 3.89 & 3.90 (3H, 2s), 5.40 & 5.54( 1H,2 dd), 5.94 & 6.04 (1H, 2 broad s), 6.17 (1H, dt), 6.21 (1H, m), 6.37 (2H, m), 7.12 & 7.19( 1H, 2d), 7.31 (1H, m), 7.35 (1H, s), 7.58 & 7.63(1H, 2d), 8.00(1H, dd), 8.72 (1H, m), 9.14 & 9.34(1H,2 broad s).
MS (+ve ion electrospray) m/z 451 (MH+).
The free base was converted into the dioxalate salt by the method of Example 1.

### Example 52. 4-[3-(2-Furyl)-propyl]amino-1-[2-(R)-hydroxy-2-(6-methoxyquinolin-4-yl)]ethylpiperidine dioxalate

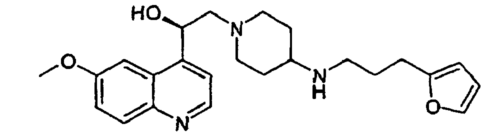

A solution of Example (48) (120mg) in ethanol (10ml) was hydrogenated over 10% palladiunm/charcoal (20mg) for 3 hours. After filtration and evaporation of solvent, the crude product was chromatographed on silica gel (methanol-dichloromethane) to give the free base (80mg).
¹H NMR (CDCl₃): δ 1.92(2H,m), 2.19(5H,m), 2.35(1H,t), 2.56(1H, dd), 2.76(2H,t), 2.89(4H,m), 3.35(1H,d), 3.93(3H,s), 5.46(1H,d), 6.07(1H,d), 6.28(1H,m), 7.16(1H,d), 7.31(d), 7.36(1H,dd), 7.63(1H,d), 8.03(1H,d), 8.77(1H,d).
MS (+ve ion electrospray) m/z 410 (MH+).
The free base was converted into the dioxalate salt by the method of Example 1

### Example 53. cis-4-(S/R)-[3-(2-Furyl)-propyl]amino-3-(R/S)-hydroxymetbyl-[6-methoxyquinolin-4-yl)]aminocarbonylpiperidine oxalate

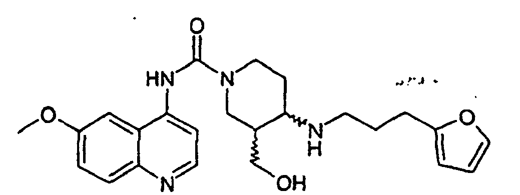

### (a) cis-3-(R/S)-Ethoxycarbonyl-4-(S/R)-[trans-1-(2-furyl)-3-propenyl]amino-1-(6-methoxyquinolin-4-yl)aminocarbonylpiperidine

This was prepared from 4-amino-6-methoxyquinoline (Example 5a) and cis-3-(R/S)-Ethoxycarbonyl-4-(S/R)-[trans-1-(2-furyl)-3-propenyl]amino piperidine (Example 51b) by urea formation by the method of Example (5b,c).
MS (+ve ion electrospray) m/z 479 (MH+).

### (b) Title compound.

The ester (53a) (90mg) in THF (2ml) was treated with lithium aluminium hydride (1M in THF, 0.4ml) at 0°C. After 4 hours the mixture was treated with dilute sodium hydroxide, diluted with ethyl acetate, dried, filtered and evaporated. Chromatography on silica gel (methanol-dichloromethane) gave a mixture of 1-(2-furyl)-3-propenylamino and 3-(2-furyl)propylamino compounds, which was hydrogenated in ethanol over 10% palladium-charcoal for 6 hours to give the title compound free base (26mg).
¹H NMR (CD₃OD): δ 1.59(2Hm), 1.87(3H,m), 2.29(1H, broad), 2.70(3H,m), 3.03(2H,m), 3.18(1H,d), 3.55-3.7(2H,m), 3.86(2H,m), 3.93(3H,s), 4.36(2H,t), 6.06(1H,d), 6.29(1H,d), 7.342H,m), 7.50(1H,d), 7.76(1H,d), 7.84(1H,d), 8.48(1H,d).
MS(+ve ion electrospray) m/z 439 (MH+).
The free base was converted into the oxalate salt by the method of Example 1

### Example 54. 4-[trans-1-(2-Nitrophenyl)-3-propenyl]amino-1-[2-(R)-hydroxy-2-(6-methoxyquinolin-4-yl)] ethylpiperidine dioxalate

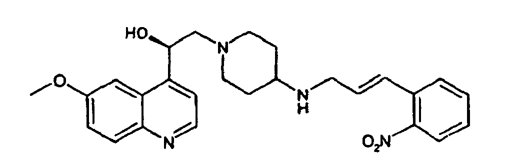

This compound was prepared from the aminopiperidine (Example 13c) (0.6g) and *trans*-2-nitrocinnamaldehyde (0.25g) by the method of Example (43) to give the free base (0.65g).
¹H NMR (CDCl₃): δ 1.49 (2H, m), 2.00 (2H, broad t), 2.25 (1H, td), 2.39-2.70 (3H, m), 2.85 (2H, m), 3.29 (1H, m), 3.52 (2H, d), 3.94 (3H, s), 5.44 (1H, dd), 6.28 (1H, dt), 7.01 (1H, d), 7.19 (1H, d), 7.39 (2H, m), 7.59 (2H, m), 7.64 (1H, d), 7.93 (1H,d), 8.04(1H, d), 8.78 (1H, d).
MS (+ve ion electrospray) m/z 463 (MH+).
The free base was converted into the dioxalate salt by the method of Example 1.

### Example 55. 4-[trans-3-(2-Aminophenyl)-propyl]amino-1-[2-(R)-hydroxy-2-(6-methoxyquinolin-4-yl)]ethylpiperidine dioxalate

This compound was prepared from Example (54) by hydrogenation over 10% palladium on charcoal, converting to the dioxalate salt (52mg) by the same procedure as for Example (1)
MS (+ve ion electrospray) m/z 435 (MH+)

### Example 56. 4-[2-(2-Fluorophenoxy)-ethyl]amino-1-[2-(R)-bydroxy-2-(6-methoxyquinolin-4-yl)]ethylpiperidine dioxalate

This was prepared by the same procedure as for Example (43), MS (+ve ion electrospray) m/z 440 (MH+)

### Example 57. 4-[2-(4-Fluorophenoxy)-ethyl]amino-1-[2-(R)-hydroxy-2-(6-methoxyquinolin-4-yl)]ethylpiperidine dioxalate

This was prepared by the same procedure as for Example (43),
MS (+ve ion electrospray) m/z 440 (MH+)

### Example 58. 4-[2-(3-Trifluoromethylphenoxy)-ethyl]amino-1-[2-(R)-hydroxy-2-(6-methoxyquinolin-4-yl)]ethylpiperidine dioxalate

This was prepared from ketone (1c) and 2-(3-trifluoromethylphenoxy)-ethylamine according to the same procedure as Example (44). The free base was converted to the dioxalate salt by the same procedure as Example (1).
MS (+ve ion electrospray) m/z 490 (MH+)

### Example 59. 4-[2-(Pyridin-3-yloxy)-ethyl]amino-1-[2-(R)-hydroxy-2-(6-methoxyquinolin-4-yl)]ethylpiperidine dioxalate

This was prepared by the same procedure as for Example (45).
MS (+ve ion electrospray) m/z 423 (MH+)

### Example 60. 4-[2-(Pyridin-4-yloxy)-ethyl]amino-1-[2-(R)-hydroxy-2-(6-methoxyquinolin-4-yl)]ethylpiperidine dioxalate

This was prepared by a similar procedure to Example (45).
MS (+ve ion electrospray) m/z 423 (MH+)

### Example 61. 4-[2-(4-Cyanophenoxy)-ethyl]amino-1-[2-(R)-hydroxy-2-(6-methoxyquinolin-4-yl)]ethylpiperidine dioxalate

This was prepared by the same procedure as for Example (43).
MS (+ve ion electrospray) m/z 447 (MH+)

### Example 62. 4-[2-(Pyrimidin-2-yloxy)-ethyl]amino-1-[2-(R)-hydroxy-2-(6-methoxyquinolin-4-yl)]ethylpiperidine dioxalate

This was prepared by a similar procedure to Example (45).
MS (+ve ion electrospray) m/z 424 (MH+)

### Example 63. 4-[(trans-1-(4-Fluorophenyl)-3-propenyl]amino-1-[2-(R)-hydroay-2-(6-methoxyquinolin-4-yl)]ethylpiperidine dioxalate

This was prepared from 4-fluorophenylcinnamaldehyde by the same procedure as for Example (48).
MS (+ve ion electrospray) m/z 436 (MH+)

### Example 64. 4-[trans-1-(2-Methoxyphenyl)-3-propenyl]amino-1-[2-(R)-hydroay-2-(6-methoxyquinolin-4-yl)]ethylpiperidine dioxalate

This was prepared from 2-methoxycinamaldehyde by the same procedure as for Example (48).
MS (+ve ion electrospray) m/z 448 (MH+)

### Example 65. 4-[trans-1-(4-Methoxyphenyl)-3-propenyl]amino-1-[2-(R)-hydroxy-2-(6-methoxyquinolin-4-yl)]ethylpiperidine dioxalate

This was prepared using 4-methoxycinnamaldehyde by the same procedure as for Example (48).
MS (+ve ion electrospray) m/z 448 (MH+)

### Example 66. 4-{trans-1-[3,5-difluorophenyl]-3-propanyl}amino-1-[2-(R)-hydroxy-2-(6-methoxyquinolin-4-yl)]ethylpiperidine dioxalate

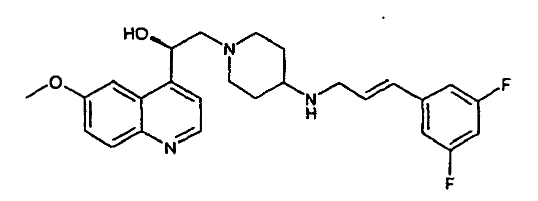

### (a) (trans)-3-(3,5-Difluorophenyl)-prop-2-en-1-ol

A solution of (trans)-3-(3,5-difluorophenyl)-acrylic acid (1 g) in tetrahydrofuran (10 ml) was treated at 0°C with triethylamine (0.9 ml), then isobutyl chloroformate (0.8 ml) was added dropwise. After 1 hour, the mixture was filtered and the filtrate diluted with ice/water (10 g). Sodium borohydride (0.5g) was added. After 3 hours, 2M aqueous hydrochloric acid was added. The mixture was partitioned between dichloromethane and brine. The organic extract was dried and evaporated. Chromatography on silica gel (ethyl acetate-hexane) afforded the alcohol as an oil (0.4g). (See R. J. Alabaster *et al*, Synthesis 1989, 598 for a related procedure.)
MS (+ve ion electrospray) m/z 171 (MH+)

### (b) (trans)-3-(3,5-Difluorophenyl)-propenal

A solution of (66a) (0.2g) in dichloromethane (3 ml) was treated with manganese (II) dioxide (0.52g). After 16 hours, the mixture was filtered and evaporated to give the aldehyde as a white solid (0.18g).
MS (+ve ion electrospray) m/z 169 (MH+)

### (c) Title compound

This was prepared from aldehyde (66b) (0.73g) and amine (13c) (0.1g) by reductive alkylation using sodium borohydride using the same procedure as for Example 48, giving the title compound as an oil (0.08g) after chromatography.
'H NMR (CDCl₃) δ: 1.55 (2H, m), 2.00 (2H, m), 2.25(1H, m), 2.40-2.65 (3H, m), 2.85(2H, m), 3.30 (1H, m), 3.50 (2H, d), 3.90 (3H, s), 5.45 (1H, dd), 6.30 (1H, m), 6.45 (1H, d),), 6.65 (1H, m),), 6.85(2H, m),7.15 (1H, m), 7.35 (1H, dd), 7.65 (1H, d), 8.05 (1H, d), 8.75 (1H, d)
MS (+ve ion electrospray) m/z 454 (MH+)
The free base was converted to the dioxalate in the normal manner.

### Example 67. 4-{trans-1-[Pyridin-2-yl]-3-propenyl}amino-1-[2-(R)-hydroxy-2-(6-metboxyquinolin-4-yl)]ethylpiperidine dioxalate

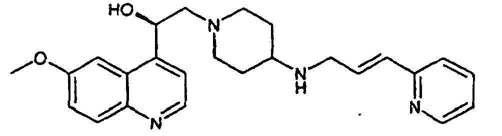

This was prepared from (*trans*)-3-pyridin-2-yl-acrylic acid by the same procedure as for Example (66).
¹H NMR (CDCl₃) δ: 1.55 (2H, m), 2.00 (2H, m), 2.25(1H, m), 2.35-2.70 (3H, m), 2.85(2H, m), 3.28 (1H, m), 3.55 (2H, d), 3.90 (3H, s), 5.45 (1H, dd), 6.68 (1H, m), 6.80 (1H, m),7.15 (2H, m), 7.25-7.40 (2H, m), 7.65 (2H, m), 8.05 (1H, d), 8.50 (1H, d) 8.78 (1H, d)
MS (+ve ion electrospray) m/z 419 (MH+)
The free base was converted to the dioxalate in the normal manner.

### Example 68 (R)-1-(6-Methoxy-[1,5]naphthyridin-4-yl)-2-{4-[2-(pyridin-2-yloxy)-ethylamino]-piperidin-1-yl}-ethanol dioxalate

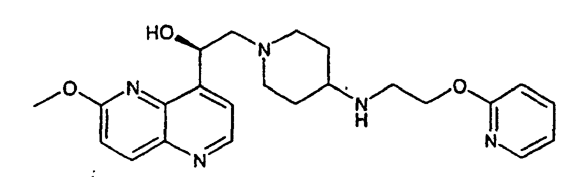

### (a) 1-[(R)-2-Hydroxy-2-(6-methoxy-[1,5]naphthyridin-4-yl)-ethyl]-piperidin-4-one

This was prepared by reaction of the naphthyridine oxirane (31d) with 1,4-dioxa-8-azaspiro-[4,5]-decane, followed by acidic hydrolysis of the ketal to give the ketone, by the same procedures as for Example (1c).
MS (+ve ion electrospray) m/z 302 (MH+).

### (b) Title compound

This was prepared by reductive alkyation of the above ketone (68a) with 2-(pyridin-2-yloxy)-ethylamine according to the procedure for Example 45, affording the title compound as an oil (62 mg, 55%).
¹H NMR (CDCl₃) δ:1.55 (2H, m), 2.00 (2H, m), 2.25(1H, m), 2.35-2.70 (3H, m), 2.85(2H, m), 3.05-3.15 (3H, m), 3.30 (1H, m), 3.95 (3H, s), 4.45 (2H, t),5.70 (1H, dd) 6.75 (1H, m), 6.85 (1H, m),7.15 (1H, d), 7.55 (1H, m), 7.85 (1H, d), 8.15 (1H, m), 8.20 (1H, d) 8.78 (1H, d)
MS (+ve ion electrospray) m/z 424 (MH+)
The free base was converted to the dioxalate in the normal manner.

### Example 69 (R)-1-(6-Methoxy-[1,5]naphthyridin-4-yl)-2-[4-((E)-3-pyridin-2-yl-allylamino)-piperidin-1-yl]-ethanol dioxalate salt

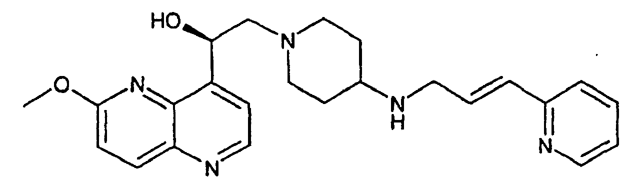

This was prepared by reductive alkylation of the amine (31f) with aldehyde (66b) according to the procedures of Example 66, affording an oil (35 mg, 32%)
¹H NMR (CDCl₃) δ: 1.55 (2H, m), 2.00 (2H, m), 2.25(1H, m), 2.35-2.70 (3H, m), 2.85(2H, m), 3.28 (1H, m), 3.55 (2H, d), 4.00 (3H, s), 5.45 (1H, dd), 6.66 (1H, m), 6.75 (1H, m),7.15 (2H, m), 7.30 (1H, m), 7.65 (1H, dt), 7.80 (1H, d), 8.20 (1H, d), 8.55 (1H, d) 8.78 (1H, d)
MS (+ve ion electrospray) m/z 420 (MH+)
The free base was converted to the dioxalate in the normal manner.

### Example 70. 4-[2-(3,5-Difluorophenylamino)-ethyl]amino-1-[2-(R)-hydroxy-2-(6-methoxyquinolin-4-yl)]ethylpiperidine dioxalate

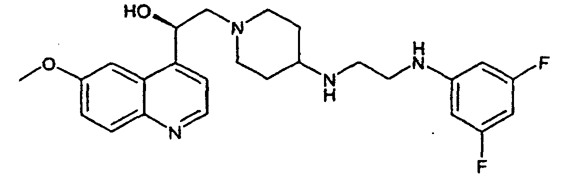

A solution of Example 29 (0.16g) in tetrahydrofuran (5 ml) was treated with a solution of lithium aluminium hydride in tetrahydrofuran (1M; 1.4 ml, 1.4 mmol). After 7h the mixture was treated with 8% aqueous sodium hydroxide solution, ethyl acetate, and sodium sulphate. Filtration, evaporation and chromatography on silica eluting with a methanoi/dichloromethane gradient afforded an oil (38 mg, 25%)
¹H NMR (CDCl₃) δ: 1.5 (2H, m), 2.00 (2H, m), 2.23(1H, m), 2.35-2.60 (3H, m), 2.80-2.95(4H, m), 3.10-3.30 (3H, m), 3.92 (3H, s), 4.48 (1H, bs), 5.43 (1H, dd), 6.13 (3H, m), 7.19 (1H, d), 7.38 (1H, dd), 7.69 (1H, d), 8.04 (1H, d), 8.78 (1H, d)
MS (+ve ion electrospray) m/z 457 (MH+)
The free base was converted to the dioxalate in the nonnal manner.

### Example 71. 4-[2-(3-Fluorophenylamino)-ethyl]amino-1-[2-(R)-hydroxy-2-(6-methoxyquinolin-4-yl)]ethylpiperidine dioxalate

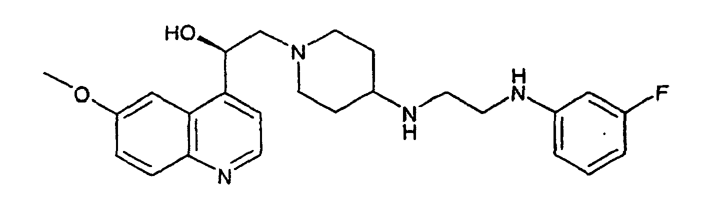

### (a) 2-(3-Fluorophenylamino)-ethylamine

A mixture of 3-fluoroaniline (1g, 9 mmol), 2-bromoethylamine hydrobromide (1.8g, 9 mmol) and toluene (10 ml) was heated to reflux under argon for 8h. The toluene was decanted off and the residue dissolved in water, basified with potassium hydroxide and extracted with dichloromethane. The dichloromethane extracts were dried (sodium sulphate) and evaporated to give an oil. Chromatography on silica eluting with a methanol/dichloromethane gradient afforded an oil (0.44g, 32%).
MS (+ve ion electrospray) m/z 155 (MH+)

### (b) Title compound

This was prepared by reductive alkylation of amine (71c) and ketone (1c) using sodium triacetoxyborohydride according to the procedure of Example 44, affording the title compound as an oil (0.42g, 79%):
¹H NMR (CDCl₃) δ: 1.5 (2H, m), 2.00 (2H, m), 2.23(1H, m), 2.35-2.60 (3H, m), 2.80-2.95(4H, m), 3.10-3.30 (3H, m), 3.94 (3H, s), 4.40 (1H, bs), 5.45 (1H, dd), 6.30-6.50 (3H, m), 7.15(1H, m), 7.20(1H, d), 7.40 (1H, dd), 7.70 (1H, d), 8.08 (1H, d), 8.78 (1H, d)
MS (+ve ion electrospray) m/z 439 (MH+)
The free base was converted to the dioxalate in the normal manner.

### Example 72 (E)-3-Furan-2-yl-N-{1-[(R)-2-hydroxy-2-(6-methoxyquinolin-4-yl)-ethyl]-piperidin-4-yl}-acrylamide oxalate

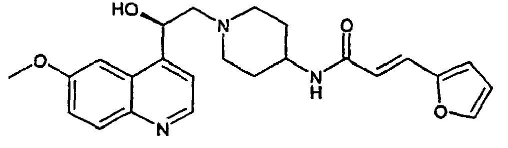

To a solution of (E)-3-furan-2-ylacrylic acid (28mg, 0.2 mmol) and di-isopropylethylamine (0.07 ml, 0.4 mmol) in N,N-dimethylformamide (0.2 ml) were added 1-[3-(dimethylamino)propyl]-3-ethylcarbodiimide hydrochloride (38mg, 0.2mmol) in dry dichloromethane (1.5 ml) and aminopiperidine (13c), (50mg, 0.17mmol) in dry dichloromethane (1 ml). The mixture was shaken for a short period then allowed to stand overnight. The mixture was then washed well with water, dried and evaporated. The crude product was chromatographed on silica gel eluted with 0-5% methanol/dichloromethane to give the free base of title compound (46mg, 64%).
¹H NMR (CDCl₃) δ 1.6 (2H, m), 2.09 (2H, m), 2.34 (1H, m), 2.53 (2H, m), 2.82 (1H, m), 2.87 (1H, dd), 3.27 (1H,m) 3.93 (3H,s & 1H, m), 5.45 (1H, dd), 5.59 (1H, d), 6.29 (1H, d), 6.47 (1H, m), 6.56 (1H, d), 7.17 (1H, d), 7.38 (1H, dd), 7.44 (2H, m), 7.64 (1H, d), 8.04 (1H, d), 8.78 (1H, d)
MS (+ve ion electrospray) m/z 422 (MH+).
The free base in chloroform/dichloromethane was treated with one equivalent of oxalic acid in ether to give the oxalate salt.

### Example 73. 4-[trans-1-(2-Thiazolyl)-3-propenyl]amino-1-[2-(R)-hydroxy-2-(6-methoxy-[1,5]-naphthyridin-4-yl)]ethylpiperidine dimethanesulphonate

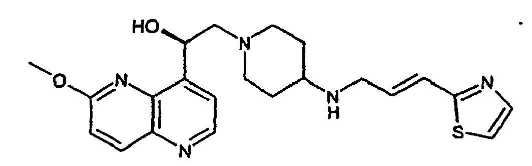

### (a) (E)-3-Thiazol-2-yl-propenal

A mixture of formylmethylene triphenylphosphorane (0.9g) and thiazole-2-carboxaldehyde (0.33g) in toluene (20 ml) was heated at 40°C for 4h. The mixture was chromatographed on silica eluting with an ethyl acetate-dichloromethane gradient affording a white solid (0.25g).
¹H NMR (CDCl₃): δ 6.90 (1H, dd), 7.55 (1H, d), 7.65 (1H, d), 8.00 (1H, d), 9.75 (1H, d) MS (+ve ion electrospray) m/z 140 (MH+)

### (b) Title compound

This was prepared by reductive alkylation of amine (31f) and aldehyde (73a) according to the procedure of Example 48 affording the free base of the title compound as an oil (0.4g, 80%).
¹H NMR (CDCl₃): δ: 1.45-1.55 (2H, m), 1.90-2.05 (2H, m), 2.20 (1H, m), 2.40-3.10 (5H, m), 3.60 (2H, d), 4.05 (3H, s), 5.75 (1H, dd), 6.68 (1H, t), 6.80 (1H, s), 7.10 (1H, d), 7.20 (1H, d), 7.73 (1H, d), 7.80 (1H, d), 8.20 (1H, d), 8.78 (1H, d)
MS (+ve ion electrospray) m/z 426 (MH+)

This was converted to the dimethanesulphonate salt by adding an ether solution of methanesulphonic acid (2 equivalents) to a solution of the free base in chloroform, followed by isolation by centrifugation (0.45g).

### Example 74. 4-[2-(Pyridin-2-yloxy)-ethylamino]-1-[2-(R)-hydroxy-2-(8-fluoro-6-methoxyquinolin-4-yl)]ethylpiperidine dioxalate

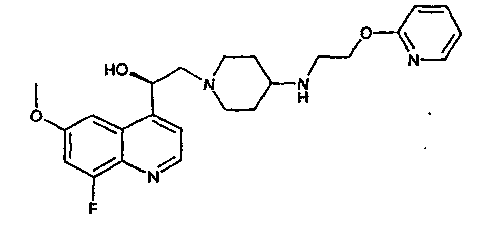

### (a) 8-Fluoro-6-methoxy-quinolin-4-ol

2-Fluoro-4-methoxy-phenylamine (3.80g;26.7mmol) and methyl propiolate (2.37ml, 0.267mol) in methanol (100ml) was stirred for 72 hours at room temperature, then heated at 50°C for 24 hours. It was evaporated and the product purified by chromatography on silica gel (dichloromethane) to give a solid (1.66g), a portion of which was recrystallised from dichloromethane-hexane.

The unsaturated ester (0.96g) in warm Dowtherm A (5ml) was added over 3 minutes to refluxing Dowtherm A (15ml), and after a further 20 minutes at reflux the mixture was cooled and poured into ether. The precipate was filtered to give the title compound (0.50g, 61%)

### (b) Bromomethyl-(8-fluoro-6-methoxy-quinolin-4-yl)-ketone

This was prepared from (74a) by the method of Example (31b).
MS (+ve ion electrospray) m/z 298/300 (MH+).

### (c) 8-Fluoro-6-methoxy-4-(R)-oxiranyl-quinoline

The bromomethyl ketone (74b) (8.6g) was converted to the oxirane (1.04g) by the methods of Examples (31c,d).
MS (+ve ion electrospray) m/z 220 (MH+).

### (d) 1-[(R)-2-(8-Fluoro-6-methoxy-quinolin-4-yl)-2-hydroxy-ethyl]-piperidin-4-one

This was prepared from oxirane (74c) (1.04g) by the method of Example (1b,c) to give the ketone (1.1g) as a foam.
MS (+ve ion electrospray) m/z 319 (MH+).

### (e) Title compound

This was prepared from ketone (74d) and amine (45a) according to the procedure of Example 45, affording the free base as an oil (84 mg, 65%).
¹H NMR (CDCl₃) δ: 1.60 (2H, m), 1.95 (2H, m), 2.15 (1H, m), 2.50 (3H, m), 2.80 (2H, m), 3.08 (2H, t), 3.25 (1H, m), 3.95 (3H, s), 4.45 (2H, t), 5.45 (1H, dd), 6.75 (1H, d), 6.85 (1H, m), 7.00 (1H, m), 7.15 (1H, m), 7.40 (1H, dd), 7.60 (1H, m), 7.70 (1H, m), 8.15 (1H, m), 8.80 (1H, d).
MS (+ve ion electrospray) m/z 441 (MH+).

This was converted to the dioxalate salt (112 mg) in the usual way

### Example 75. 5-Fluoro-1-(3-{1-[(R)-2-hydroxy-2-(6-methoxy-quinolin-4-yl)-ethyl]-piperidin-4-ylamino}-propyl)-1H-pyrimidine-2,4-dione dioxolate

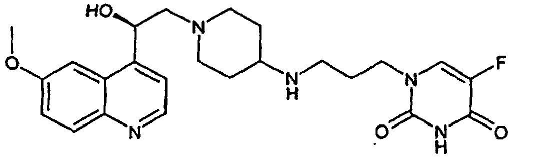

### (a) 1-(3,3-Dimethoxy-propyl)-5-fluoro-1H-pyrimidine-2,4-dione

A mixture of 5-fluoro-1H-pyrimidine-2,4-dione (1 g), potassium carbonate (1 g) and 3-bromo-1,1-dimethoxy-propane (1.4 g) in N,N-dimethylformamide (10 ml) was heated overnight at 70°C. The mixture was partitioned between ether and water. The ether extract was dried and evaporated to afford an oil (0.8gg). MS (+ve ion electrospray) m/z 233 (MH+).

### (b) 3-(5-Fluoro-2,4-dioxo-3,4-dibydro-2H-pyrimidin-1-yl)-propionaldehyde

The above compound was dissolved in a 2% solution of water in tetrahydrofuran (5 ml), treated with acidic Amberlyst-15 resin (0.75g) and heated at 60°C for 10 hours. The mixture was filtered and evaporated affording the aldehyde as an oil (0.2g).
MS (+ve ion electrospray) m/z 187 (MH+)

### (c) Title compound

This was prepared from aldehyde (75b) (0.2g) and aminopiperidine (13c) (0.15g) by reductive alkylation according to the procedure of Example 43b to give the title compound as an oil (23 mg)
¹H NMR (d-6 MeOH) δ 1.6-1.8 (2H, m), 2.0-2.2 (4H, m), 2.3-2.4 (2H, m), 2.7-2.8 (2H, m), 2.9-3.0 (2H, m), 3.2-3.3 (2H, m), 3.7 (1H, m), 3.8 (2H, m), 3.95 (3H, s), 5.60 (1H, m), 7.30-7.40 (2H, m), 7.65 (1H, d, 7.85 (1H, d), 7.95 (1H, d), 8.65 (1H, d)
MS (+ve ion electrospray) m/z 472 (MH+)

This was converted to the dioxolate salt (30 mg) in the usual way
The following Examples of Table I were prepared by analogous methods.
A: by method of Example 43
B: by method of Example 55
C: by method of Example 62
D: by method of Example 48
E: by method of Example 52
F: by method of Example 44
G: by method of Example 71
H: by method of Example 45
I: by method of Example 70
J: by method of Example 13
K: by method of Example 1
L: by method of Example 33
M: Prepared in ca 2% yield by alkylation of 4-amino-1-[(R)-2-hydroxy-2-(6-methoxy-quinolin-4-yl)-ethyl]-piperidine-4-carboxylic acid carbamoylmethyl-amide with (E)-(3-bromo-propenyl)-benzene.
N: by method of Example 72
O: by method of Example 75
Salts:
   AY = dioxalate
   DZ = dimesylate
   G = oxalate

**Table 1**

| **Example** | **Method of Synthesis** | **Isolated as salt** | **D** | **AB** | **X** | **L** | **R** |
|---|---|---|---|---|---|---|---|
| 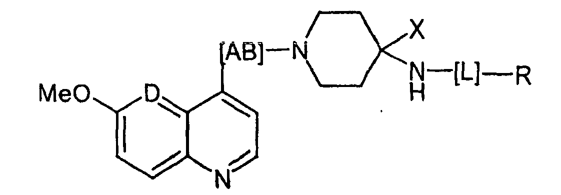 | | | | | | | |
| 200 | A | AY | CH | (R)-CH(OH)CH₂ | H | CH₂CH₂O | 3-fluorophenyl |
| 201 | A | AY | CH | (R)-CH(OH)CH₂ | H | CH₂CH₂O | 3,4-difluorophenyl |
| 202 | A | AY | CH | (R)-CH(OH)CH₂ | H | CH₂CH₂O | 2,3-difluorophenyl |
| 203 | A | AY | CH | (R)-CH(OH)CH₂ | H | CH₂CH₂O | 2,5-difluorophenyl |
| 204 | A | AY | CH | (R)-CH(OH)CH₂ | H | CH₂CH₂O | 2,4-difluorophenyl |
| 205 | A | AY | CH | (R)-CH(OH)CH₂ | H | CH₂CH₂O | 3,4,5-trifluorophenyl |
| 206 | A | AY | CH | (R)-CH(OH)CH₂ | H | CH₂CH₂O | 2,3-difluoro-6-nitrophenyl |
| 207 | A | AY | CH | (R)-CH(OH)CH₂ | H | CH₂CH₂O | 3,4-dichlorophenyl |
| 208 | A | AY | CH | (R)-CH(OH)CH₂ | H | CH₂CH₂O | 2-fluoro-6-methylphenyl |
| 209 | A | AY | CH | (R)-CH(OH)CH₂ | H | CH₂CH₂O | 2-bromo-5-fluorophenyl |
| 210 | A | AY | CH | (R)-CH(OH)CH₂ | H | CH₂CH₂O | 2-chloro-4-fluorophenyl |
| 211 | A | AY | CH | (R)-CH(OH)CH₂ | H | CH₂CH₂O | 2-chloro-3-trifluoromethylphenyl |
| 212 | A | AY | CH | (R)-CH(OH)CH₂ | H | CH₂CH₂O | 2-cyanophenyl |
| 213 | A | AY | CH | (R)-CH(OH)CH₂ | H | CH₂CH₂O | 2-chloro-3,5-difluorophenyl |
| 214 | A | AY | CH | (R)-CH(OH)CH₂ | H | CH₂CH₂O | 2-fluoro-6-nitrophenyl |
| 215 | A | AY | CH | (R)-CH(OH)CH₂ | H | CH₂CH₂O | 2-fluoro-5-trifluoromethylphenyl |
| 216 | A | AY | CH | (R)-CH(OH)CH₂ | H | CH₂CH₂O | 4-fluoro-2-nitrophenyl |
| 217 | A | AY | CH | (R)-CH(OH)CH₂ | H | CH₂CH₂O | 2-nitrophenyl |
| 218 | A | AY | CH | (R)-CH(OH)CH₂ | H | CH₂CH₂O | 2-chloro-5-nitromethylphenyl |
| 219 | A | AY | CH | (R)-CH(OH)CH₂ | H | CH₂CH₂O | 2-methyl-5-fluorophenyl |
| 220 | A | AY | CH | (R)-CH(OH)CH₂ | H | CH₂CH₂O | 3-cyanophenyl |
| 221 | A | AY | CH | (R)-CH(OH)CH₂ | H | CH₂CH₂O | 3-fluoro-2-nitrophenyl |
| 222 | B | KT | CH | (R)-CH(OH)CH₂ | H | CH₂CH₂O | 2-aminophenyl |
| 223 | B | KT | CH | (R)-CH(OH)CH₂ | H | CH₂CH₂O | 6-amino-3-fluorophenyl |
| 224 | A | AY | CH | (R)-CH(OH)CH₂ | H | CH₂CH₂O | 2-methoxyphenyl |
| 225 | A | AY | CH | (R)-CH(OH)CH₂ | H | CH₂CH₂O | 2-trifluoromethylphenyl |
| 226 | C | AY | CH | (R)-CH(OH)CH₂ | H | CH₂CH₂O | pyrimidin-5-yl |
| 227 | A | AY | CH | (R)-CH(OH)CH₂ | H | CH₂CH₂O | 2,6-difluorophenyl |
| 228 | A | AY | CH | (R)-CH(OH)CH₂ | H | CH₂CH₂O | 3,5-dimethoxyphenyl |
| 229 | A | AY | CH | (R)-CH(OH)CH₂ | H | CH₂CH₂O | 2,3-dimethoxyophenyl |
| 230 | A | AY | CH | (R)-CH(OH)CH₂ | H | CH₂CH₂O | 3-methoxyphenyl |
| 231 | A | AY | CH | (R)-CH(OH)CH₂ | H | CH₂CH₂O | 2-acetamidophenyl |
| 232 | A | AY | CH | (R)-CH(OH)CH₂ | H | CH₂CH₂O | 3-methoxycarbonylphenyl |
| 300 | D | DZ | CH | (R)-CH(OH)CH₂ | H | (*E*)CH₂CH=CH | thiazol-2-yl |
| 301 | E | AY | CH | (R)-CH(OH)CH₂ | H | CH₂CH₂CH₂ | 3,5-difluorophenyl |
| 302 | D | DZ | CH | (R)-CH(OH)CH₂ | H | (*E*)CH₂CH=CH | pyridin-3-yl |
| 303 | O | AY | CH | (R)-CH(OH)CH₂ | H | CH₂CH₂CH₂ | 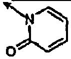 |
| 304 | O | AY | CH | (R)-CH(OH)CH₂ | H | CH₂CH₂CH₂ | 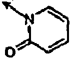 |
| 305 | F | AY | CH | (R)-CH(OH)CH₂ | H | CH₂CH₂CH₂ | 3-imidazol-1-yl |
| 306 | O | AY | CH | (R)-CH(OH)CH₂ | H | CH₂CH₂CH₂ | 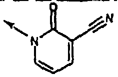 |
| 307 | D | AY | CH | (R)-CH(OH)CH₂ | H | (*E*)CH₂CH=CH | 4-dimethylaminophenyl |
| 308 | F | AY | CH | (R)-CH(OH)CH₂ | H | CH₂CH₂CH₂ | 1-*H*-[1,2,4]triazol-5-yl |
| 309 | A | AY | CH | (R)-CH(OH)CH₂ | H | CH₂CH₂CH₂ | 4-fluorophenyl |
| 310 | A | AY | N | (R)-CH(OH)CH₂ | H | CH₂CH₂O | 3,5-difluorophenyl |
| 311 | M | G | CH | (R)-CH(OH)CH₂ | CONHCH₂CONH₂ | (*E*)CH₂CH=CH | phenyl |
| 400 | A | s | CH | NHCO | H | CH₂CH₂O | 3,5-difluorophenyl |
| 401 | A | G | N - | NHCO | H | CH₂CH₂O | 3,5-difluorophenyl |
| 402 | H | AY | N | NHCO | H | CH₂CH₂O | pyridin-2-yl |
| 403 | D | AY | N | NHCO | H | (*E*)CH₂CH=CH | pyridin-2-yl |
| 404 | I | AY | N | NHCO | H | CH₂CH₂NH | 3,5-difluoroplienyl |
| 405 | G | AY | N | NHCO | H | CH₂CH₂NH | 3-fluorophenyl |
| 500 | F | DZ | CH | (R)-CH(OH)CH₂ | H | CH₂CH₂NH | 6-fluoropyridin-2-yl |
| 501 | F | AY | CH | (R)-CH(OH)CH₂ | H | CH₂CH₂NH | phenyl |
| 502 | F | AY | CH | (R)-CH(OH)CH₂ | H | CH₂CH₂NH | 3-trifluoromethylpyridin-2-yl |
| 503 | F | AY | CH | (R)-CH(OH)CH₂ | H | CH₂CHNH | pyridin-2-yl |
| 504 | F | G | CH | (R)-CH(OH)CH₂ | H | CH₂CH₂NH | 4-azido-2-nitrophenyl |
| 505 | F | G | CH | (R)-CH(OH)CH₂ | H | CH₂CH₂NH | 5-nitropyridin-2-yl |
| 506 | F | G | CH | (R)-CH(OH)CH₂ | H | CH₂CH₂NH | 6-nitropyridin-2-yl |
| 507 | F | AY | N | (R)-CH(OH)CH₂ | H | CH₂CH₂NH | 3-fluorophenyl |
| 601 | J | AY | CH | (R)-CH(OH)CH₂ | H | CH₂CONH | 3-fluorophenyl |
| 602 | J | AY | N | (R)-CH(OH)CH₂ | H | CH₂CONH | pyrid-2-yl |
| 603 | J | AY | N | (R)-CH(OH)CH₂ | H | CH₂CONH | 3,5-difluorophenyl |
| 604 | K | AY | CH | (R)-CH(OH)CH₂ | H | CH₂CONH | pyrazyl |
| 605 | J | AY | CH | (R)-CH(OH)CH₂ | H | CH₂CONH | 6-methylpyrid-2-yl |
| 606 | J | AY | CH | (R)-CH(OH)CH₂ | H | CH₂CONH | 3-methoxyphenyl |
| 607 | J | AY | CH | (R)-CH(OH)CH₂ | H | CH₂CONH | 2-*n*-butylphenyl |
| 608 | J | AY | CH | (R)-CH(OH)CH₂ | H | CH₂CONH | 3-*n*-butylaminosulphonylphenyl |
| 609 | L | AY | CH | (R)-CH(OH)CH₂ | H | CH₂CONH | 3-carboxyphenyl |
| 610 | N | AY | CH | (R)-CH(OH)CH₂ | H | *E*-COCH=CH | 3,5-difluorophenyl |
| 611 | N | AY | CH | (R)-CH(OH)CH₂ | H | *E*-COCH=CH | 3-thienyl |
| 612 | N | AY | CH | (R)-CH(OH)CH₂ | H | *E*-COCH=CH | 3-bromo-5-fluorophenyl |
| 613 | N | AY | CH | (R)-CH(OH)CH₂ | H | *E*-COCH=CH | 3-fluoro-pyridin-2-yi |
| 614 | N | G | CH | (R)-CH(OH)CH₂ | H | *E*-COCH=CH | 2-nitrophenyl |
| 615 | N | G | CH | (R)-CH(OH)CH₂ | H | COCH₂O | phenyl |
| 616 | J | DZ | CH | (R)-CH(OH)CH₂ | H | CH₂CONH | 3-hydroxyphenyl |

### Biological Activity

The MIC (µg/ml) of test compounds against various organisms was determined: **S. aureus Oxford, S. aureus WCUH29, S. pneumoniae 1629, S. pneumoniae N1387, S. pneumoniae ERY 2, E. faecalis 1, E. faecalis 7, H. influenzae Q1, H. influenzae NEMC1, M. catarrhalis 1502, E. coli 7623, E.coli 120.**
Examples 1, 2, 13, 17, 19, 20, 29, 43-52, 54, 56-58, 63, 66-71, 73, 74, 200-217, 219, 221, 222, 225, 300, 301 304, 310, 401, 404, 500, 501, 507, 601-604 have an MIC of less than or equal to 0.25µg/ml; 3,4,11, 18,21-25, 38, 41, 42, 53, 55, 59, 62, 64, 72, 218, 220, 223, 224, 231, 302, 303, 402, 403, 405, 502-504, 506, 605, 606, 610-614 have an MIC of less than or equal to 2µg/ml; 5,6, 12, 26-28, 34, 36, 37, 75, 226-230, 232, 311, 400, 505, 607-610 have an MIC less than or equal to 32µg/ml; others have an MIC level >32ug/ml against one or more of the above range of gram positive and gram negative bacteria.

## Claims

1. A compound of formula (I) or a pharmaceutically acceptable derivative thereof: 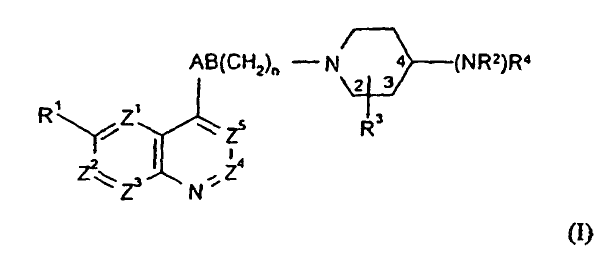 wherein:
one of Z¹, Z², Z³, Z⁴ and Z⁵ is N, one:is CR^{1a} and the remainder are CH , or one or two of Z¹, Z², Z³, Z⁴ and Z⁵ are independently CR^{1a} and the remainder are CH;
R¹ and R^{1a} are independently hydrogen; hydroxy; (C₁₋₆)alkoxy optionally substituted by (C ₁₋₆)alkoxy, amino, piperidyl, guanidino or amidino any of which is optionally N- substituted by one or two (C₁₋₆)alkyl, acyl or (C₁₋₆)alkylsulphonyl groups, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted(C₁₋₆)alkyl; halogen; (C₁₋₆)alkyl; (C₁₋₆)alkylthio; trifluoromethyl; trifluoromethoxy; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups;
or when Z⁵ is CR^{1a}, R^{1a} may instead be cyano, hydroxy methyl or carboxy;
provided that when Z¹, Z², Z³, Z⁴ and Z⁵ are CR^{1a} or CH, then R¹ is not hydrogen;
R² is hydrogen, or (C₁₋₄)alkyl or (C₂₋₄)alkenyl optionally substituted with 1 to 3 groups selected from:
amino optionally substituted by one or two (C₁₋₄)alkyl groups; carboxy; (C₁₋₄)alkoxycarbonyl; (C₁₋₄)alkylcarbonyl; (C₂₋₄)alkenyloxycarbonyl; (C₂₋₄)alkenylcarbonyl; aminocarbonyl wherein the amino group is optionally substituted by hydroxy, (C₁₋₄)alkyl, hydroxy(C₁₋₄)alkyl, aminocarbonyl(C₁₋₄)alkyl, (C₂₋₄)alkenyl, (C₁₋₄)alkylsulphonyl, trifluoromethylsulphonyl, (C₂₋₄)alkenylsulphonyl, (C₁₋₄)alkoxycarbonyl, (C₁₋₄)alkylcarbonyl, (C₂₋₄)alkenyloxycarbonyl or (C₂₋₄)alkenylcarbonyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; 5-oxo-1,2,4-oxadiazol-3-yl; halogen; (C₁₋₄)alkylthio; trifluoromethyl; hydroxy optionally substituted by (C₁₋₄)alkyl, (C₂₋₄)alkenyl, (C₁₋₄)alkoxycarbonyl, (C₁₋₄)alkylcarbonyl, (C₂₋₄)alkenyloxycarbonyl, (C₂₋₄)alkenylcarbonyl; oxo; (C ₁₋₄)alkylsulphonyl; (C₂₋₄)alkenylsulphonyl; or (C₁₋₄)aminosulphonyl wherein the amino group is optionally substituted by (C₁₋₄)alkyl or (C₂₋₄)alkenyl;
R³ is hydrogen; or
R³ is in the 2-, 3- or 4-position and is:
carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C ₁₋₆)alkyl, hydroxy(C ₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
(C₁₋₄)alkyl or ethenyl optionally substituted with any of the substituents listed above for R³ and/or 0 to 2 groups R¹² independently selected from:
halogen; (C₁₋₆)alkylthio; trifluoromethyl; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylearbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocarbonyl(C ₁₋₆)alkyl, (C₂₋₆)alkenyl, (C₁₋₆)alkoxycarbonyl, (C ₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl; or when R³ is in the 3-position, hydroxy optionally substituted as described above;
in addition when R³ is disubstituted with a hydroxy or amino containing substituent and carboxy containing substituent these may together form a cyclic ester or amide linkage, respectively;
R⁴ is a group -X ¹-X²-X³-X⁴ in which:
X ¹ is CH₂, CO or SO₂;
X² is CR¹⁴R¹⁵;
X³ is NR¹³, O, S, SO₂ or CR¹⁴R¹⁵; wherein:
each of R¹⁴ and R¹⁵ is independently selected from: hydrogen; halo; (C₁₋₄)alkoxy; (C₁₋₄)alkylthio; trifluoromethyl; cyano; carboxy; nitro; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; carboxy(C₁₋₄)alkyl; halo(C₁₋₄)alkoxy; halo(C₁₋₄)alkyl; (C₁₋₄)alkylcarbonyloxy; (C₁₋₄)alkoxycarbonyl(C₁₋₄)alkyl; hydroxy(C₁₋₄)alkyl; mercapto(C₁₋₄)alkyl; (C₁₋₄)alkoxy; hydroxy, amino or aminocarbonyl optionally substituted as for corresponding substituents in R³; (C₁₋₄)alkylsulphonyl; (C₂₋₄)aJkenylsulphonyl; or aminosulphonyl wherein the amino group is optionally mono- or di-substituted by (C₁₋₄)alkyl or (C₂₋₄)alkenyl, provided that R¹⁴ and R¹⁵ on the same carbon atom are not both selected from optionally substituted hydroxy and optionally substituted amino; or
R¹⁴ and R¹⁵ together represent oxo;
R¹³ is hydrogen; trifluoromethyl; (C ₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C ₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl; or
two R¹⁴ groups or an R¹³ and an R¹⁴ group on adjacent atoms together represent a bond and the remaining R¹³, R¹⁴ and R¹⁵ groups are as above defined;
two R¹⁴ groups and two R¹⁵ groups on adjacent atoms together represent bonds such that X² and X³ is triple bonded;
two R¹⁴ groups or an R¹³ and an R¹⁴ group in X² and X³ together with the atoms to which they are attached form a 5 or 6 membered carbocyclic or heterocyclic ring and the remaining R^{15a} groups are as above defined;
X⁴ is phenyl or C or N linked monocyclic aromatic 5- or 6-membered heterocycle containing up to four heteroatoms selected from O, S and N and optionally C-substituted by up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C ₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; aryl, aryl(C₁₋₄)alkyl or aryl(C₁₋₄)alkoxy; and optionally N substituted by trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl(C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C ₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
n is 0 or 1;
A is NR¹¹, O or CR⁶R⁷ and B is NR¹¹, O, SO₂ or CR⁸R⁹ and wherein:
each of R⁶, R⁷, R⁸ and R⁹ is independently selected from: hydrogen; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C ₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or aminosulphonyl wherein the amino group is optionally substituted by (C₁₋₆)alkyl or (C₂₋₆)alkenyl;
or when n=1 R⁶ and R⁸ together represent a bond and R⁷ and R⁹ are as above defined;
or R⁶ and R⁷ or R⁸ and R⁹ together represent oxo;
provided that:
when A is NR¹¹, B is not NR¹¹ or O;
when A is CO, B is not CO, O or SO₂;
when n is 0 and A is NR¹¹, CR⁸R⁹ can only be CO;
when A is CR⁶R⁷ and B is SO₂, n is 0;
when n is 0, B is not NR¹¹ or O or R⁸ and R⁹ are not optionally substituted hydroxy or amino;
when A is O, B is not NR¹¹, O, SO₂ or CO and n=1; and
when A-B is CR⁷=CR⁹, n is 1
R¹⁰ is selected from (C₁₋₄)alkyl; (C₂₋₄)alkenyl and aryl any of which may be optionally substituted by a group R¹² as defined above; carboxy; aminocarbonyl wherein the amino group is optionally substituted by hydroxy, (C₁₋₆)alkyl, (C₂₋₆)alkenyl, (C_{1- 6})alkylsulphonyl, trifluoromethylsulphony), (C₂₋₆)aJkenyJsuJphonyl. (C₁₋₆)alkoxycarbonyl, (C₁₋₆)alkyJcarbonyl, (C₂₋₆)alkenytoxycarbonyl or (C₂₋₆)alkenylcarbonyl and optionally further substituted by (C ₁₋₆)alkyl or (C₂₋₆)alkenyl; (C₁₋₆)alkylsulphonyl; trifluoromethylsulphonyl; (C₂₋₆)alkenylsulphonyl; (C₁₋₆)alkoxycarbonyl; (C₁₋₆)alkylcarbonyl; (C₂₋₆)alkenyloxycarbonyl; and (C₂₋₆)alkenylcarbonyl; and
R¹ is hydrogen; trifluoromethyl, (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C ₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)aikenyl and optionally further substituted by (C₁₋₆)alkyl or (C₂₋₆)alkenyl;
or where one of R³ and R⁶, R⁷, R⁸ or R⁹ contains a carboxy group and the other contains a hydroxy or amino group they may together form a cyclic ester or amide linkage.

2. A compound according to claim 1 wherein Z⁵ is CH or N, Z³ is CH or CF and Z¹, Z² and Z⁴ are each CH, or Z¹ is N, Z³ is CH or CF and Z², Z⁴ and Z⁵ are each CH.

3. A compound according to any preceding claim wherein R¹ is methoxy and R^{1a} is H or when Z³ is CR^{1a} it may be C-F.

4. A compound according to any preceding claim wherein R² is hydrogen, carboxymethyl, hydroxyethyl, aminocarbonylmethyl, ethoxycarbonylmethyl, ethoxycarbonylallyl or carboxyallyl.

5. A compound according to any preceding claim wherein R³ is hydrogen; optionally substituted hydroxy; (C₁₋₄) alkyl; ethenyl; optionally substituted 1-hydroxy-(C₁₋₄) alkyl; optionally substituted aminocarbonyl; carboxy(C₁₋₄)alkyl; optionally substituted aminocarbonyl(C₁₋₄)alkyl; cyano(C₁₋₄)alkyl; optionally substituted 2-oxo-oxazolidinyl and optionally substituted 2-oxo-oxazolidinyl(C₁₋₄alkyl); in the 3- or 4-position.

6. A compound according to any preceding claim wherein n is 0, A-B is CHOH-CH₂, NR¹¹-CH₂ or NR¹¹-CO and R¹¹ is hydrogen or (C₁₋₄)alkyl.

7. A compound according to any preceding claim wherein-X¹-X²-X³- is -(CH₂)₂-0-, -CH₂-CH=CH-, -(CH₂)₃-, -(CH₂)₂-NH- or -CH₂CONH-.

8. A compound according to any preceding claim wherein X⁴ is 2-pyridyl, 3-fluorophenyl, 3,5-difluorophenyl or thiazol-2-yl.

9. A compound according to claim 1 selected from:
2-{1 -[(R)-2-Hydroxy-2-(6-methoxy-[I,S]-naphthyridine-4-yl)-ethyl]-piperidin-4-ylamino)-N-phenyl-acetamide;
4-{*trans*-1-[3,5-Difluorophenyl]-3-propenyl}amino- 1-[2-(R)-hydroxy-2-(6-methoxyquinolin-4-yl)]ethylpiperidine;
(R)-1-(6-Methoxy-[1,5]naphthyridin-4-yl)-2-{4-[2-(pyridin-2-yloxy)-ethylamino]-piperidin-1-yl}-ethanol;
(R)-1-(6-Methoxy-[1,5]naphthyridin-4-yl)-2-[4-((E)-3-pyridin-2-yl-allylamino)-piperidin-1-yt]-ethanol;
4-[2-(3,5-Difluorophenylamino)-ethyl]amino-1-[2-(R)-hydroxy-2-(6-methoxyquinolin-4-yl)]ethylpiperidine;
4-[2-(3-Fluorophenylamino)-ethyl]amino-1-[2-(R)-hydroxy-2-(6-methoxyquinolin-4-yl)]ethylpiperidine; and
4-[*trans*-1-(2-thiazolyl)-3-propenyl]amino-1-[2-(R)-hydroxy-2-(6-methoxy-[1,5]-naphthyridin-4-yl)]ethylpiperidine
or a pharmaceutically acceptable derivative thereof.

10. The use of a compound according to claim 1, in the manufacture of a medicament for use in the treatment of bacterial infections in mammals.

11. A pharmaceutical composition comprising a compound according to claim 1, and a pharmaceutically acceptable carrier.

12. A process for preparing compounds according to claim 1, which process comprises:
reacting a compound of formula (IV) with a compound of formula (V): 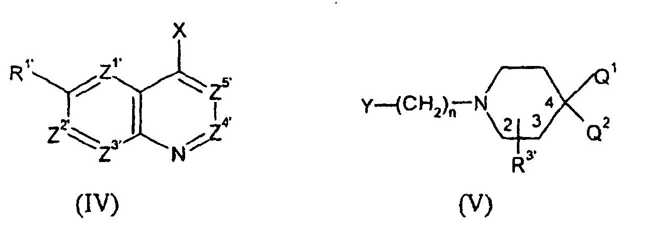
wherein n is as defined in formula (I); Z^{1'}, Z^{2'}, Z^{3'}, Z^{4'}, Z^{5'}, R^{1'} and R^{3'} are Z¹, Z², Z³, Z⁴, Z⁵, R¹ and R³ as defined in formula (I) or groups convertible thereto; Q¹ is NR²R⁴ or a group convertible thereto wherein R^{2'} and R^{4'} are R² and R⁴ as defined
in formula (I) or groups convertible thereto and Q² is H or R^{3'} or Q¹ and Q² together form an optionally protected oxo group;
and X and Y may be the following combinations:
(i) X is A'-COW, Y is H and n is 0;
(ii) X is CR⁶=CR⁸R⁹, Y is H and n is 0;
(iii) X is oxirane, Y is H and n is 0;
(iv) X is N=C=O and Y is H and n is 0;
(v) one of X and Y is CO₂Ry and the other is CH₂CO₂R^{x};
(vi) X is CHR⁶R⁷ and Y is C(=O)R⁸;
(vii) X is CR⁷=PR^{z} ₃ and Y is C(=O)R⁹ and n=1;
(viii) X is C(=O)R⁷ and Y is CR⁹=PR^{z} ₃ and n=1;
(ix) Y is COW and X is NHR^{11'} or NR^{11'}COW and n=0 or 1 or when n=1 X is COW and Y is NHR^{11'} , NCO or NR^{11'}COW;
(x) X is C(=O)R⁶ and Y is NHR^{11'} or X is NHR^{11'} and Y is C(=O)R⁸ and n=1;
(xi) X is NHR^{11'} and Y is CR⁸R⁹W and n=1;
(xii) X is CR⁶R⁷W and Y is NHR^{11'} or OH and n=1; or
(xiii) X is CR⁶R⁷SO₂W and Y is H and n=0;
(xiv) X is W or OH and Y is CH₂OH and n is 1;
(xv) X is NHR^{11'} and Y is SO₂W or X is NR^{11'}SO₂W and Y is H, and n is 0;
(xvi) X is NR^{11'}COCH₂W and Y is H and n=0;
in which W is a leaving group, e.g. halo or imidazolyl; R^{x} and R^{y} are (C₁₋₆)alkyl; R^{z} is aryl or (C₁₋₆)alkyl; A' and NR ^{11'} are A and NR¹¹ as defined in formula (I), or groups convertible thereto; and oxirane is: 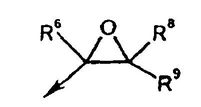 wherein R⁶, R⁸ and R⁹ are as defined in formula (I);
and thereafter optionally or as necessary converting Q ¹ and Q² to NR^{2'}R^{4'}; converting A', Z^{1'}, Z^{2'}, Z^{3'}, Z^{4'}, Z^{5'}, R^{1'}, R^{2'}, R^{3'}, R^{4'} and NR^{11'} to A, Z¹, Z², Z³, Z⁴, Z⁵, R¹, R², R³, R⁴ and NR¹¹; converting A-B to other A-B, interconverting R¹, R², R³ and/or R⁴, and/or forming a pharmaceutically acceptable derivative thereof.

13. A compound of formula (VI): 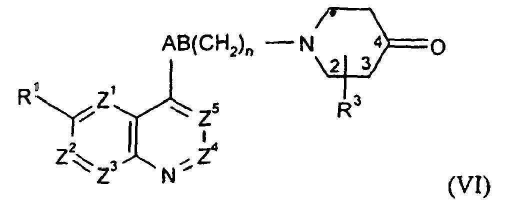 wherein the variables are as described for formula (I) in claim 1.

## Patentansprüche

1. Verbindung der Formel (I) oder ein pharmazeutisch verträgliches Derivat davon: 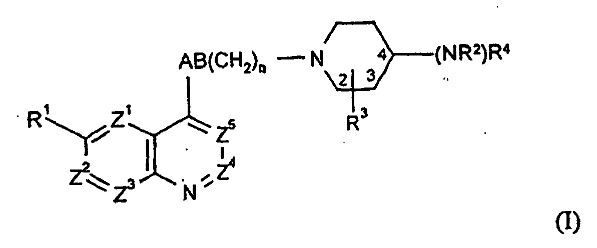 wobei
eines von Z¹, Z², Z³, Z⁴ und Z⁵ die Bedeutung N hat, eines CR^{1a} ist und die übrigen CH sind, oder ein oder zwei von Z¹, Z², Z³, Z⁴ und Z⁵ unabhängig voneinander CR^{1a} sind und die übrigen CH sind;
die Reste R¹ und R^{1a} unabhängig voneinander Wasserstoff; Hydroxy; (C₁₋₆)Alkoxy, welches gegebenenfalls mit (C₁₋₆)Alkoxy, Amino, Piperidyl, Guanidino oder Amidino substituiert ist, die jeweils gegebenenfalls mit einem oder zwei (C₁₋₆)Alkyl, Acyl oder (C₁₋₆)Alkylsulfonylresten, CONH₂, Hydroxy, (C₁₋₆)Alkylthio, Heterocyclylthio, Heterocyclyloxy, Arylthio, Aryloxy, Acylthio, Acyloxy oder (C₁₋₆)Alkylsulfonyloxy N-substituiert sind; (C₁₋₆)Alkoxy-substituiertes (C₁₋₆)Alkyl; Halogen; (C₁₋₆)Alkyl; (C₁₋₆)Alkylthio; Trifluormethyl; Trifluormethoxy; Nitro; Azido; Acyl; Acyloxy; Acylthio; (C₁₋₆)Alkylsulfonyl; (C₁₋₆)Alkylsulfoxid; Arylsulfonyl; Arylsulfoxid oder ein Amino, Piperidyl, Guanidino oder eine Amidinogruppe sind, welche gegebenenfalls mit einem oder zwei (C₁₋₆)Alkyl-, Acyl- oder (C₁₋₆)Alkylsulfonylresten N-substituiert ist, sind;
oder wenn Z⁵ die Bedeutung CR^{1a} hat, kann der Rest R^{1a} stattdessen Cyano, Hydroxymethyl oder Carboxy sein;
mit der Maßgabe, dass wenn Z¹, Z², Z³, Z⁴ und Z⁵ die Bedeutung CR^{1a} oder CH haben, dann ist der Rest R¹ nicht Wasserstoff;
der Rest R² Wasserstoff oder (C₁₋₄)Alkyl oder (C₂₋₄)Alkenyl ist, welche gegebenenfalls mit 1 bis 3 Resten substituiert sind, welche ausgewählt sind aus:
Amino, welches gegebenenfalls mit einem oder zwei (C₁₋₄)Alkylresten substituiert ist; Carboxy; (C₁₋₄)Alkoxycarbonyl; (C₁₋₄)Alkylcarbonyl; (C₂₋₄)Alkenyloxycarbonyl; (C₂₋₄)Alkenylcarbonyl; Aminocarbonyl, wobei die Aminogruppe gegebenenfalls mit Hydroxy, (C₁₋₄)Alkyl, Hydroxy(C₁₋₄)alkyl, Aminocarbonyl(C₁₋₄)alkyl, (C₂₋₄)Alkenyl, (C₁₋₄)Alkylsulfonyl, Trifluormethylsulfonyl, (C₂₋₄)Alkenylsulfonyl, (C₁₋₄)Alkoxycarbonyl, (C₁₋₄)Alkylcarbonyl, (C₂₋₄)Alkenyloxycarbonyl oder (C₂₋₄)Alkenylcarbonyl substituiert ist; Cyano; Tetrazolyl; 2-Oxo-oxazolidinyl, welches gegebenenfalls mit dem Rest R¹⁰ substituiert ist; 3-Hydroxy-3-cyclobuten-1,2-dion-4-yl; 2,4-Thiazolidindion-5-yl; Tetrazol-5-ylaminocarbonyl; 1,2,4-Triazol-5-yl, welches gegebenenfalls mit dem Rest R¹⁰ substituiert ist; 5-Oxo-1,2,4-oxadiazol-3-yl; Halogen; (C₁₋₄)Alkylthio; Trifluormethyl, Hydroxy, welches gegebenenfalls mit (C₁₋₄)Alkyl, (C₂₋₄)Alkenyl, (C₁₋₄)Alkoxycarbonyl, (C₁₋₄)Alkylcarbonyl, (C₂₋₄)Alkenyloxycarbonyl, (C₂₋₄)Alkenylcarbonyl substituiert ist; Oxo; (C₁₋₄)Alkylsulfonyl; (C₂₋₄)Alkenylsulfonyl; oder (C₁₋₄)Aminosulfonyl, wobei die Aminogruppe gegebenenfalls mit (C₁₋₄)Alkyl oder (C₂₋₄)Alkenyl substituiert ist;
der Rest R³ Wasserstoff ist; oder
der Rest R³ in der 2-, 3- oder 4-Position ist und die Bedeutung hat:
Carboxy; (C₁₋₆)Alkoxycarbonyl; Aminocarbonyl, wobei die Aminogruppe gegebenenfalls mit Hydroxy, (C₁₋₆)Alkyl, Hydroxy(C₁₋₆)alkyl, Aminocarbonyl(C₁₋₆)alkyl, (C₂₋₆)Alkenyl, (C₁₋₆)Alkylsulfonyl, Trifluormethylsulfonyl, (C₂₋₆)Alkenylsulfonyl, (C₁₋₆)Alkoxycarbonyl, (C₁₋₆)Alkylcarbonyl, (C₂₋₆)Alkenyloxycarbonyl oder (C₂₋₆)Alkenylcarbonyl substituiert ist und gegebenenfalls ferner mit (C₁₋₆)Alkyl, Hydroxy(C₁₋₆)alkyl, Aminocarbonyl(C₁₋₆)alkyl oder (C₂₋₆)Alkenyl substituiert ist; Cyano; Tetrazolyl; 2-Oxo-oxazolidinyl, welches gegebenenfalls mit dem Rest R¹⁰ substituiert ist; 3-Hydroxy-3-cyclobuten-1,2-dion-4-yl; 2,4-Thiazolidindion-5-yl; Tetrazol-5-ylaminocarbonyl; 1,2,4-Triazol-5-yl, welches gegebenenfalls mit dem Rest R¹⁰ substituiert ist; oder 5-Oxo-1,2,4-oxadiazol-3-yl; oder
(C₁₋₄)Alkyl oder Ethenyl, die gegebenenfalls mit einem der Substituenten, die oben für den Rest R³ aufgelistet sind, und/oder mit 0 bis 2 Resten R¹² substituiert sind, die unabhängig voneinander ausgewählt sind aus:
Halogen; (C₁₋₆)Alkylthio; Trifluormethyl; (C₁₋₆)Alkoxycarbonyl; (C₁₋₆)Alkylcarbonyl; (C₂₋₆)Alkenyloxycarbonyl; (C₂₋₆)Alkenylcarbonyl; Hydroxy, welches gegebenenfalls mit (C₁₋₆)Alkyl, (C₂₋₆)Alkenyl, (C₁₋₆)Alkoxycarbonyl, (C₁₋₆)Alkylcarbonyl, (C₂₋₆)Alkenyloxycarbonyl, (C₂₋₆)Alkenylcarbonyl oder Aminocarbonyl substituiert ist, wobei die Aminogruppe gegebenenfalls mit (C₁₋₆)Alkyl, (C₂₋₆)Alkenyl, (C₁₋₆)Alkylcarbonyl oder (C₂₋₆)Alkenylcarbonyl substituiert ist; Amino, welches gegebenenfalls mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl, (C₂₋₆)Alkenyl, (C₁₋₆)Alkylsulfonyl, (C₂₋₆)Alkenylsulfonyl oder Aminocarbonyl mono- oder disubstituiert ist, wobei die Aminogruppe gegebenenfalls mit (C₁₋₆)Alkyl oder (C₂₋₆)Alkenyl substituiert ist; Aminocarbonyl, wobei die Aminogruppe gegebenenfalls mit (C₁₋₆)Alkyl, Hydroxy(C₁₋₆)alkyl, Aminocarbonyl(C₁₋₆)alkyl, (C₂₋₆)Alkenyl, (C₁₋₆)Alkoxycarbonyl, (C₁₋₆)Alkylcarbonyl, (C₂₋₆)Alkenyloxycarbonyl oder (C₂₋₆)Alkenylcarbonyl substituiert ist und gegebenenfalls ferner mit (C₁₋₆)Alkyl, Hydroxy(C₁₋₆)alkyl, Aminocarbonyl(C₁₋₆)alkyl oder (C₂₋₆)Alkenyl substituiert ist; Oxo; (C₁₋₆)Alkylsulfonyl; (C₂₋₆)Alkenylsulfanyl; oder (C₁₋₆)Aminosulfonyl, wobei die Aminogruppe gegebenenfalls mit (C₁₋₆)Alkyl oder (C₂₋₆)Alkenyl substituiert ist; oder wenn der Rest R³ in der 3-Position ist, ist Hydroxy wie oben beschrieben gegebenenfalls substituiert; zusätzlich, wenn der Rest R³ mit einem Hydroxy oder einem eine Aminogruppeenthaltenden Substituenten und einem eine Carboxygruppe-enthaltenden Substituenten disubstituiert ist, können diese zusammen jeweils eine cyclische Ester- oder Amidbindung bilden;
R⁴ ein Rest -X¹-X²-X³-X⁴ ist, wobei:
X¹ die Bedeutung CH₂, CO oder SO₂ hat;
X² die Bedeutung CR¹⁴R¹⁵ hat;
X³ die Bedeutung NR¹³, O, S, SO₂ oder CR¹⁴R¹⁵ hat; wobei:
jeweils die Reste R¹⁴ und R¹⁵ unabhängig voneinander ausgewählt sind aus: Wasserstoff; Halogen; (C₁₋₄)Alkoxy; (C₁₋₄)Alkylthio; Trifluormethyl; Cyano; Carboxy; Nitro; (C₁₋₄)Alkyl; (C₂₋₄)Alkenyl; (C₁₋₄)Alkoxycarbonyl; Formyl; (C₁₋₄)Alkylcarbonyl; (C₂₋₄)Alkenyloxycarbonyl; (C₂₋₄)Alkenylcarbonyl; Carboxy(C₁₋₄)alkyl; Halogen(C₁₋₄)alkoxy; Halogen(C₁₋₄)alkyl; (C₁₋₄)Alkylcarbonyloxy; (C₁₋₄)Alkoxycarbonyl(C₁₋₄)alkyl; Hydroxy(C₁₋₄)alkyl; Mercapto(C₁₋₄)alkyl; (C₁₋₄)Alkoxy; Hydroxy, Amino oder Aminocarbonyl, die gegebenenfalls mit den entsprechenden Substituenten in dem Rest R³ substituiert sind; (C₁₋₄)Alkylsulfonyl; (C₂₋₄)Alkenylsulfonyl; oder Aminosulfonyl, wobei die Aminogruppe gegebenenfalls mit (C₁₋₄)Alkyl oder (C₂₋₄)Alkenyl mono- oder disubstituiert ist, mit der Maßgabe, dass die Reste R¹⁴ und R¹⁵ an dem selben Kohlenstoffatom nicht beide aus gegebenenfalls substituiertem Hydroxy und gegebenenfalls substituiertem Amino ausgewählt sind; oder
die Reste R¹⁴ und R¹⁵ zusammen Oxo sind;
der Rest R¹³ Wasserstoff; Trifluormethyl; (C₁₋₆)Alkyl; (C₂₋₆)Alkenyl; (C₁₋₆)Alkoxycarbonyl; (C₁₋₆)Alkylcarbonyl; oder Aminocarbonyl ist, wobei die Aminogruppe gegebenenfalls mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl oder (C₂₋₆)Alkenyl substituiert ist und gegebenenfalls ferner mit (C₁₋₆)Alkyl oder (C₂₋₆)Alkenyl substituiert ist; oder
zwei R¹⁴-Reste oder ein R¹³- und ein R¹⁴-Rest an benachbarten Atomen zusammen eine Bindung bedeuten und die übrigen R¹³-, R¹⁴- und R¹⁵-Reste wie oben definiert sind;
zwei R¹⁴-Reste und zwei R¹⁵-Reste an benachbarten Atomen zusammen Bindungen bedeuten, so dass X² und X³ eine Dreifachbindung ist;
zwei R¹⁴-Reste oder ein R¹³- und ein R¹⁴-Rest in X² und X³ zusammen mit den Atomen, an denen sie gebunden sind, einen 5- oder 6-gliedrigen carbocyclischen oder heterocyclischen Ring bilden und die übrigen R^{15a}-Reste wie oben definiert sind;
X⁴ Phenyl oder ein an C- oder N-gebundener monocyclischer aromatischer 5- oder 6- gliedriger Heterocyclus ist, welcher bis zu vier Heteroatome, ausgewählt aus O, S und N, enthält und der gegebenenfalls mit bis zu drei Resten, ausgewählt aus (C₁₋₄)Alkylthio; Halogen; Carboxy(C₁₋₄)alkyl; Halogen(C₁₋₄)alkoxy; Halogen(C₁₋₄)alkyl; (C₁₋₄)Alkyl; (C₂₋₄)Alkenyl; (C₁₋₄)Alkcoxycarbonyl; Formyl; (C₁₋₄)Alkylcarbonyl; (C₂₋₄)Alkenyloxycarbonyl; (C₂₋₄)Alkenylcarbonyl; (C₁₋₄)Alkylcarbonyloxy; (C₁₋₄)Alkoxycarbonyl(C₁₋₄)alkyl; Hydroxy; Hydroxy(C₁₋₄)alkyl; Mercapto(C₁₋₄)alkyl; (C₁₋₄)Alkoxy; Nitro; Cyano; Carboxy; Amino oder Aminocarbonyl, die gegebenenfalls mit den entsprechenden Substituenten in dem Rest R³ substituiert sind; (C₁₋₄)Alkylsulfonyl; (C₂₋₄)Alkenylsulfonyl; oder Aminosulfonyl, wobei die Aminogruppe gegebenenfalls mit (C₁₋₄)Alkyl oder (C₂₋₄)Alkenyl substituiert ist; Aryl, Aryl(C₁₋₄)alkyl oder Aryl(C₁₋₄)alkoxy; und gegebenenfalls N, mit Trifluormethyl substituiert; (C₁₋₄)Alkyl, das gegebenenfalls mit Hydroxy, (C₁₋₆)Alkoxy, (C₁₋₆)Alkylthio, Halogen oder Trifluormethyl substituiert ist; (C₂₋₄)Alkenyl; Aryl; Aryl(C₁₋₄)alkyl; (C₁₋₄)Alkoxycarbonyl; (C₁₋₄)Alkylcarbonyl; Formyl; (C₁₋₆)Alkylsulfonyl; oder Aminocarbonyl, wobei die Aminogruppe gegebenenfalls mit (C₁₋₄)Alkoxycarbonyl, (C₁₋₄)A1kylcarbonyl, (C₂₋₄)Alkenyloxycarbonyl, (C₂₋₄)Alkenylcarbonyl, (C₁₋₄)AlkyI oder (C₂₋₄)Alkenyl substituiert ist und gegebenenfalls ferner mit (C₁₋₄)Alky1 oder (C₂₋₄)Alkenyl substituiert ist, C-substituiert ist;
n gleich 0 oder 1 ist;
A die Bedeutung NR¹¹, O oder CR ⁶R⁷ hat und B die Bedeutung NR¹¹, O, SO₂ oder CR⁸R⁹ hat und wobei:
jeweils die Reste R⁶, R⁷, R⁸ und R⁹ unabhängig voneinander ausgewählt sind aus: Wasserstoff, (C₁₋₆)Alkoxy; (C₁₋₆)Alkylthio; Halogen; Trifluormethyl; Azido; (C₁₋₆)Alkyl; (C₂₋₆)Alkenyl; (C₁₋₆)Akoxycarbonyl; (C₁₋₆)Alkylcarbonyl; (C₂₋₆)Alkenyloxycarbonyl; (C₂₋₆)Alkenylcarbonyl; Hydroxy, Amino oder Aminocarbonyl, die gegebenenfalls mit den entsprechenden Substituenten in dem Rest R³ substituiert sind; (C₁₋₆)Alkylsulfonyl; (C₂₋₆)Alkenylsulfonyl; oder Aminosulfonyl, wobei die Aminogruppe gegebenenfalls mit (C₁₋₆)Alkyl oder (C₂₋₆)Alkenyl substituiert ist;
oder wenn n=1, bedeuten die Reste R⁶ und R⁸ zusammen eine Bindung und die Reste R⁷ und R⁹ sind wie oben definiert, oder die Reste R⁶ und R⁷ oder die Reste R⁸ und R⁹ bedeuten zusammen Oxo;
mit der Maßgabe, dass :
wenn A die Bedeutung NR¹¹ hat, ist B nicht NR¹¹ oder O;
wenn A die Bedeutung CO hat, ist B nicht CO, O oder SO₂;
wenn n gleich 0 ist und A die Bedeutung NR¹¹ hat, kann CR⁸R⁹ nur CO sein;
wenn A die Bedeutung CR⁶R⁷ hat und B die Bedeutung SO₂ hat, ist n gleich 0;
wenn n gleich 0 ist, hat B nicht die Bedeutung NR¹¹ oder O oder die Reste R⁸ und R⁹ sind nicht gegebenenfalls substituiertes Hydroxy oder Amino;
wenn A die Bedeutung O hat, ist B nicht NR¹¹, O, SO₂ oder CO und n=1; und
wenn A-B die Bedeutung CR⁷=CR⁹ hat, ist n gleich 1
der Rest R¹⁰ ausgewählt ist aus (C₁₋₄)Alkyl; (C₂₋₄)Alkenyl und Aryl, die jeweils gegebenenfalls mit einem Rest R¹², wie oben definiert, substituiert sein können; Carboxy; Aminocarbonyl, wobei die Aminogruppe gegebenenfalls mit Hydroxy, (C₁₋₆)Allcyl, (C₂₋₆)Alkenyl, (C₁₋₆)Alkylsulfonyl, Trifluormethylsulfonyl, (C₂₋₆)Alkenylsulfonyl, (C₁₋₆)Alkoxycarbonyl, (C₁₋₆)Alkylearbonyl, (C₂₋₆)Alkenyloxycarbonyl oder (C₂₋₆)Alkenylcarbonyl substituiert ist und gegebenenfalls ferner mit (C₁₋₆)Alkyl oder (C₂₋₆)Alkenyl substituiert ist; (C₁₋₆)Alkylsulfonyl; Trifluormethylsulfonyl; (C₂₋₆)Alkenylsulfonyl; (C₁₋₆)Alkoxycarbonyl; (C₁₋₆)Alkylcarbonyl; (C₂₋₆)Alkenyloxycarbonyl; und (C₂₋₆)Alkenylcarbonyl; und
der Rest R¹¹ Wasserstoff; Trifluormethyl; (C₁₋₆)Alkyl; (C₂₋₆)Alkenyl; (C₁₋₆)Alkoxycarbonyl; (C₁₋₆)A1kylcarbonyl; oder Aminocarbonyl ist, wobei die Aminogruppe gegebenenfalls mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl oder (C₂₋₆)Alkenyl substituiert ist und gegebenenfalls ferner mit (C₁₋₆)Alkyl oder (C₂₋₆)Alkenyl substituiert ist;
oder wenn einer der Reste R³ und R⁶, R⁷, R⁸ oder R⁹ eine Carboxygruppe und der andere eine Hydroxy- oder Aminogruppe enthält, können sie zusammen eine cyclische Ester- oder Amidbindung bilden.

2. Verbindung gemäß Anspruch 1, wobei Z⁵ die Bedeutung CH oder N hat, Z³ die Bedeutung CH oder CF hat und Z¹, Z² und Z⁴ jeweils CH sind, oder Z¹ die Bedeutung N hat, Z³ die Bedeutung CH oder CF hat und Z², Z⁴ und Z⁵ jeweils CH sind.

3. Verbindung gemäß einem der vorhergehenden Ansprüche, wobei der Rest R¹ die Bedeutung Methoxy hat und der Rest R^{1a} die Bedeutung H hat oder wenn Z³ die Bedeutung CR^{1a} hat, kann es C-F sein.

4. Verbindung gemäß einem der vorhergehenden Ansprüche, wobei der Rest R² Wasserstoff, Carboxymethyl, Hydroxyethyl, Aminocarbonylmethyl, Ethoxycarbonylmethyl, Ethoxycarbonylallyl oder Carboxyallyl ist.

5. Verbindung gemäß einem der vorhergehenden Ansprüche, wobei der Rest R³ Wasserstoff; gegebenenfalls substituiertes Hydroxy; (C₁₋₄)Alkyl; Ethenyl; gegebenenfalls substituiertes 1-Hydroxy(C₁₋₄)alkyl; gegebenenfalls substituiertes Aminocarbonyl; Carboxy(C₁₋₄)alkyl; gegebenenfalls substituiertes Aminocarbonyl(C₁₋₄)alkyl; Cyano(C₁₋₄)alkyl; gegebenenfalls substituiertes 2-Oxo-oxazolidinyl und gegebenenfalls substituiertes 2-Oxo-oxazolidinyl(C₁₋₄-alkyl); in der 3- oder 4-Position ist.

6. Verbindung gemäß einem der vorhergehenden Ansprüche, wobei n gleich 0 ist, A-B die Bedeutung CHOH-CH₂, NR¹¹-CH₂ oder NR¹¹-CO hat und der Rest R¹¹ Wasserstoff oder (C₁₋₄)Alkyl ist.

7. Verbindung gemäß einem der vorhergehenden Ansprüche, wobei -X¹-X²-X³- die Bedeutung -(CH₂)₂-O-, -CH₂-CH=CH-, -(CH₂)₃-, -(CH₂)₂-NH- oder -CH₂CONH- hat.

8. Verbindung gemäß einem der vorhergehenden Ansprüche, wobei X⁴ die Bedeutung 2-Pyridyl, 3-Fluorphenyl, 3,5-Difluorphenyl oder Thiazol-2-yl hat.

9. Verbindung gemäß Anspruch 1, ausgewählt aus:
2-{1-[(R)-2-Hydroxy-2-(6-methoxy-[1,5]-naphthyridin-4-yl)ethyl]-piperidin-4-ylamino}-N-phenyl-acetamid;
4-{*trans*-1-[3,5-Difluorphenyl]-3-propenyl}amino-1-[2-(R)-hydroxy-2-(6-methoxychinolin-4-yl)]ethylpiperidin;
(R)-1-(6-Methoxy-[1,5]-naphthyridin-4-yl)-2-{4-[2-(pyridin-2-yloxy)ethylamino]-piperidin-1-yl}ethanol;
(R)-1-(6-Methoxy-[1,5]-naphthyridin-4-yl)-2-[4-((E)-3-pyridin-2-yl-allylamino)piperidin-1-yl]ethanol;
4-[2-(3,5-Difluorphenylamino)ethyl]amino-1-[2-(R)-hydroxy-2-(6-methoxychinolin-4-yl)]ethylpiperidin;
4-[2-(3-Fluorphenylamino)ethyl]amino-1-[2-(R)-hydroxy-2-(6-methoxychinolin-4-yl)]ethylpiperidin; und
4-[*trans*-1-(2-Thiazolyl)-3-propenyl]amino-1-[2-(R)-hydroxy-2-(6-methoxy-[1,5]-naphthyridin-4-yl)]ethylpiperidin
oder ein pharmazeutisch verträgliches Derivat davon.

10. Verwendung einer Verbindung gemäß Anspruch 1 zur Herstellung eines Medikaments zur Verwendung in der Behandlung von bakteriellen Infektionen in Säugern.

11. Arzneimittel, umfassend eine Verbindung gemäß Anspruch 1 und einen pharmazeutisch verträglichen Träger.

12. Verfahren zur Herstellung von Verbindungen gemäß Anspruch 1, wobei das Verfahren umfasst:
Umsetzen einer Verbindung der Formel (IV) mit einer Verbindung der Formel (V): 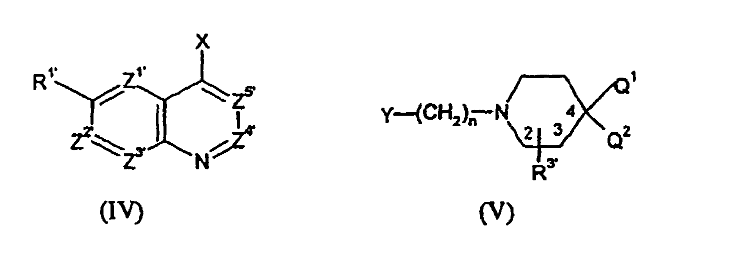
wobei n wie in Formel (I) definiert ist; Z^{1'}, Z^{2'}, Z^{3'}, Z^{4'}, Z^{5'}, R^{1'} und R^{3'} die Bedeutung von Z¹, Z², Z³, Z⁴, Z⁵, R¹ und R³ wie in Formel (I) definiert oder von dazu umwandelbaren Resten haben;
Q¹ die Bedeutung NR²R⁴ oder von einem dazu umwandelbaren Rest hat, wobei die Reste R^{2'} und R^{4'} die Bedeutung der Reste R² und R⁴ wie in Formel (I) definiert oder von dazu umwandelbaren Resten haben, und Q² die Bedeutung H oder R^{3'} hat oder Q¹ und Q² zusammen eine gegebenenfalls geschützte Oxogruppe bilden;
und X und Y die folgenden Kombinationen sein können:
(i) X ist A'-COW, Y ist H und n ist 0;
(ii) X ist CR⁶=CR⁸R⁹, Y ist H und n ist 0;
(iii) X ist Oxiran, Y ist H und n ist 0;
(iv) X ist N=C=O und Y ist H und n ist 0;
(v) einer von X und Y ist CO₂R^{y} und der andere ist CH₂CO₂R^{x};
(vi) X ist CHR⁶R⁷ und Y ist C(=O)R⁸;
(vü) X ist CR⁷=PR^{z} ₃ und Y ist C(=O)R⁹ und n=1;
(viii) X ist C(=O)R⁷ und Y ist CR⁹=PR^{z} ₃ und n=1;
(ix) Y ist COW und X ist NHR^{11'} oder NR^{11'}COW und n=0 oder 1 oder wenn n=1, hat X die Bedeutung COW und Y die Bedeutung NHR^{11'}, NCO oder NR^{11'}COW;
(x) X ist C(=O)R⁶ und Y ist NHR^{11'} oder X ist NHR^{11'} und Y ist C(=O)R⁸ und n=1;
(xi) X ist NHR^{11'} und Y ist CR⁸R⁹W und n=1;
(xii) X ist CR⁶R⁷W und Y ist NHR^{11'} oder OH und n=1; oder
(xiii) X ist CR⁶R⁷SO₂W und Y ist H und n=0;
(xiv) X ist W oder OH und Y ist CH₂OH und n ist 1;
(xv) X ist NHR^{11'} und Y ist SO₂W oder X ist NR^{11'}SO₂W und Y ist H, und n ist 0;
(xvi) X ist NR^{11'}COCH₂W und Y ist H und n=0;
wobei W eine Abgangsgruppe ist, z.B. Halogen oder Imidazolyl; die Reste R^{x} und R^{y} die Bedeutung (C₁₋₆)Alkyl haben; der Rest R^{z} die Bedeutung Aryl oder (C₁₋₆)Alkyl hat; A' und NR^{11'} die Bedeutung von A und NR¹¹ wie in Formel (I) definiert oder von dazu umwandelbaren Reste haben; und ein Oxiran der Formel: 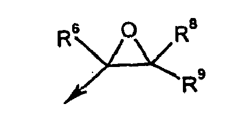 wobei die Reste R⁶, R⁸ und R⁹ wie in Formel (I) definiert sind;
und danach gegebenenfalls oder falls notwendig Umwandeln von Q¹ und Q² in NR^{2'}R^{4'}; Umwandeln von A', Z^{1'}, Z^{2'}, Z^{3'}, Z^{4'}, Z^{5'}, R^{1'}, R^{2'}, R^{3'}, R^{4'} und NR^{11'} in A, Z¹, Z², Z³, Z⁴, Z⁵, R¹, R², R³, R⁴ und NR¹¹; Umwandeln von A-B in ein anderes A-B, gegenseitiges Umwandeln von R¹, R², R³ und/oder R⁴, und/oder Bilden eines pharmazeutisch verträglichen Derivats davon.

13. Verbindung der Formel (VI): 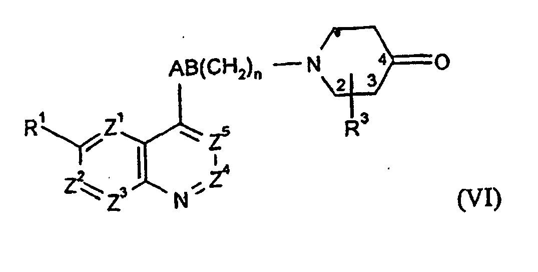 wobei die Variablen die Bedeutung wie für die Formel (I), in Anspruch 1 beschrieben, haben.

## Revendications

1. Composé de formule (I) ou un de ses dérivés pharmaceutiquement acceptables : 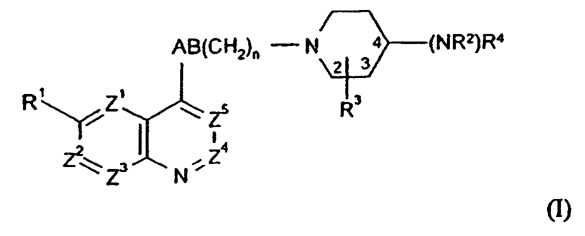 dans laquelle :
un des groupes Z¹, Z², Z³, Z⁴ et Z⁵ représente un atome de N, l'un représente un groupe CR^{1a} et les groupes restants représentent des groupes CH, ou bien un ou deux des groupes Z¹, Z², Z³, Z⁴ et Z⁵ représentent indépendamment des groupes CR^{1a} et le reste représente des groupes CH ;
R¹ et R^{1a} représentent indépendamment un atome d'hydrogène ; un groupe hydroxy ; alkoxy en C₁ à C₆ facultativement substitué avec un substituant alkoxy en C₁ à C₆, amino, pipéridyle, guanidino ou amidino, dont n'importe lequel est facultativement N-substitué avec un ou deux groupes alkyle en C₁ à C₆, acyle ou alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkyle en C₁ à C₆ à substituant alkoxy en C₁ à C₆ ; halogéno ; alkyle en C₁ à C₆ ; alkylthio en C₁ à C₆ ; trifluorométhyle ; trifluorométhoxy ; nitro ; azido ; acyle ; acyloxy ; acylthio ; alkylsulfonyle en C₁ à C₆ ; alkylsulfoxyde en C₁ à C₆ ; arylsulfonyle ; arylsulfoxyde ou un groupe amino, pipéridyle, guanidino ou amidino facultativement N-substitué avec un ou deux groupes alkyle en C₁ à C₆ , acyle ou alkylsulfonyle en C₁ à C₆ ;
ou bien, lorsque Z⁵ représente un groupe CR^{1a}, R^{1a} peut en variante représenter un groupe cyano, hydroxyméthyle ou carboxy ;
sous réserve que, lorsque Z¹, Z², Z³, Z⁴ et Z⁵ représentent des groupes CR^{1a} ou CH, alors R¹ ne représente pas un atome d'hydrogène ;
R² représente un atome d'hydrogène ou un groupe alkyle en C₁ à C₄ ou alcényle en C₂ à C₄ facultativement substitué avec 1 à 3 groupes choisis entre des groupes :
amino facultativement substitué avec un ou deux groupes alkyle en C₁ à C₄ ; carboxy ; (alkoxy en C₁ à C₄)carbonyle ; (alkyle en C₁ à C₄)carbonyle ; (alcényle en C₂ à C₄)oxycarbonyle ; (alcényle en C₂ à C₄)carbonyle ; aminocarbonyle dans lequel le groupe amino est facultativement substitué avec un substituant hydroxy, alkyle en C₁ à C₄, hydroxyalkyle en C₁ à C₄, aminocarbonyl (alkyle en C₁ à C₄), alcényle en C₂ à C₄, alkylsulfonyle en C₁ à C₄, trifluorométhylsulfonyle, alcénylsulfonyle en C₂ à C₄, (alkoxy en C₁ à C₄) carbonyle, (alkyle en C₁ à C₄) carbonyle, (alcényle en C₂ à C₄)oxycarbonyle ou (alcényle en C₂ à C₄)carbonyle ; cyano ; tétrazolyle ; 2-oxo-oxazolidinyle facultativement substitué avec un substituant R¹⁰ ; 3-hydroxy-3-cyclobutène-1,2-dione-4-yle ; 2,4-thiazolidinedione-5-yle ; tétrazole-5-ylaminocarbonyle ; 1,2,4-triazole-5-yle facultativement substitué avec un substituant R¹⁰ ; 5-oxo-1,2,4-oxadiazole-3-yle ; halogéno ; alkylthio en C₁ à C₄ ; trifluorométhyle ; hydroxy facultativement substitué avec un substituant alkyle en C₁ à C₄, alcényle en C₂ à C₄, (alkoxy en C₁ à C₄)carbonyle, (alkyle en C₁ à C₄) carbonyle, (alcényle en C₂ à C₄) oxycarbonyle, (alcényle en C₂ à C₄)carbonyle ; oxo ; alkylsulfonyle en C₁ à C₄ ; alcénylsulfonyle en C₂ à C₄ ; ou aminosulfonyle en C₁ à C₄
dans lequel le groupe amino est facultativement substitué avec un substituant alkyle en C₁ à C₄ ou alcényle en C₂ à C₄.
R³ représente un atome d'hydrogène ; ou
R³ est en position 2, 3 ou 4 et représente un groupe :
carboxy ; (alkoxy en C₁ à C₆)carbonyle ; aminocarbonyle dans lequel le groupe amino est facultativement substitué avec un substituant hydroxy, alkyle en C₁ à C₆, hydroxyalkyle en C₁ à C₆, aminocarbonyl (alkyle en C₁ à C₆), alcényle en C₂ à C₆, alkylsulfonyle en C₁ à C₆, trifluorométhylsulfonyle, alcénylsulfonyle en C₂ à C₆, (alkoxy en C₁ à C₆) carbonyle, (alkyle en C₁ à C₆) carbonyle, (alcényle en C₂ à C₆)oxycarbonyle ou (alcényle en C₂ à C₆)carbonyle et est en outre facultativement substitué avec un substituant alkyle en C₁ à C₆ , hydroxyalkyle en C₁ à C₆, aminocarbonyl (alkyle en C₁ à C₆) ou alcényle en C₂ à C₆ ; cyano ; tétrazolyle ; 2-oxo-oxazolidinyle facultativement substitué avec un substituant R¹⁰ ; 3-hydroxy-3-cyclobutène-1,2-dione-4-yle ; 2,4-thiazolidinedione-5-yle ; tétrazole-5-ylaminocarbonyle ; 1,2,4-triazole-5-yle facultativement substitué avec un substituant R¹⁰ ; ou 5-oxo-1,2,4-oxadiazole-3-yle ; ou
alkyle en C₁ à C₄ ou éthényle facultativement substitué avec n'importe lesquels des substituants énumérés ci-dessus pour R³ et/ou 0 à 2 groupes R¹² choisis indépendamment entre des groupes :
halogéno ; alkylthio en C₁ à C₆ ; trifluorométhyle ; (alkoxy en C₁ à C₆) carbonyle ; (alkyle en C₁ à C₆) carbonyle ; (alcényle en C₂ à C₆) oxycarbonyle ; (alcényle en C₂ à C₆)carbonyle ; hydroxy facultativement substitué avec un substituant alkyle en C₁ à C₆, alcényle en C₂ à C₆, (alkoxy en C₁ à C₆) carbonyle, (alkyle en C₁ à C₆) carbonyle, (alcényle en C₂ à C₆) oxycarbonyle, (alcényle en C₂ à C₆)carbonyle ou aminocarbonyle dans lequel le groupe amino est facultativement substitué avec un substituant alkyle en C₁ à C₆, alcényle en C₂ à C₆, (alkyle en C₁ à C₆) carbonyle ou (alcényle en C₂ à C₆) carbonyle ; amino facultativement mono- ou disubstitué avec un substituant (alkoxy en C₁ à C₆) carbonyle, (alkyle en C₁ à C₆) carbonyle, (alcényle en C₂ à C₆)oxycarbonyle, (alcényle en C₂ à C₆)carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ; aminocarbonyle dans lequel le groupe amino est facultativement substitué avec un substituant alkyle en C₁ à C₆, hydroxyalkyle en C₁ à C₆, aminocarbonyl (alkyle en C₁ à C₆), alcényle en C₂ à C₆, (alkoxy en C₁ à C₆)carbonyle, (alkyle en C₁ à C₆)carbonyle, (alcényle en C₂ à C₆) oxycarbonyle ou (alcényle en C₂ à C₆) carbonyle et facultativement substitué en outre avec un substituant alkyle en C₁ à C₆, hydroxyalkyle en C₁ à C₆, aminocarbonyl(alkyle en C₁ à C₆) ou alcényle en C₂ à C₆ ; oxo ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ; ou, lorsque R³ est en position 3, un groupe hydroxy facultativement substitué de la manière décrite ci-dessus ;
en outre, lorsque R³ est disubstitué avec un substituant à fonction hydroxy ou amino et un substituant à fonction carboxy, ces substituants peuvent, conjointement, former une liaison ester ou amide cyclique, respectivement
R⁴ représente un groupe -X¹-X²-X³-X⁴ dans lequel :
X¹ représente un groupe CH₂, CO ou SO₂ ;
X² représente un groupe CR¹⁴R¹⁵
X³ représente un groupe NR¹³, O, S, SO₂ ou CR¹⁴R¹⁵ ;
dans lesquels :
chacun des groupes R¹⁴ et R¹⁵ est choisi indépendamment entre : un atome d'hydrogène ; des groupes halogéno ; alkoxy en C₁ à C₄ ; alkylthio en C₁ à C₄ ; trifluorométhyle ; cyano ; carboxy ; nitro ; alkyle en C₁ à C₄, alcényle en C₂ à C₄ ; (alkoxy en C₁ à C₄) carbonyle ; formyle ; (alkyle en C₁ à C₄) carbonyle ; (alcényle en C₂ à C₄) oxycarbonyle ; (alcényle en C₂ à C₄) carbonyle ; carboxy (alkyle en C₁ à C₄) ; halogénalkoxy en C₁ à C₄ ; halogénalkyle en C₁ à C₄ ; (alkyle en C₁ à C₄)carbonyloxy ; (alkoxy en C₁ à C₄) carbonyl (alkyle en C₁ à C₄) ; hydroxyalkyle en C₁ à C₄ ; mercapto(alkyle en C₁ à C₄) ; alkoxy en C₁ à C₄ ; hydroxy, amino ou aminocarbonyle facultativement substitué comme pour les substituants correspondants de R³ ; alkylsulfonyle en C₁ à C₄; alcénylsulfonyle en C₂ à C₄ ; ou aminosulfonyle dans lequel le groupe amino est facultativement mono- ou disubstitué avec un substituant alkyle en C₁ à C₄ ou alcényle en C₂ à C₄, sous réserve que R¹⁴ et R¹⁵ sur le même atome de carbone ne soient pas l'un et l'autre choisis entre un groupe hydroxy facultativement substitué et un groupe amino facultativement substitué ; ou bien
R¹⁴ et R¹⁵, conjointement, représentent un groupe oxo ;
R¹³ représente un atome d'hydrogène ; un groupe trifluorométhyle ; alkyle en C₁ à C₆ ; alcényle en C₂ à C₆ ; (alkoxy en C₁ à C₆)carbonyle ; (alkyle en C₁ à C₆)carbonyle ; ou aminocarbonyle dans lequel le groupe amino est facultativement substitué avec un substituant (alkoxy en C₁ à C₆)carbonyle, (alkyle en C₁ à C₆)carbonyle, (alcényle en C₂ à C₆)oxycarbonyle, (alcényle en C₂ à C₆)carbonyle, alkyle en C₁ à C₆ ou alcényle en C₂ à C₆ et facultativement substitué en outre avec un substituant alkyle en C₁ à C₆ ou alcényle en C₂ à C₆ ; ou
deux groupes R¹⁴ ou un groupe R¹³ et un groupe R¹⁴ sur des atomes de carbone adjacents, conjointement, représentent une liaison et les groupes R¹³, R¹⁴ et R¹⁵ restants répondent aux définitions précitées ;
deux groupes R¹⁴ et deux groupes R¹⁵ sur des atomes adjacents, conjointement, représentent des liaisons de telle sorte que X² et X³ soient triplement liés ;
deux groupes R¹⁴ ou un groupe R¹³ et un groupe R¹⁴ dans X² et X³, conjointement avec les atomes auxquels ils sont fixés, forment un noyau carbocyclique ou hétérocyclique penta-- ou hexagonal et les groupes R^{15a} restants répondent aux définitions précitées ;
X⁴ représente un groupe phényle ou un hétérocycle penta- ou hexagonal aromatique monocyclique, lié à C ou N, contenant jusqu'à quatre hétéroatomes choisis entre O, S et N et facultativement C-substitué avec jusqu'à trois groupes choisis entre des groupes alkylthio en C₁ à C₄ ; halogéno ; carboxy(alkyle en C₁ à C₄) ; halogénalkoxy en C₁ à C₄ ; halogénalkyle en C₁ à C₄ ; alkyle en C₁ à C₄ ; alcényle en C₂ à C₄ ; (alkoxy en C₁ à C₄) carbonyle ; formyle ; (alkyle en C₁ à C₄)carbonyle ; (alcényle en C₂ à C₄) oxycarbonyle, (alcényle en C₂ à C₄)carbonyle ; (alkyle en C₁ à C₄)carbonyloxy ; (alkoxy en C₁ à C₄) carbonyl (alkyle en C₁ à C₄) ; hydroxy ; hydroxyalkyle en C₁ à C₄ ; mercapto(alkyle en C₁ à C₄ ; alkoxy en C₁ à C₄ ; nitro ; cyano ; carboxy ; amino ou aminocarbonyle facultativement substitué comme pour les substituants correspondants dans R³ ; alkylsulfonyle en C₁ à C₄ ; alcénylsulfonyle en C₂ à C₄ ; ou aminosulfonyle dans lequel le groupe amino est facultativement substitué avec un substituant alkyle en C₁ à C₄ ou alcényle en C₂ à C₄ ; aryle, aryl (alkyle en C₁ à C₄) ou aryl(alkoxy en C₁ à C₄) ; et facultativement N-substitué avec un substituant trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à. C₄ ; aryle ; aryl (alkyle en C₁ à C₄) ; (alkoxy en C₁ à C₄)carbonyle ; (alkyle en C₁ à C₄)carbonyle ; formyle ; alkylsulfonyle en C₁ à C₆ ou aminocarbonyle dans lequel le groupe amino est facultativement substitué avec un substituant (alkoxy en C₁ à C₄) carbonyle, (alkyle en C₁ à C₄) carbonyle, (alcényle en C₂ à C₄) oxycarbonyle, (alcényle en C₂ à C₄) carbonyle, alkyle en C₁ à C₄ ou alcényle en C₂ à C₄ et facultativement substitué en outre avec un substituant alkyle en C₁ à C₄ ou alcényle en C₂ à C₄.
n est égal à 0 ou 1 ;
A représente un groupe NR¹¹, O ou CR⁶R⁷ et B représente un groupe NR¹¹, O, SO₂ ou CR⁸R⁹ et, dans lesquels :
chacun des groupes R⁶, R⁷, R⁸ et R⁹ est choisi indépendamment entre : un atome d'hydrogène ; des groupes alkoxy en C₁ à C₆ ; alkylthio en C₁ à C₆ ; halogéno ; trifluorométhyle ; azido ; alkyle en C₁ à C₆ ; alcényle en C₂ à C₆ ; (alkoxy en C₁ à C₆) carbonyle ; (alkyle en C₁ à C₆) carbonyle ; (alcényle en C₂ à C₆) oxycarbonyle ; (alcényle en C₂ à C₆)carbonyle ; hydroxy, amino ou aminocarbonyle facultativement substitué comme pour les substituants correspondants de R³ ; alkylsulfonyle en C₁ à C₆, alcénylsulfonyle en C₂ à C₆ ; ou aminosulfonyle dans lequel le groupe amino est facultativement substitué avec un substituant alkyle en C₁ à C₆ ; ou alcényle en C₂ à C₆ ;
ou lorsque n=1, R⁶ et R⁸, conjointement, représentent une liaison et R⁷ et R⁹ répondent aux définitions précitées ;
ou R⁶ et R⁷ ou R⁸ et R⁹, conjointement, représentent un groupe oxo ;
sous réserve que :
lorsque A représente un groupe NR¹¹, B ne représente pas un groupe NR¹¹ ou O ;
lorsque A représente un groupe CO, B ne représente pas un groupe CO, O ou SO₂ ;
lorsque n est égal à 0 et A représente un groupe NR¹¹, le groupe CR⁸R⁹ puisse représenter seulement un groupe CO ;
lorsque A représente un groupe CR⁶R⁷ et B représente un groupe SO₂, n soit égal à 0 ;
lorsque n est égal à 0, B ne représente pas un groupe NR¹¹ ou O ou R⁸ et R⁹ ne représentent pas un groupe hydroxy ou amino facultativement substitué ;
lorsque A représente un atome de O, B ne représente pas un groupe NR¹¹, O, SO₂ ou CO et n=1 ; et
lorsque A-B représente un groupe CR⁷=CR⁹, n soit égal à 1
R¹⁰ est choisi entre des groupes alkyle en C₁ à C₄ ; alcényle en C₂ à C₄ et aryle, n'importe lequel de ces groupes pouvant être facultativement substitué avec un groupe R¹² répondant à la définition précitée ; carboxy ; aminocarbonyle dans lequel le groupe amino est facultativement substitué avec un substituant hydroxy, alkyle en C₁ à C₆ ; alcényle en C₂ à C₆ ; alkylsulfonyle en C₁ à C₆ ; trifluorométhylsulfonyle ; alcénylsulfonyle en C₂ à C₆ ; (alkoxy en C₁ à C₆) carbonyle ; (alkyle en C₁ à C₆) carbonyle, (alcényle en C₂ à C₆) oxycarbonyle ou (alcényle en C₂ à C₆)carbonyle et facultativement substitué en outre avec un substituant alkyle en C₁ à C₆ ou alcényle en C₂ à C₆ ; alkylsulfonyle en C₁ à C₆ ; trifluorométhylsulfonyle ; alcénylsulfonyle en C₂ à C₆ ; (alkoxy en C₁ à C₆) carbonyle ; (alkyle en C₁ à C₆) carbonyle; (alcényle en C₂ à C₆) oxycarbonyle ; et (alcényle en C₂ à C₆) carbonyle ; et
R¹¹ représente un atome d'hydrogène ; un groupe trifluorométhyle, alkyle en C₁ à C₆ ; alcényle en C₂ à C₆ ; (alkoxy en C₁ à C₆) carbonyle ; (alkyle en C₁ à C₆)carbonyle ; ou aminocarbonyle dans lequel le groupe amino est facultativement substitué avec un substituant (alkoxy en C₁ à C₆) carbonyle, (alkyle en C₁ à C₆) carbonyle, (alcényle en C₂ à C₆)oxycarbonyle, (alcényle en C₂ à C₆) carbonyle, alkyle en C₁ à C₆ ou alcényle en C₂ à C₆ et facultativement substitué en outre avec un substituant alkyle en C₁ à C₆ ou alcényle en C₂ à C₆ ;
ou, lorqu'un des groupes R³ et R⁶, R⁷, R⁸ ou R⁹ contient un groupe carboxy et l'autre contient un groupe hydroxy ou amino, ils peuvent former, conjointement une liaison ester ou amide cyclique.

2. Composé suivant la revendication 1, dans laquelle Z⁵ représente un groupe CH ou N, Z³ représente un groupe CH ou CF et Z¹, Z² et Z⁴ représentent chacun un groupe CH, ou Z¹ représente un atome de N, Z³ représente un groupe CH ou CF et Z², Z⁴ et Z⁵ représentent chacun un groupe CH.

3. Composé suivant l'une quelconque des revendications précédentes, dans lequel R¹ représente un groupe méthoxy et R^{1a} représente H ou, lorsque Z³ représente le groupe CR^{1a}, il peut représenter un groupe C-F.

4. Composé suivant l'une quelconque des revendications précédentes, dans lesquelles R² représente un atome d'hydrogène, un groupe carboxyméthyle, hydroxyéthyle, aminocarbonylméthyle, éthoxycarbonylméthyle, éthoxycarbonylallyle ou carboxyallyle.

5. Composé suivant l'une quelconque des revendications précédentes, dans lequel R³ représente un atome d'hydrogène ; un groupe hydroxy facultativement substitué ; alkyle en C₁ à C₄ ; éthényle ; 1-hydroxyalkyle en C₁ à C₄ facultativement substitué ; aminocarbonyle facultativement substitué ; carboxy(alkyle en C₁ à C₄) ; aminocarbonyl(alkyle en C₁ à C₄) facultativement substitué ; cyano(alkyle en C₁ à C₄) ; 2-oxo-oxazolidinyle facultativement substitué ou 2-oxo-oxazolidinyl(alkyle en C₁ à C₄) facultativement substitué ; en position 3 ou 4.

6. Composé suivant l'une quelconque des revendications précédentes, dans lequel n est égal à 0, A-B représente un groupe CHOH-CH₂, NR¹¹-CH₂ ou NR¹¹-CO et R¹¹ représente un atome d'hydrogène ou un groupe alkyle en C₁ à C₄.

7. Composé suivant l'une quelconque des revendications précédentes, dans lequel -X¹-X²-X³- représente un groupe -(CH₂)₂-O-, -CH₂-CH=CH-, -(CH₂)₃-, -(CH₂)₂-NH- ou -CH₂CONH-.

8. Composé suivant l'une quelconque des revendications précédentes, dans lequel X⁴ représente un groupe 2-pyridyl, 3-fluorophényl, 3,5-difluorophényl ou thiazole-2-yle.

9. Composé suivant la revendication 1, choisi entre les suivants :
2-{1-[(R)-2-hydroxy-2-(6-méthoxy-[1,5]-naphthyridine-4-yl)-éthyl]-pipéridin-4-ylamino}-N-phénylacétamide ;
4-{*trans*-1-[3,5-difluorophényl]-3-propényl}amino-1-[2-(R)-hydroxy-2-(6-méthoxyquinolin-4-yl)]éthylpipéridine ;
(R)-1-(6-méthoxy-[1,5]naphthyridin-4-yl)-2-{4-[2-(pyridin-2-yloxy)-éthylamino]-pipéridin-1-yl}-éthanol ;
(R)-1-(6-méthoxy-[1,5]naphthyridin-4-yl)-2-[4-((E)-3-pyridin-2-yl-allylamino)-pipéridin-1-yl]-éthanol ;
4-[2(3,5-difluorophénylamino)-éthyl]amino-1-[2-(R)-hydroxy-2-(6-méthoxyquinolin-4-yl)]éthylpipéridine ;
4-[2(3-fluorophénylamino)-éthyl]amino-1-[2-(R)-hydroxy-2-(6-méthoxyquinolin-4-yl)]éthylpipéridine ; et
4- [trans-1- (2-thiazolyl) -3-propényl]amino-1-[2-(R)-hydroxy-2-(6-méthoxy-[1,5]-naphtyridin-4-yl)]éthylpipéridine
ou un de leurs dérivés pharmaceutiquement acceptables.

10. Utilisation d'un composé suivant la revendication 1 dans la production d'un médicament destiné à être utilisé dans le traitement d'infections bactériennes chez des mammifères.

11. Composition pharmaceutique comprenant un composé suivant la revendication 1 et un support pharmaceutiquement acceptable.

12. Procédé pour la préparation de composés suivant la revendication 1, procédé qui comprend :
la réaction d'un composé de formule (IV) avec un composé de formule (V) : 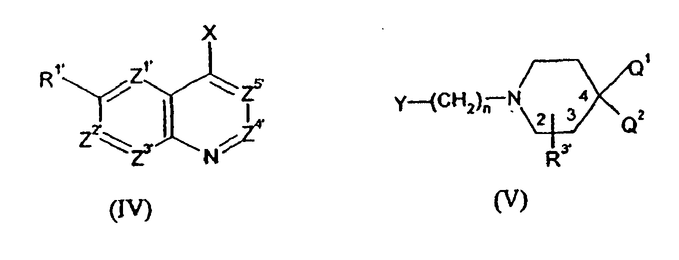
formules dans lesquelles n répond à la définition mentionnée par la formule (I) ; Z^{1'}, Z^{2'}, Z^{3'}, Z^{4'}, Z^{5'}, R^{1'} et R^{3'} représentent des groupes Z¹, Z², Z³, Z⁴, Z⁵, R¹ et R³ tels que définis pour la formule (I) ou des groupes pouvant être convertis en ceux-ci ;
Q¹ représente un groupe NR²R⁴ ou un groupe pouvant être converti en celui-ci, dans lequel R^{2'} et R^{4'} représentent des groupes R² et R⁴ tels que définis pour la formule (1) ou des groupes pouvant être convertis en ceux-ci et Q² représente H ou un groupe R³' ou bien Q¹ et Q², conjointement, forment un groupe oxo facultativement protégé ;
et X et Y peuvent représenter les associations suivantes :
(i) X représente un groupe A'-COW, Y représente H et n est égal à 0 ;
(ii) X représente un groupe CR⁶=CR⁸R⁹, Y représente H et n est égal à 0 ;
(iii) X représente un groupe oxiranne, Y représente H et n est égal à 0 ;
(iv) X représente un groupe N=C=O, Y représente H et n est égal à 0 ;
(v) un de X et Y représente un groupe CO₂R^{y} et l'autre représente un groupe CH₂CO₂R^{x} ;
(vi) X représente un groupe CHR⁶R⁷ et Y représente un groupe C(=O)R⁸ ;
(vii) X représente un groupe CR⁷=PR^{z} ₃, Y représente un groupe C(=O)R⁹ et n est égal à 1 ;
(viii) X représente un groupe C(=O)R⁷ et Y représente un groupe CR⁹=PR^{z} ₃ et n est égal à 1 ;
(ix) Y représente un groupe COW et X représente un groupe NHR^{11'} ou NR^{11'} COW et n est égal à 0 ou 1 ou, lorsque n est égal à 1, X représente un groupe COW et Y représente un groupe NHR^{11'}, NCO ou NR^{11'}COW ;
(x) X représente un groupe C(=O)R⁶ et Y représente un groupe NHR^{11'} ou bien X représente un groupe NHR^{11'} et Y représente un groupe C(=O)R⁸ et n est égal à 1 ;
(xi) X représente un groupe NHR^{11'} et Y représente un groupe CR⁸R⁹W et n est égal à 1 ;
(xii) X représente un groupe CR⁶R⁷W et Y représente un groupe NHR^{11'} ou OH et n est égal à 1 ; ou
(xiii) X représente un groupe CR⁶R⁷SO₂W et Y représente H et n est égal à 0 ;
(xiv) X représente un groupe W ou OH et Y représente un groupe CH₂OH et n est égal à 1 ;
(xv) X représente un groupe NHR^{11'} et Y représente un groupe SO₂W ou X représente un groupe NR^{11'}SO₂W et Y représente H et n est égal à 0 ;
(xvi) X représente un groupe NHR^{11'}COCH₂W et Y représente H et n est égal à 0 ;
dans laquelle W représente un groupe partant, par exemple halogéno ou imidazolyle ; R^{x} et R^{y} représentent des groupes alkyle en C₁ à C₆ ; R^{z} représente un groupe aryle ou alkyle en C₁ à C₆ ; A' et NR^{11'} représentent des groupes A et NR¹¹ tels que définis pour la formule (I), ou des groupes pouvant être convertis en ceux-ci ; et l'oxiranne répond à la formule : 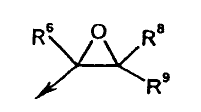 dans laquelle R⁶, R³ et R⁹ répondent aux définitions mentionnées par la formule (I) ;
et ensuite, facultativement ou si nécessaire, la conversion de Q¹ et Q² en un groupe NR^{2'}R^{4'} ; la conversion de A', Z^{1'}, Z^{2'}, Z^{3'}, Z^{4'}, Z^{5'}, R^{1'}, R^{2'}, R^{3'}, R^{4'} et NR^{11'} en A, Z¹, Z², Z³, Z⁴, Z⁵, R¹, R², R³, R⁴ et NR¹¹ ; la conversion de A-B en un autre groupe A-B, l'interconversion de R¹, R², R³ et/ou R⁴, et/ou la formation d'un dérivé pharmaceutiquement acceptable du composé obtenu.

13. Composé de formule (VI) 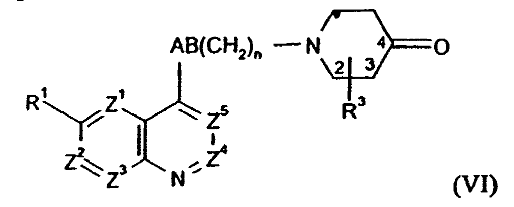 dans laquelle les variables sont telles que décrites pour la formule (I) dans la revendication 1.
